# EUROPEAN PATENT SPECIFICATION

(11) **EP 1 922 329 B1**
(45) Date of publication and mention of the grant of the patent: **29.05.2013**
(21) Application number: 06844159.1
(22) Date of filing: 02.08.2006
(51) Int. Cl.: C07K 14/095, C07K 14/705, C12N 15/82

(54) **IMPROVED CHIMERIC TOXIN RECEPTOR PROTEINS AND CHIMERIC TOXIN RECEPTOR PROTEINS FOR TREATMENT AND PREVENTION OF ANTHRAX**
VERBESSERTE CHIMÄRE TOXINREZEPTORPROTEINE UND CHIMÄRE TOXINREZEPTOR-PROTEINE ZUR BEHANDLUNG UND VORBEUGUNG VON ANTHRAX
PROTÉINES CHIMÉRIQUES DE RÉCEPTEUR DE TOXINES AMÉLIORÉES ET PROTÉINES CHIMÉRIQUES DE RÉCEPTEUR DE TOXINES POUR LE TRAITEMENT PROPHYLACTIQUE ET THÉRAPEUTIQUE DE L'ANTHRAX

(30) Priority: 02.08.2005 US 704829 P
(43) Date of publication of application: 21.05.2008
(73) Proprietor: PLANET BIOTECHNOLOGY, INC., Hayward, CA 94545 (US)
(72) Inventor: YU, Lloyd, M., Mountain View, CA 94040 (US); WYCOFF, Keith, L., Palo Alto, CA 94303 (US); LARRICK, James, W., Woodside, CA 94062 (US)
(74) Representative: Williams, Richard Andrew Norman
(86) International application number: PCT/US2006/030325
(87) International publication number: WO 2007/044115

(56) References cited:
- WO-A-01/83529
- WO-A-03/064992
- WO-A-2006/046072
- SCOBIE H M ET AL: "Human capillary morphogenesis protein 2 functions as an anthrax toxin receptor" PROCEEDINGS OF THE NATIONAL ACADEMY OF SCIENCES OF USA, NATIONAL ACADEMY OF SCIENCE, WASHINGTON, DC, US, vol. 100, no. 9, 29 April 2003 (2003-04-29), pages 5170-5174, XP002386353 ISSN: 0027-8424 cited in the application
- BRADLEY K A ET AL: "Identification of the cellular receptor for anthrax toxin" NATURE, NATURE PUBLISHING GROUP, LONDON, GB, vol. 414, no. 6860, 8 November 2001 (2001-11-08), pages 225-229, XP002228301 ISSN: 0028-0836 cited in the application
- WYCOFF K: "Production of biodefense-related proteins in tobacco." IN VITRO CELLULAR & DEVELOPMENTAL BIOLOGY ANIMAL, vol. 42, no. Suppl. S, April 2006 (2006-04), page 14A, XP009090622 & MEETING OF THE SOCIETY-FOR-IN-VITRO-BIOLOGY; MINNEAPOLIS, MN, USA; JUNE 03 -07, 2006 ISSN: 1071-2690
- GOYAL ANITA ET AL: "Inclusion of a furin-sensitive spacer enhances the cytotoxicity of ribotoxin restrictocin containing recombinant single-chain immunotoxins", BIOCHEMICAL JOURNAL, vol. 345, no. 2, 15 January 2000 (2000-01-15), pages 247-254, ISSN: 0264-6021
- SIDHU SACHDEV S ET AL: "Phage-displayed antibody libraries of synthetic heavy chain complementarity determining regions", JOURNAL OF MOLECULAR BIOLOGY, vol. 338, no. 2, 23 April 2004 (2004-04-23), pages 299-310, ISSN: 0022-2836

## Description

The present invention relates to chimeric toxin receptor proteins, immunoadhesins, their production from plants, and their use in the treatment and prevention of toxicity and pathogen-mediated ailments.

Soluble receptors and binding proteins that act as decoys for toxins and pathogen components that mediate infection and pathogenesis are a promising means to treat and prevent toxicity and other pathogen-mediated effects.

One example of a condition treatable with a receptor decoy is anthrax infection. Two cell-surface receptors for PA (protective antigen, a component of anthrax toxin) have been identified: tumor endothelial marker 8 (TEM8) and capillary morphogenesis protein 2 (CMG2) (30, 31). Soluble forms of the extracellular domains of both of these proteins have been shown to inhibit intoxication of cells expressing endogenous toxin receptors. The soluble form of TEM8 inhibited lethal toxin action by 50% at 80 nM (IC50 = 80 nM) (Bradley, K. A., Mogridge, J., Mourez, M., Collier, R. J. & Young, J. A. Identification of the cellular receptor for anthrax toxin. Nature 414, 225-9 (2001)). CMG2-derived protein had an affinity for PA of 170 pM, and an IC50 in the macrophage protection assay of 3.1 nM (67 ng/ml), making it a potentially potent inhibitor of anthrax toxin action (Scobie, H. M., Rainey, G. J., Bradley, K. A. & Young, J. A. Human capillary morphogenesis protein 2 functions as an anthrax toxin receptor. Proc Natl Acad Sci U S A 100, 5170-4 (2003); Wigelsworth, D. J., Krantz, B. A., Christensen, K. A., Lacy, D. B., Juris, S. J. & Collier, R. J. Binding stoichiometry and kinetics of the interaction of a human anthrax toxin receptor, CMG2, with protective antigen. J Biol Chem 279, 23349-56 (2004)). In cell culture assays, soluble CMG2 extracellular domain neutralized 50% of lethal toxin activity at a 3:1 molar ratio with PA (Scobie et al. Proc Natl Acad Sci U S A 100, 5170-4 (2003)). However, the high concentration of bacilli in blood during an active infection suggests that the concentration of PA may be quite high, requiring high doses of antitoxin to provide protection.

WO 03/064992 (PLANET) describes a chimeric immunoglobulin anthrax toxin receptor complex including a portion of an immunoglobulin heavy chain and a portion of a immunoglobulin light chain.

To minimize the amount of receptor decoy protein needed to ensure efficacy, it would be desirable to increase the specific activity or potency of the receptor decoys even further. Thus, there remains a need for potent decoys with higher activity and improved pharmacokinetics.

The receptor decoys that have been described suffer from several drawbacks, including laborious production techniques and high costs associated with those production methods. In addition, these receptor decoys have limited stability as multimers in the harsh environment in which the molecule may need to inactivate pathogens. Thus, there is a need for decoy technologies with increased stability to increase yield and which can be produced in commercially feasible volumes at a reasonable cost.

The invention provides a chimeric receptor protein as set forth in any of claims 1 to 9.

In preferred embodiments, the chimeric toxin receptor proteins are expressed in plants, including monocotyledonous plants and dicotyledonous plants as a part of the plants' genome. Tobacco is a preferred plant for expression. Expression in plants, as opposed to expression in cultured mammalian cells, allows for a significant reduction in the cost of producing the chimeric toxin receptor proteins.

In a preferred embodiment, all proteins used to make the chimeric toxin are receptor proteins expressed in heterologous cells derived from plants, vertebrates or invertebrates such as mammalian cells, hairy root cells, or plant tissue culture cells.

Immunoadhesins including the chimeric toxin receptors of the invention are described herein. In some embodiments, the immunoadhesin is a multimer, including at least two chimeric toxin receptor proteins. In other embodiments, the immunoadhesin includes chimeric toxin receptor protein associated with J chain. In another embodiment, the composition comprising the immunoadhesin associated with J chain is further associated with secretory component.

In one embodiment, the present invention contemplates a chimeric toxin receptor protein having plant-specific glycosylation. A gene coding for a polypeptide having within its amino acid sequence the glycosylation signal asparagine-X-serine/threonine, where X can be any amino acid residue, is glycosylated via oligosaccharides linked to the asparagine residue of the sequence when expressed in a plant cell. See Marshall, Ann. Rev. Biochem., 41:673 (1972) and Marshall, Biochem. Soc. Symp., 40:17 (1974) for a general review of the polypeptide sequences that function as glycosylation signals. These signals are recognized in both mammalian and in plant cells. At the end of their maturation, proteins expressed in plants or plant cells have a different pattern of glycosylation than do proteins expressed in other types of cells, including mammalian cells and insect cells. Detailed studies characterizing plant-specific glycosylation and comparing it with glycosylation in other cell types have been performed, for example, in studies described by Cabanes-Macheteau et al., Glycobiology 9(4):365-372 (1999), and Altmann, Glycoconjugate J. 14:643-646 (1997). These groups and others have shown that plant-specific glycosylation generates glycans that have xylose linked β(1,2) to mannose, but xylose is not linked β(1,2) to mannose as a result of glycosylation in mammalian and insect cells. Plant-specific glycosylation results in a fucose linked a(1,3) to the proximal GlcNAc, while glycosylation in mammalian cells results in a fucose linked a(1,6) to the proximal GlcNAc. Furthermore, plant-specific glycosylation does not result in the addition of a sialic acid to the terminus of the protein glycan, whereas in glycosylation in mammalian cells, sialic acid is added.

In another embodiment, the foregoing compositions are additionally associated with plant material. The plant material present may be plant cell walls, plant organelles, plant cytoplasms, intact plant cells, plant seeds, viable plants, and the like. The particular plant materials or plant macromolecules that may be present include ribulose bisphosphate carboxylase, light harvesting complex, pigments, secondary metabolites or chlorophyll and fragments thereof. Compositions of the present invention may have a chimeric toxin receptor protein concentration of between 0.001% and 99.9% mass excluding water. In other embodiments, the chimeric toxin receptor protein is present in a concentration of 0.01% to 99% mass excluding water. In other embodiments, the compositions of the present invention have plant material or plant macromolecules present at a concentration of 0.01% to 99% mass excluding water.

In another aspect, the invention provides methods for reducing binding of a pathogen antigen as set forth in claim 2. The invention includes a chimeric toxin receptor as hereinbefore defined for use as a medicament for a patient or animal.

The invention further includes a chimeric toxin receptor protein as hereinbefore defined for use in preventing or treating anthrax infection, or the effects of anthrax toxin.

In another aspect, the invention provides pharmaceutical compositions including the chimeric toxin receptor proteins described herein and a pharmaceutically acceptable carrier.

In another aspect, the invention provides an expression vector encoding a CMG2 chimeric toxin receptor protein as described.

In another aspect, the invention provides methods for producing a chimeric toxin receptor protein as set forth in claims 16 or 17.

Alterations and modifications to the receptor protein or portion thereof are also contemplated, provided such modifications do not destroy the ability of the receptor to bind the toxin, toxicant, pathogen, or pathogen component.

As described above the Anthrax chimeric toxin receptor proteins of the invention can be expressed in plants and a variety of heterologous cells. In a preferred embodiment, all proteins used to make the chimeric toxin receptor proteins of the present invention are human proteins.

In another aspect, the invention provides immunoadhesins including the Anthrax chimeric toxin receptors described herein, such as multimers with at least two Anthrax chimeric toxin receptor proteins, Anthrax chimeric toxin receptor protein associated with J chain, and Anthrax chimeric toxin receptor protein associated with J chain and secretory component.

In other embodiments, the present invention contemplates an Anthrax chimeric toxin receptor protein having plant-specific glycosylation and the foregoing compositions additionally associated with plant material.

Also described herein is a method for reducing or preventing the binding of protective antigen (PA) of *Bacillus anthracis* to host cells by contacting PA with the Anthrax chimeric toxin receptors described herein. Other aspects feature methods for reducing morbidity and mortality of anthrax and morbidity and mortality due to protective antigen.

In other aspects, the invention provides pharmaceutical compositions including Anthrax chimeric toxin receptors, expression vectors for such receptors, and methods for making such receptors.

### BRIEF DESCRIPTION OF THE DRAWINGS

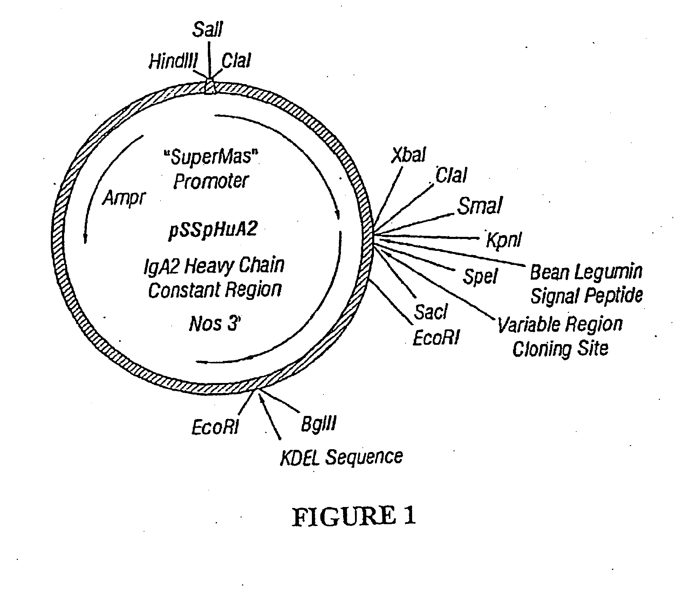

FIG. 1 illustrates pSSPHuA2, vector in which DNAs encoding a chimeric ICAM-1 molecule containing the first five domains of human ICAM-1 and the Fc region of human IgA2m(2) were fused [SEQ ID NO:9, 48]. This vector contains the SuperMas promoter for driving the expression of a signal peptide and the constant regions of the human IgA2m(2) heavy-chain. Sequences encoding ICAM domains 1-5 were amplified by PCR to contain convenient restriction sites (5' SpeI and 3' SpeI) for insertion between the signal peptide and the Cα1 domain. DNA encoding an ER retention signal (RSEKDEL) [SEQ ID NO:5] was appended to the 3' end of the heavy-chain in order to boost the expression level of the construct.

FIG. 2 illustrates a chimeric ICAM molecule. 2A shows the DNA expression cassette from which the chimeric ICAM-1 molecule was produced. 2B shows the amino acid sequence, after signal peptide cleavage, of the mature form of the fusion protein [SEQ ID NO:8]. Amino acids introduced by the cloning procedure are underlined and mark the junction between the five extracellular domains of ICAM-1 and the Cα1-Cα3 domains of the IgA2m(2) heavy chain. The bolded N's indicate the fifteen potential glycosylation sites.

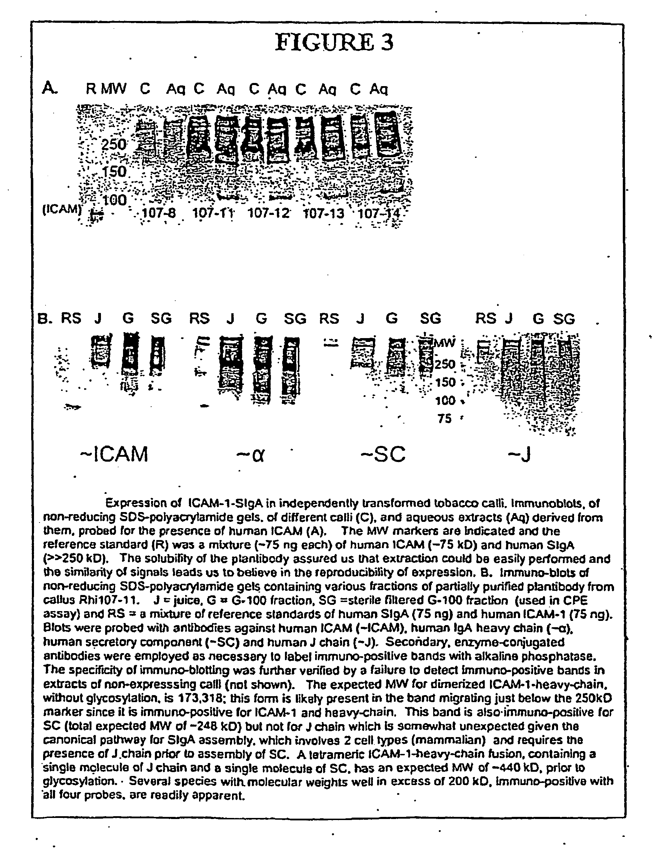

FIG. 3 illustrates the expression of the immunoadhesin in independently transformed tobacco calli. 3A shows immunoblots of non-reducing SDS-polyacrylamide gels on which samples containing different transformed tobacco calli (C) and aqueous extracts (Aq) were run and probed for the presence of human ICAM. The molecular weight markers are indicated, and the reference standard (R) was a mixture (∼75 ng each) of human ICAM (∼75 kD) and human SigA (>>250 kD). 3B shows immunoblots of nonreducing SDS-polyacrylamide gels containing various fractions of partially purified immunoadhesin from callus Rhi107-11. The purification fractions analyzed were juice (3), G-100 fraction (G), sterile filtered G-100 fraction (SG), and a mixture of reference standards of human SigA (75 ng) and human ICAM-1 (75ng) (RS). Blots were probed with antibodies against human ICAM (∼ICAM), human IgA heavy chain (∼α), human secretory component (∼SC) and human J chain (∼J). Secondary, enzyme-conjugated antibodies were employed as necessary to label immuno-positive bands with alkaline phosphatase.

FIG. 4 illustrates the results of an enzyme-linked immunosorbent assay (ELISA) showing competition between plant extract and soluble ICAM-1 for binding to an anti-ICAM mAb. For the assay, 96-well plates were coated with 0.25 *µ*g soluble ICAM-1/ml. The squares represent the increasing concentrations of sICAM and the circles represent the increasing amounts of callus extract (sterile filtered fraction from G-100) used to compete with the adhered ICAM for a constant amount of a mouse (anti-human ICAM) antibody.

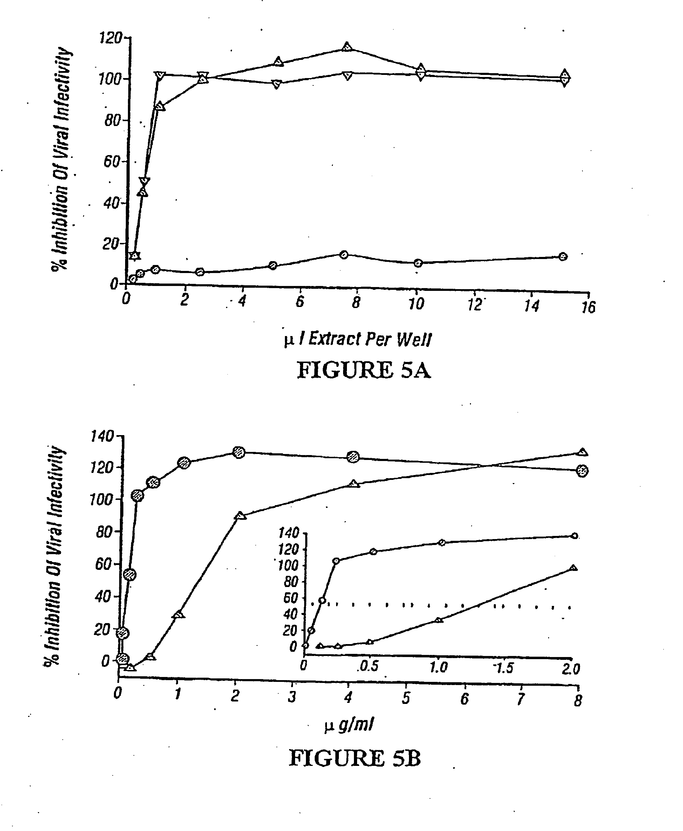

FIG. 5 illustrates the results of an assay showing the ability of an immunoadhesin to inhibit human rhinovirus killing of HeLa cells (cytopathic effect, or CPE, assay). 5A shows the results of an assay comparing the CPE of human rhinovirus on HeLa cells in the presence of partially purified extracts containing either the immunoadhesin in the ICAM-Fc fusion (IC1-5D/IgA) or containing an antibody against doxorubicin. (The right side-up and upside-down triangles represent two extracts derived from Rhi107-11, containing the immunoadhesin.) 5B shows the results of an assay comparing the CPE of human rhinovirus on HeLa cells in the presence of soluble human ICAM-1 or an extract from the immunoadhesin in the ICAM-Fc fusion (IC1-5D/IgA). The Inset shows scale expansion in the range of the IC50 for soluble ICAM (1.35 *µ*g/ml) and for IC1-5D/IgA (0.12 *µ*g/ml; 11.3 fold-less).

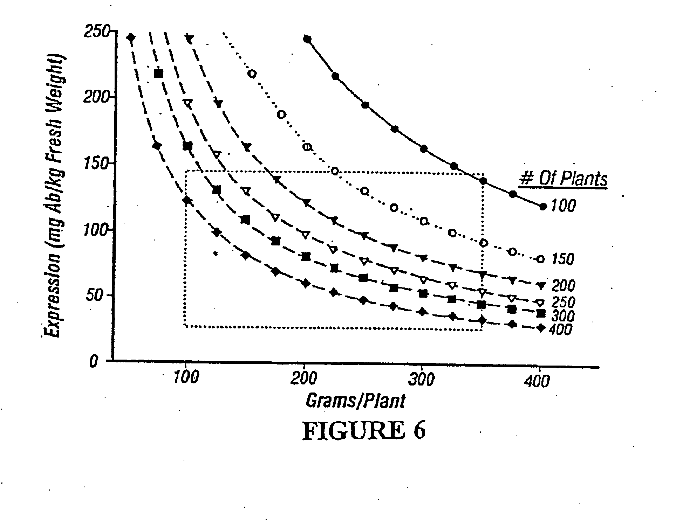

FIG. 6 shows an evaluation of the production necessities for making 1 gram of finished immunoadhesin. In this diagram, the number of plants needed for 1 g of immunoadhesin, at 20% yield, at expected levels of expression and plant weight is illustrated. At different levels of immunoadhesin expression (mg/kg fresh weight) and overall recovery (set at 20%), the weight of each plant, and so the total number of plants, may be determined for a specified production target (1 g/harvest) within a window (dotted square) of reasonable possibilities. The number of required plants decreases, inversely, with the number of specified growth and re-growth periods. The expected biomass production, a function of time and growth conditions, influences the time to harvest and the time between harvests. These growth periods can be adjusted to the realities of the purification schedule by staggering planting and harvesting dates.

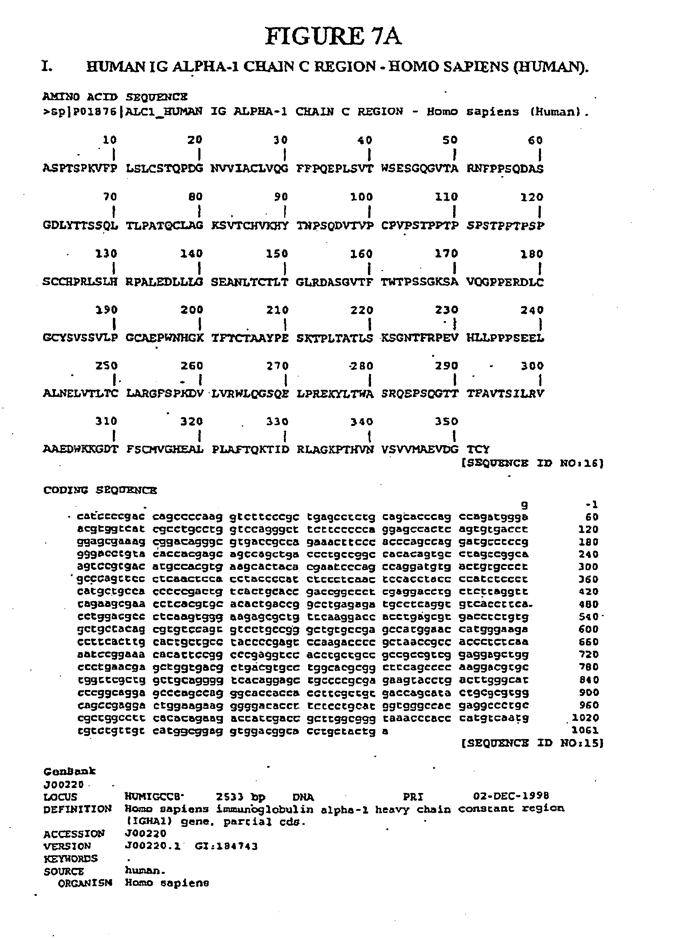

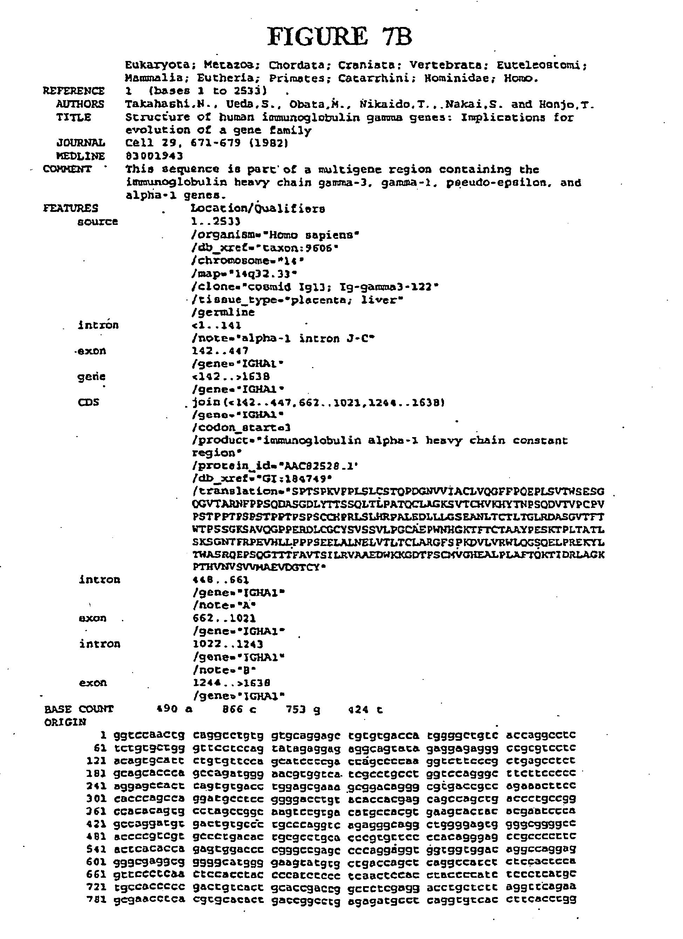

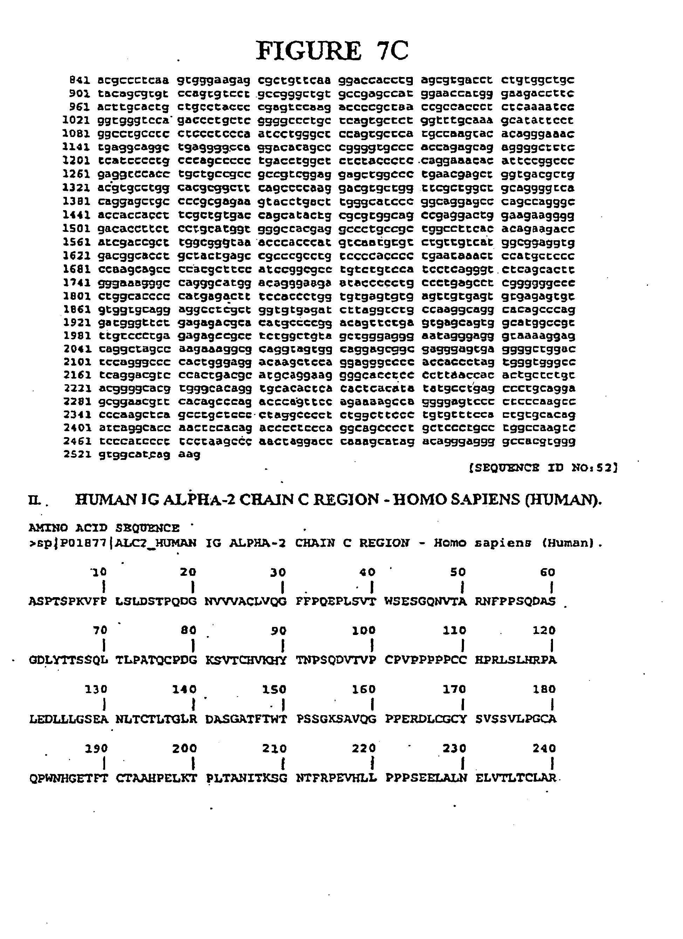

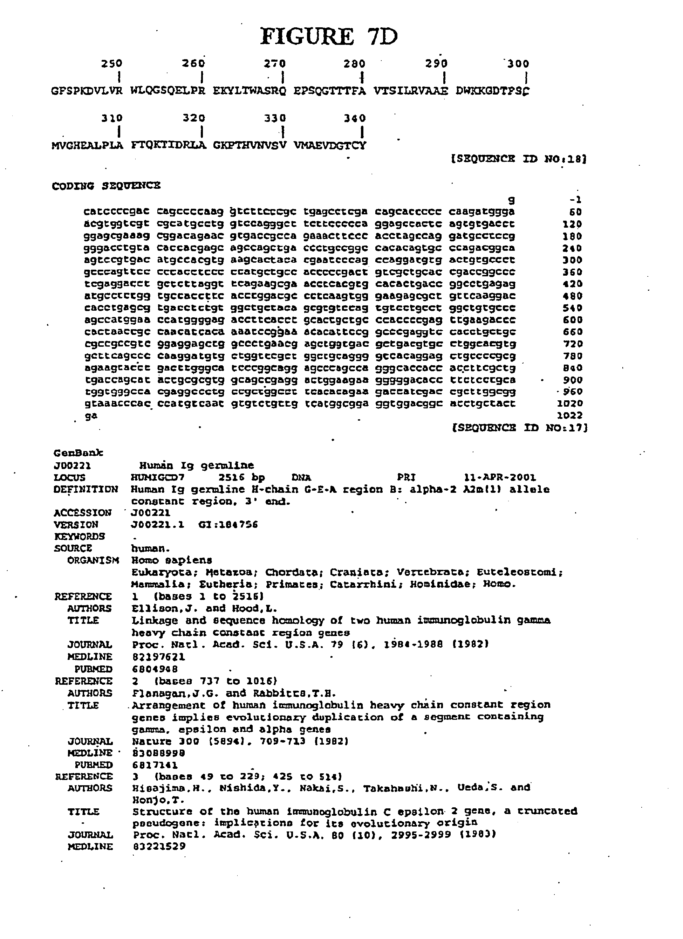

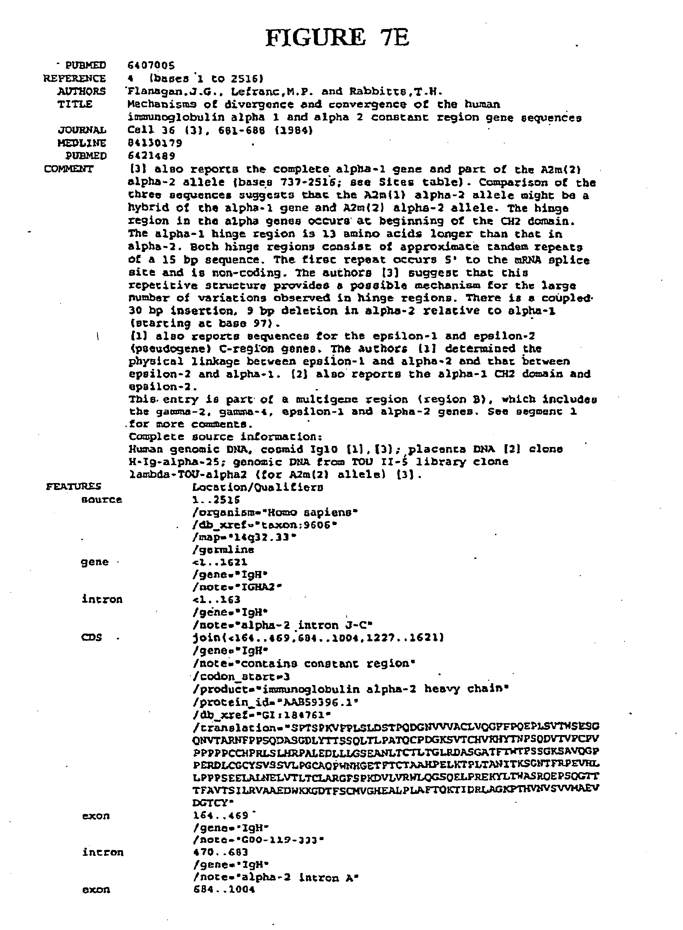

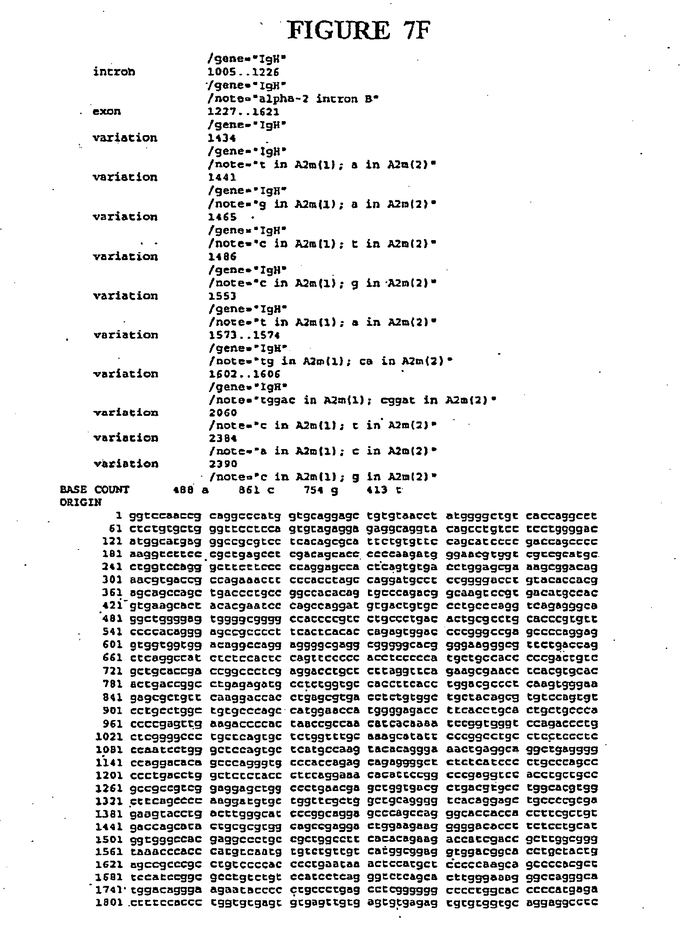

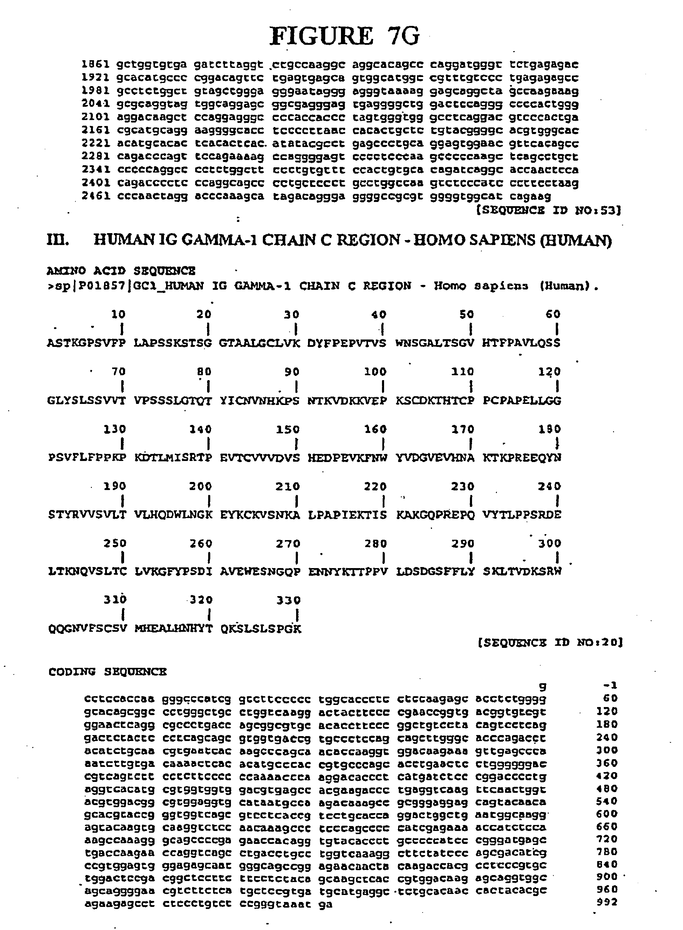

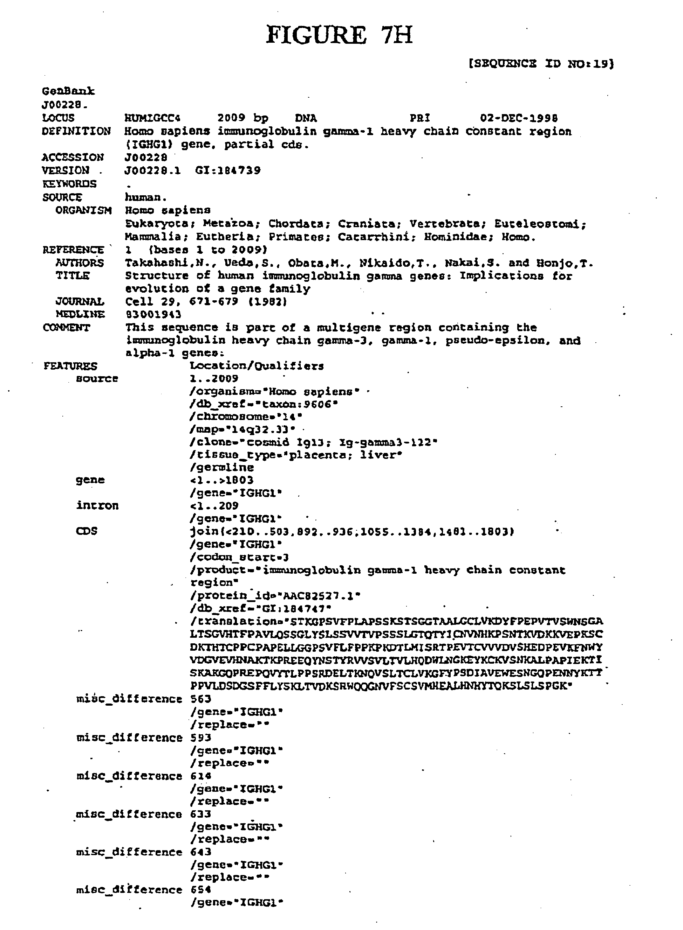

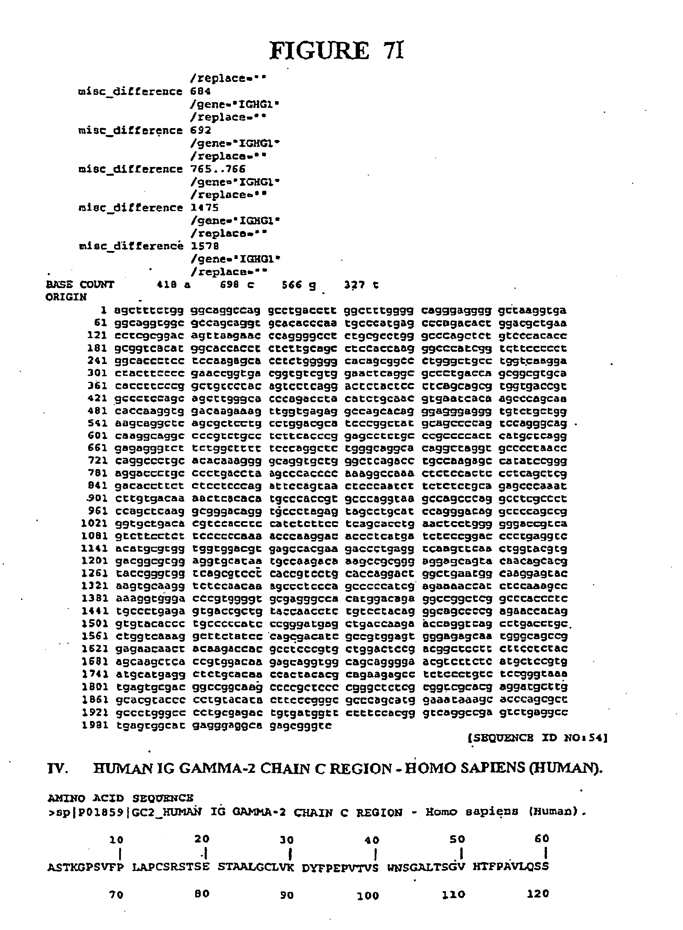

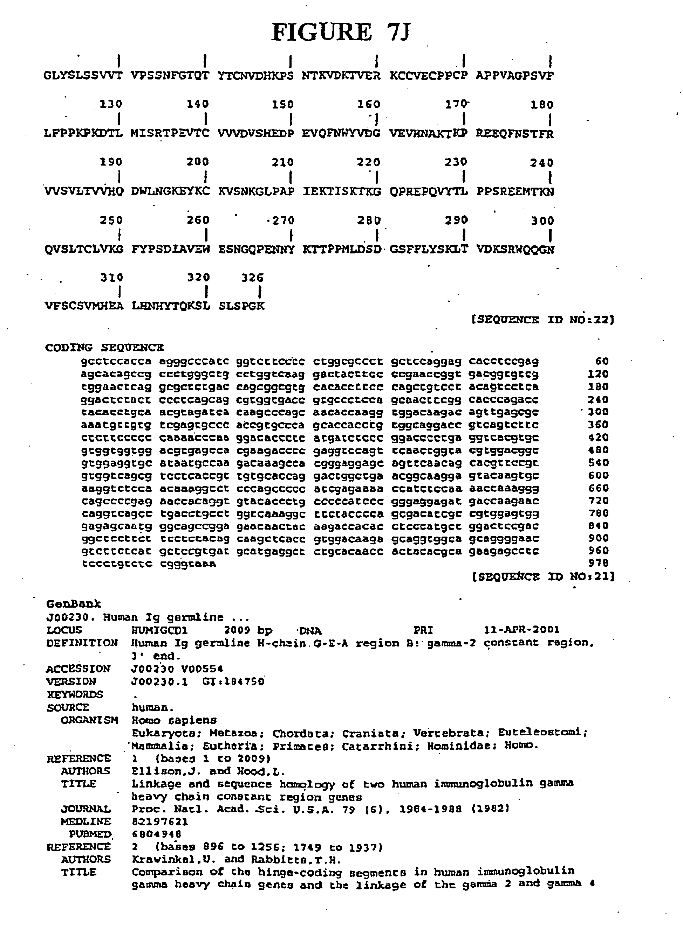

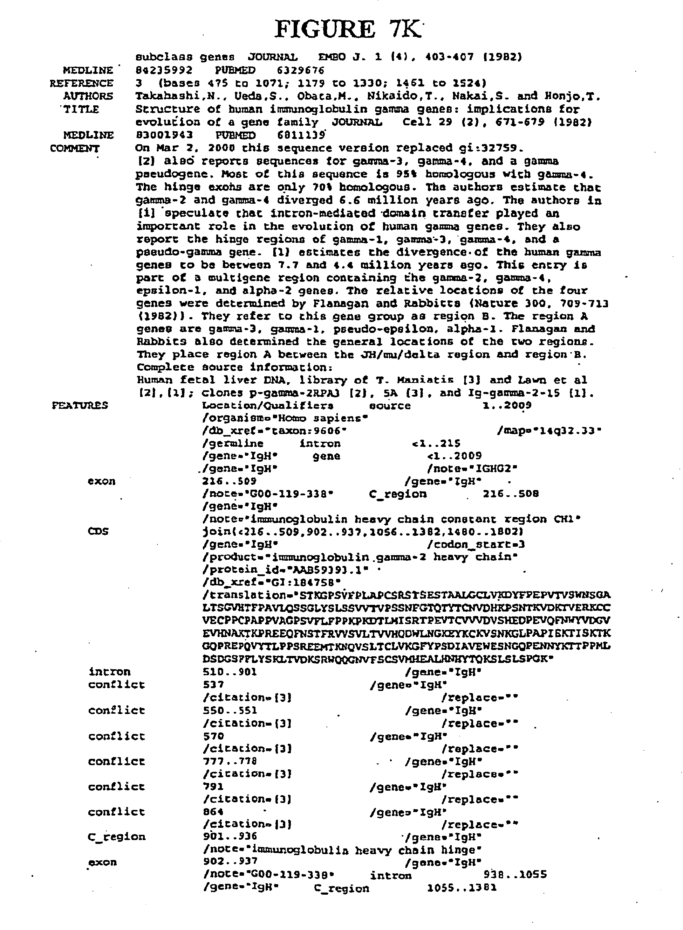

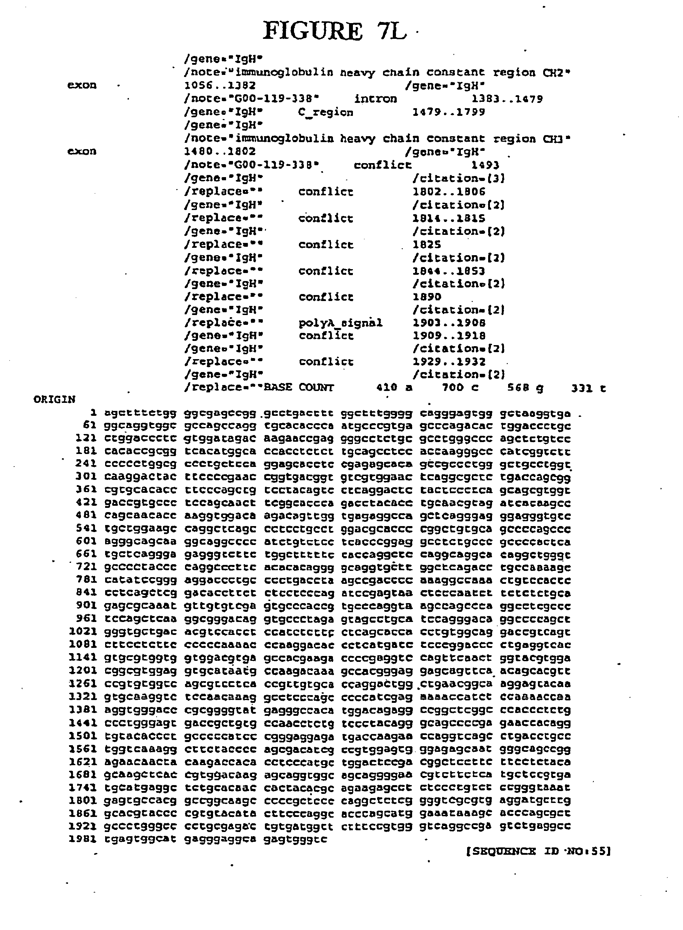

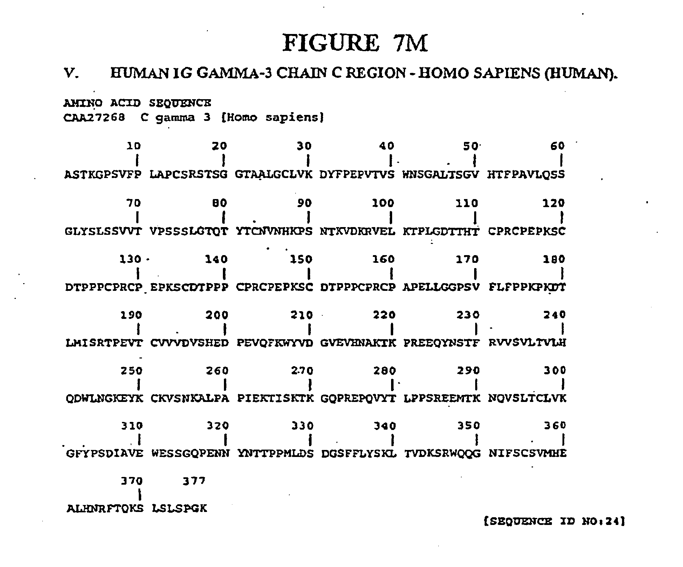

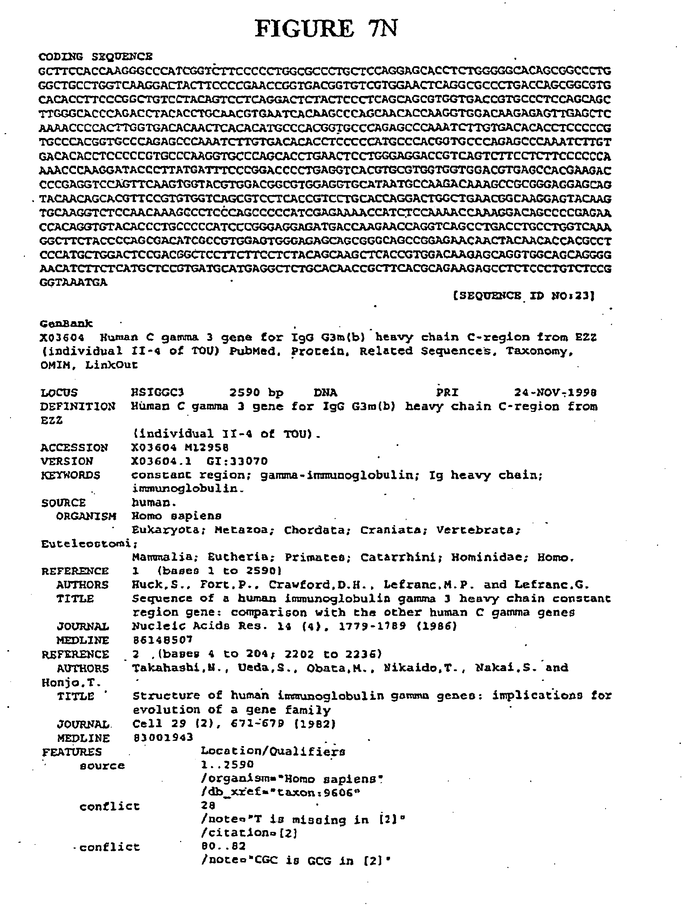

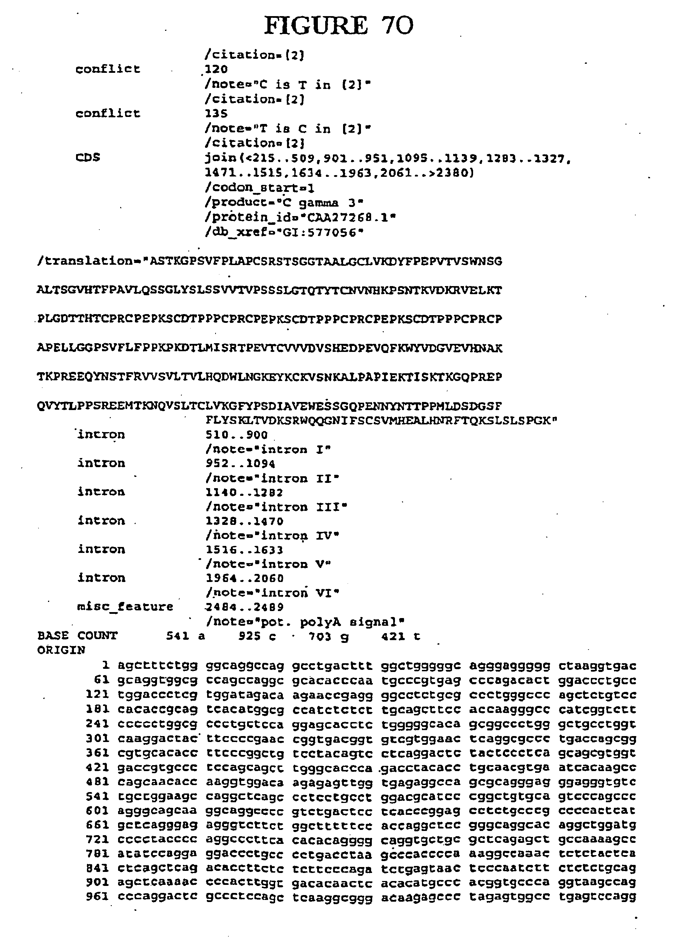

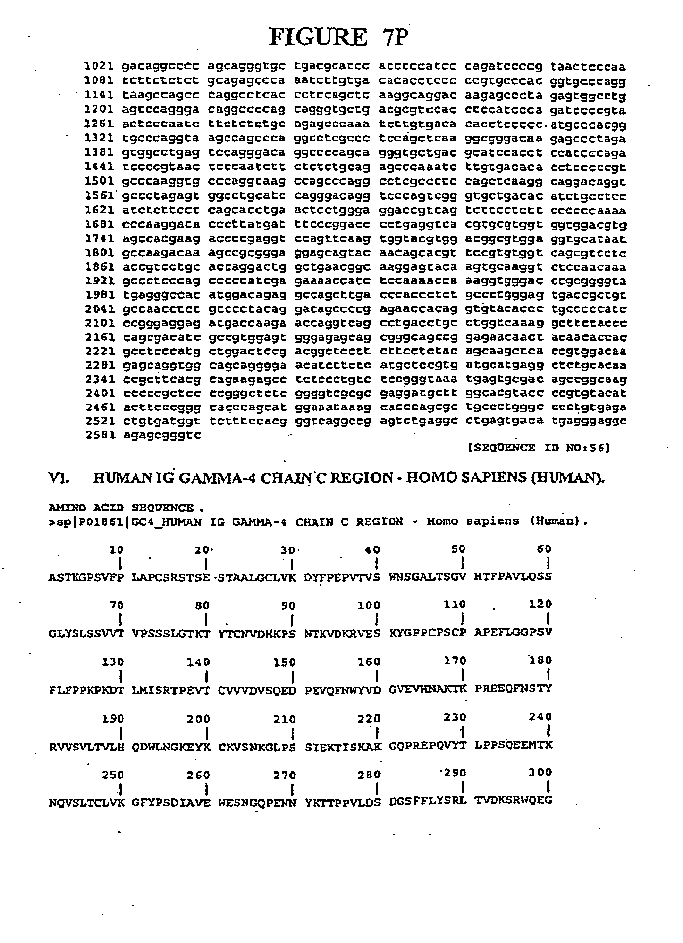

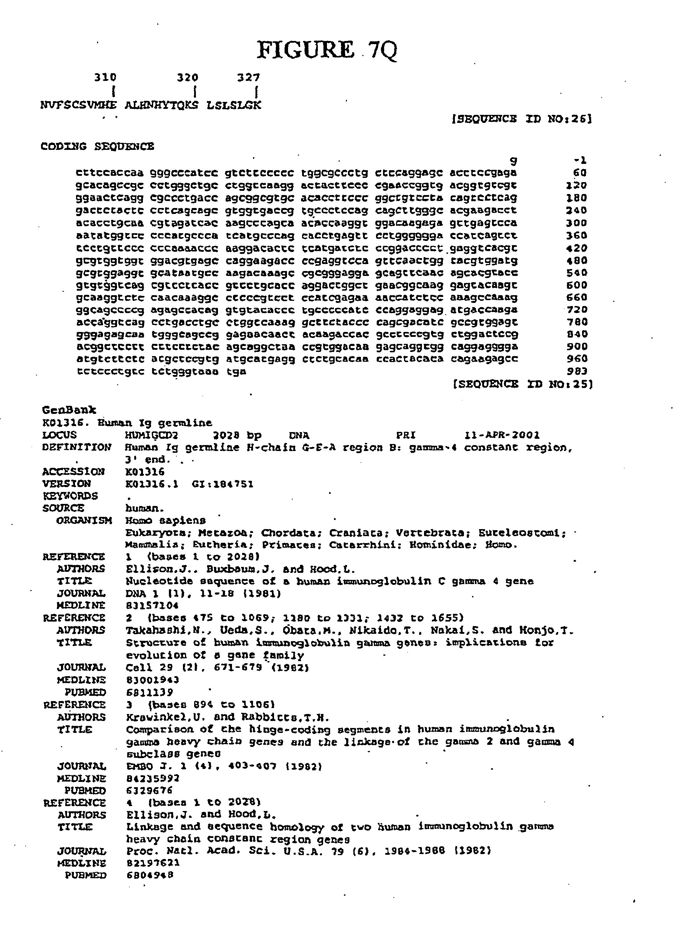

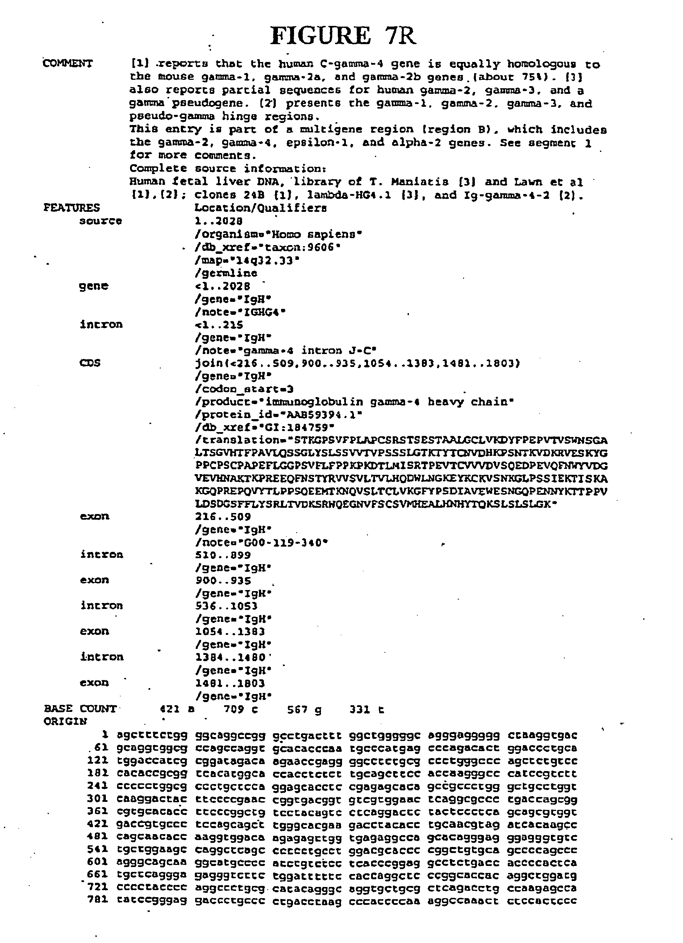

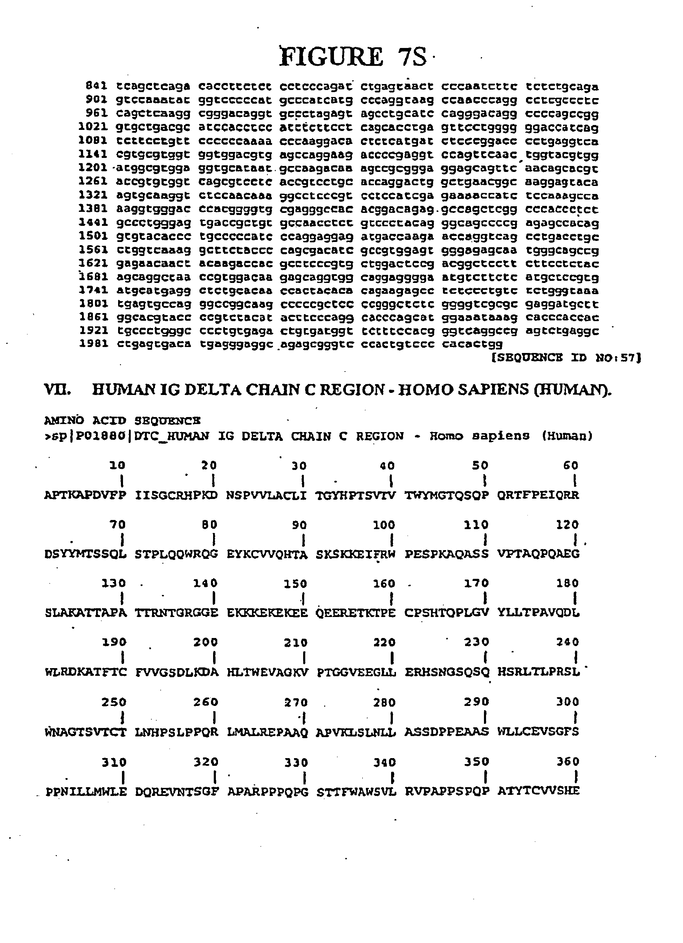

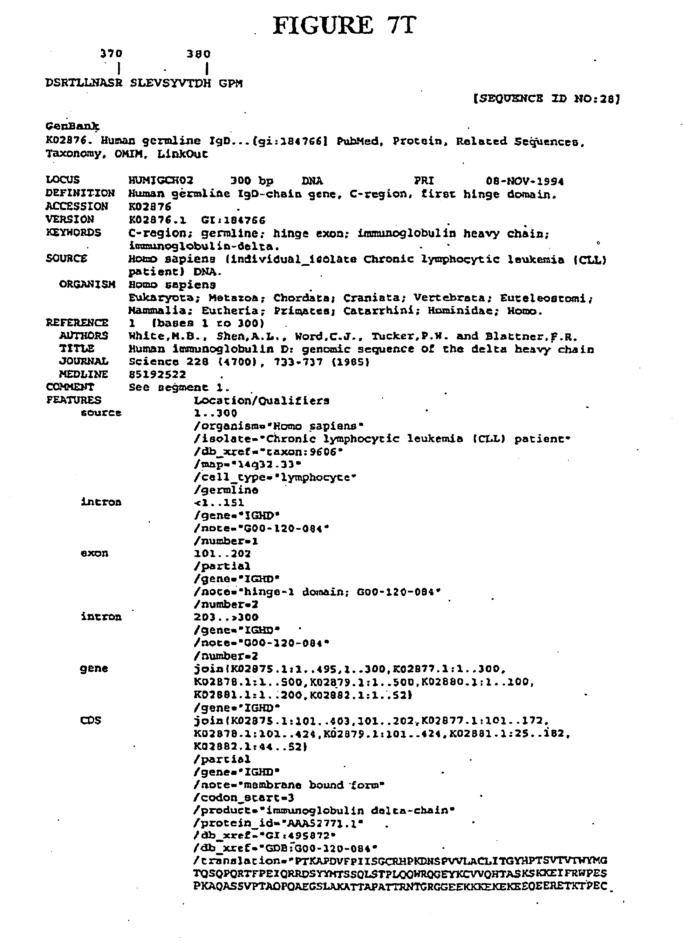

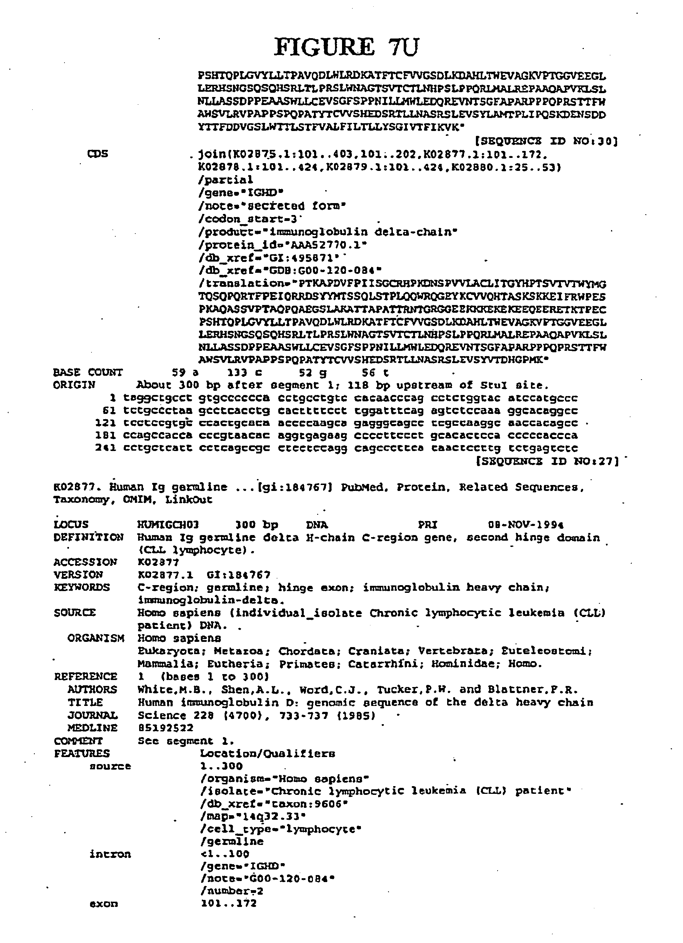

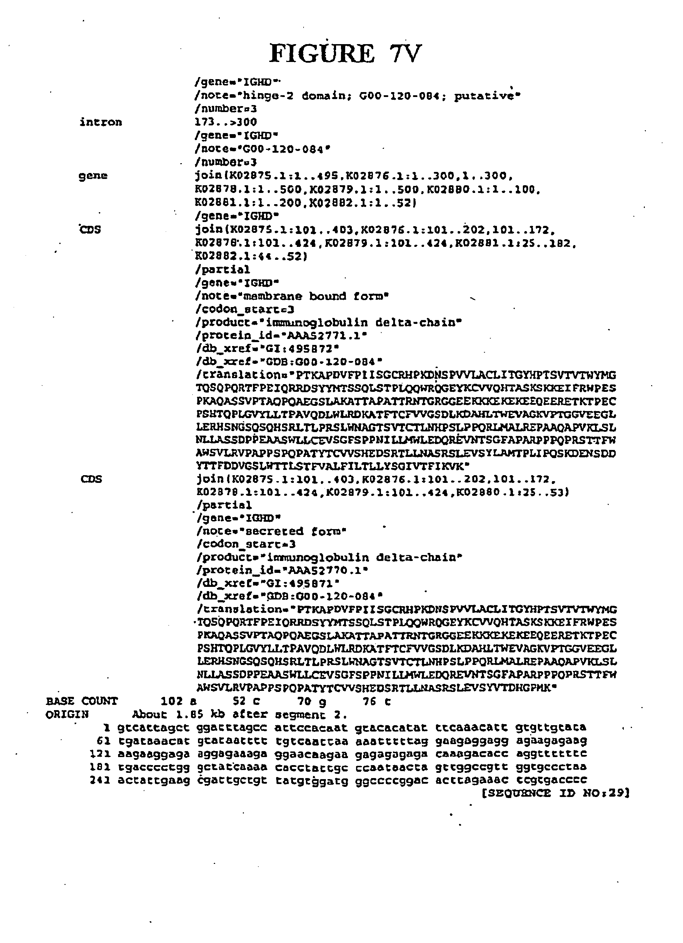

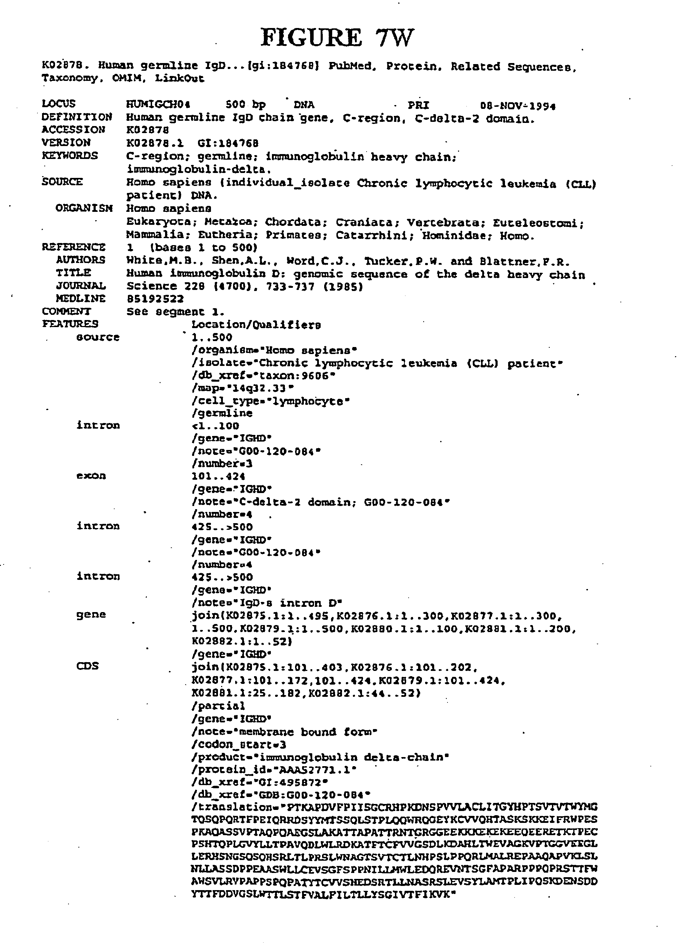

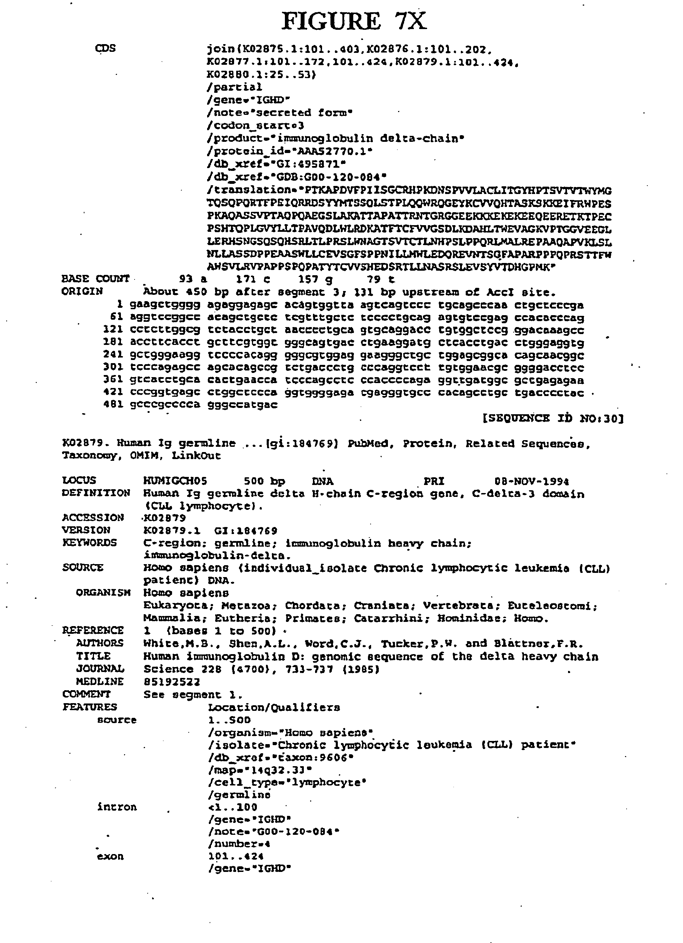

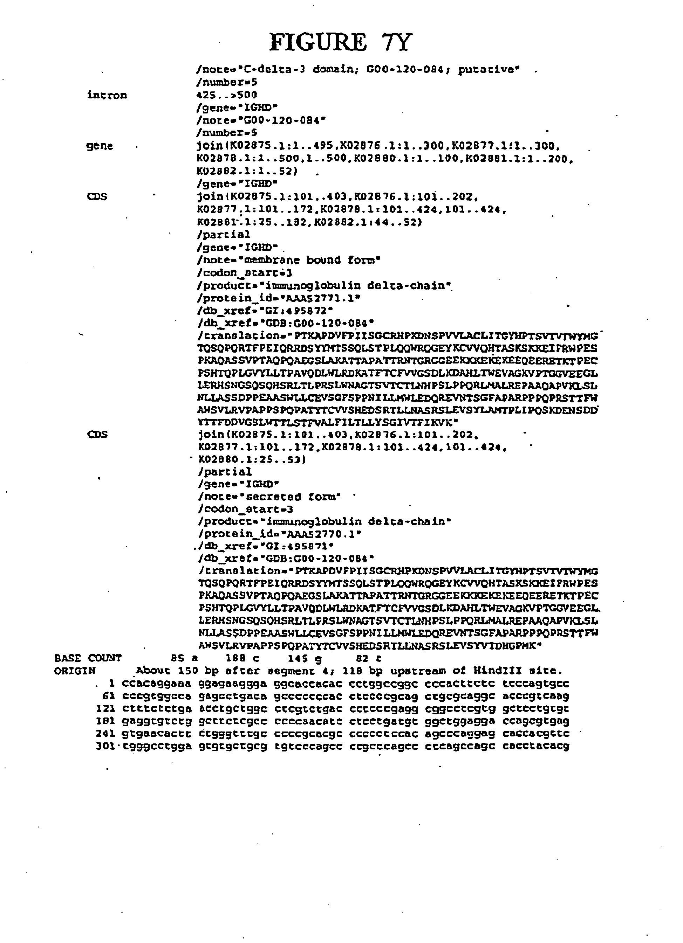

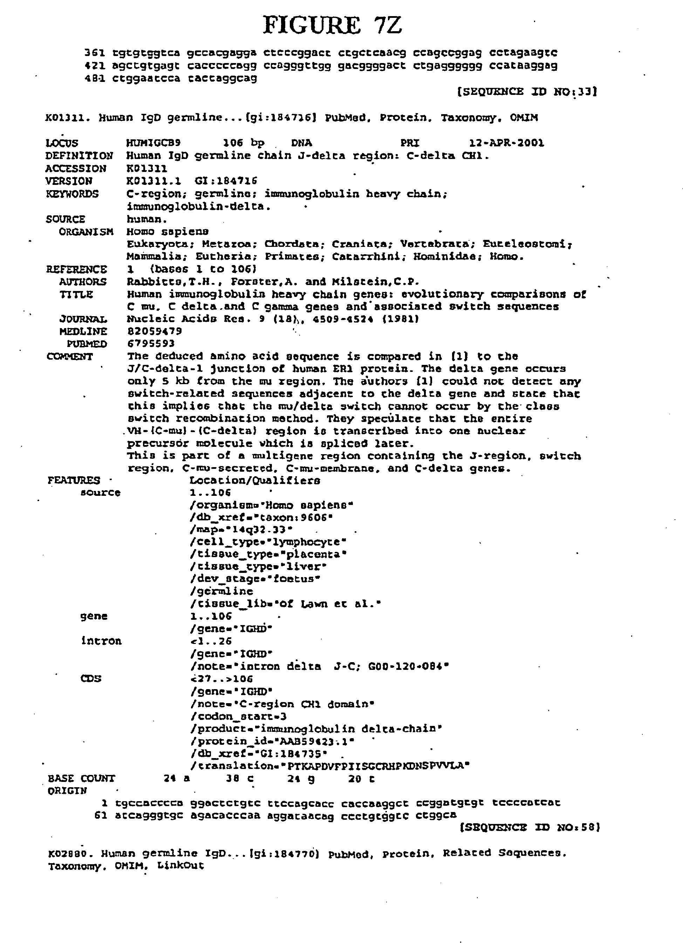

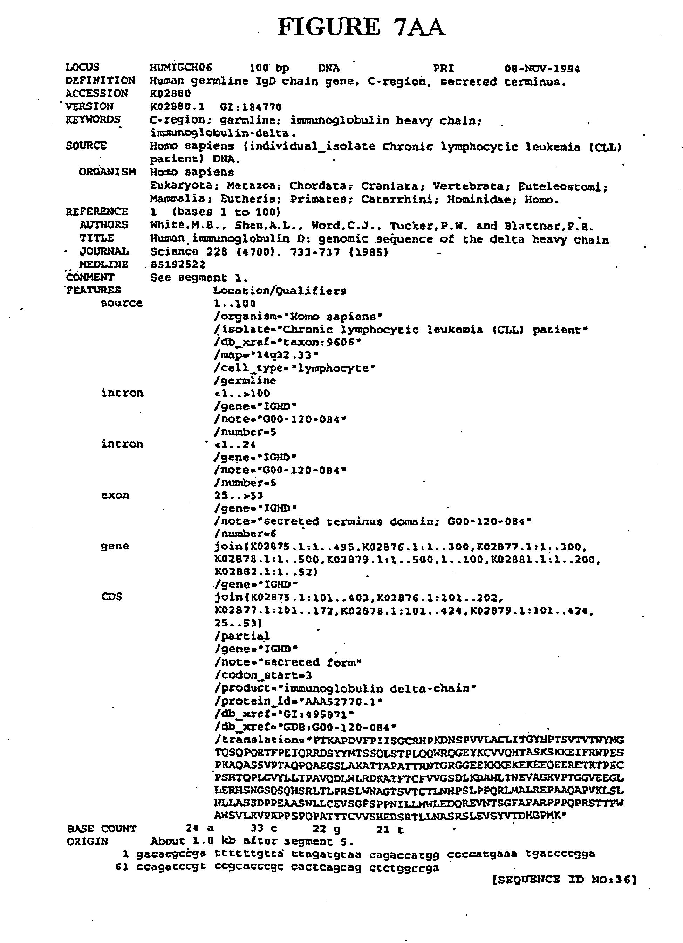

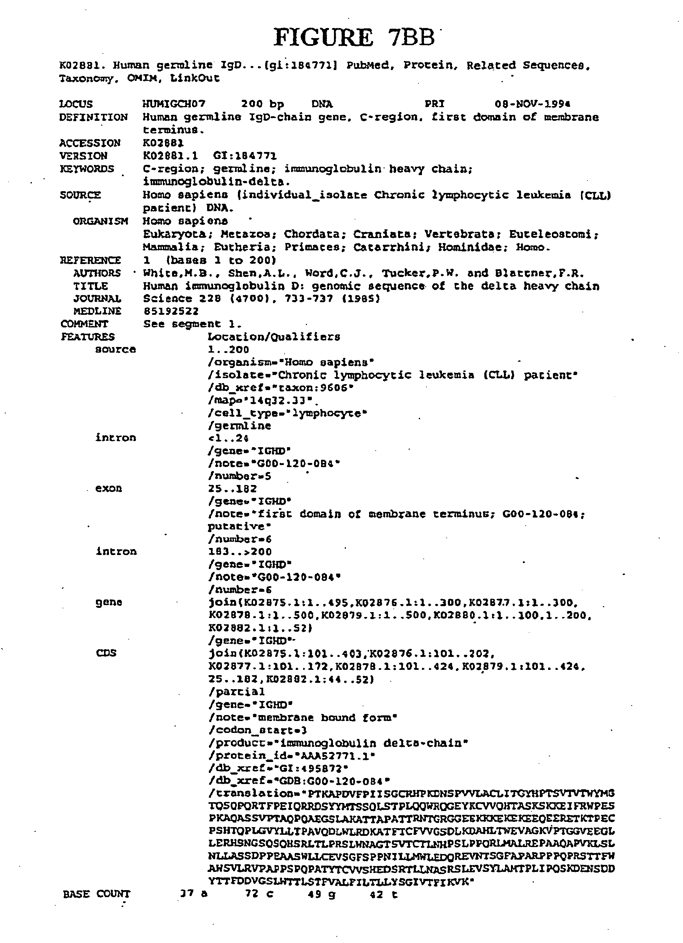

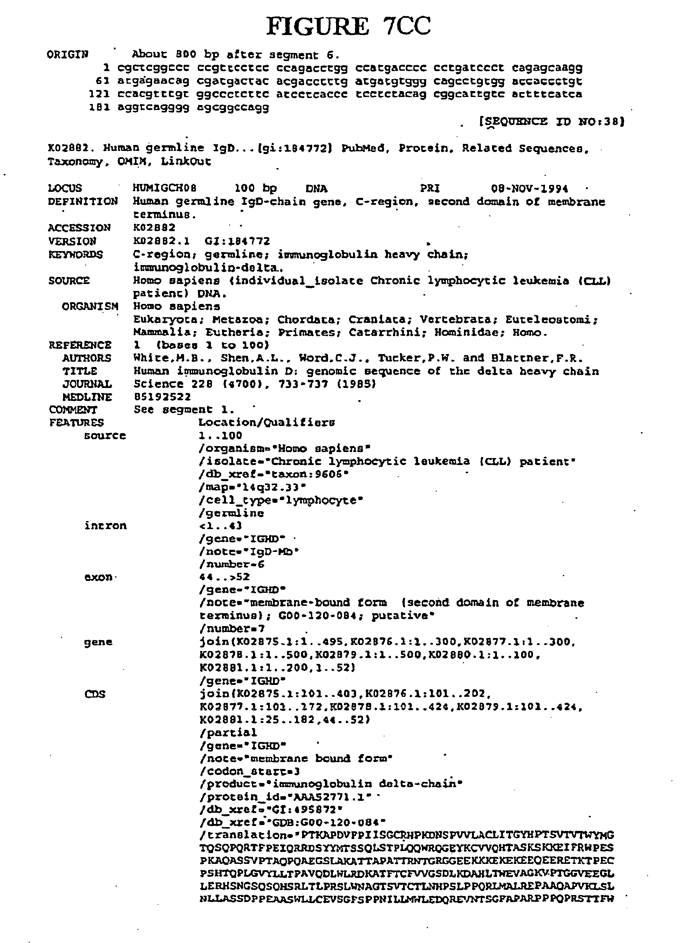

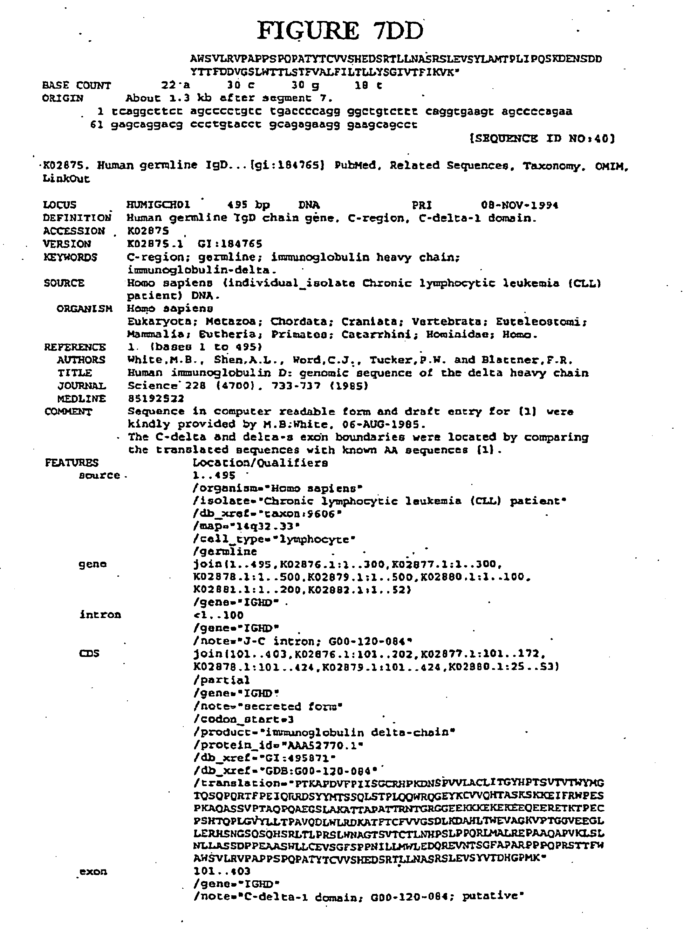

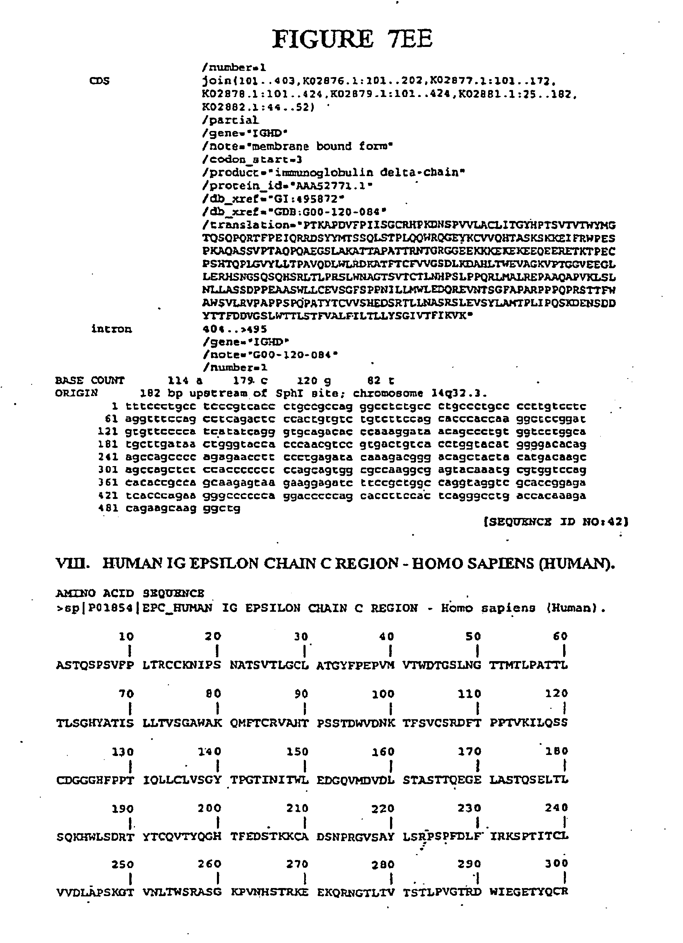

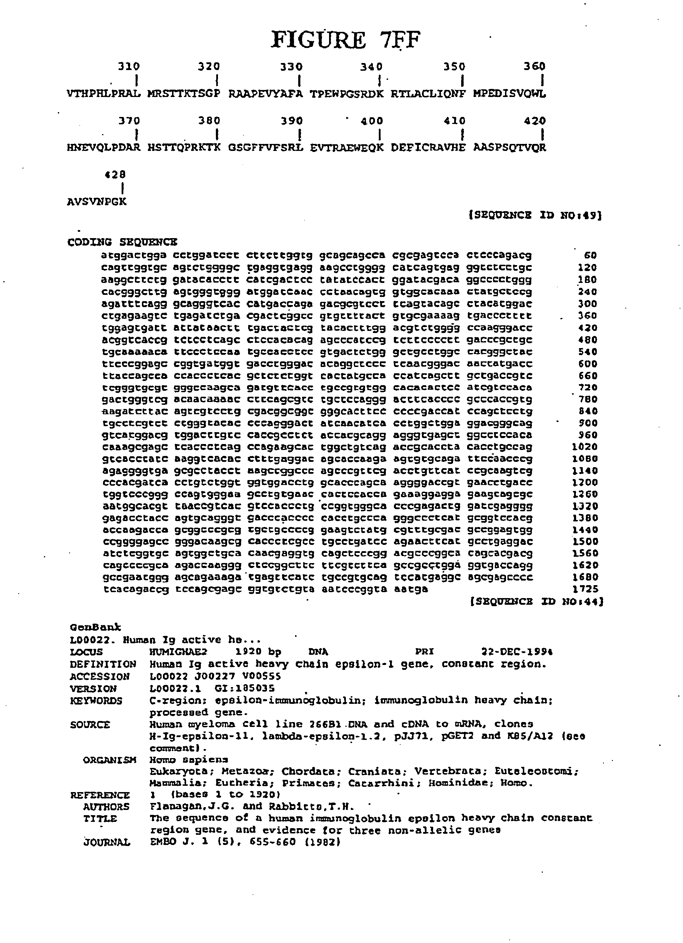

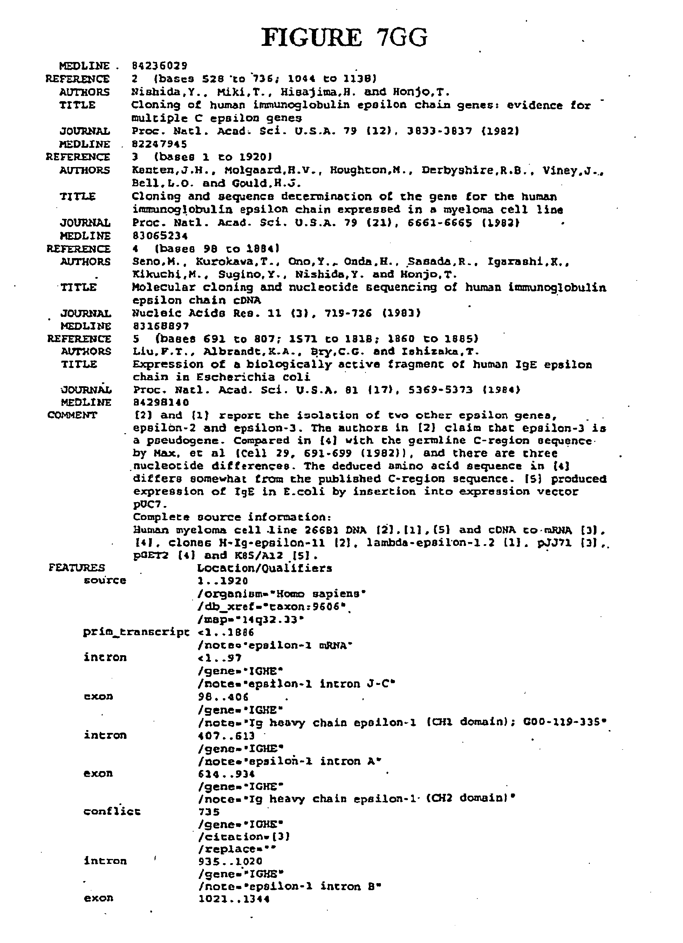

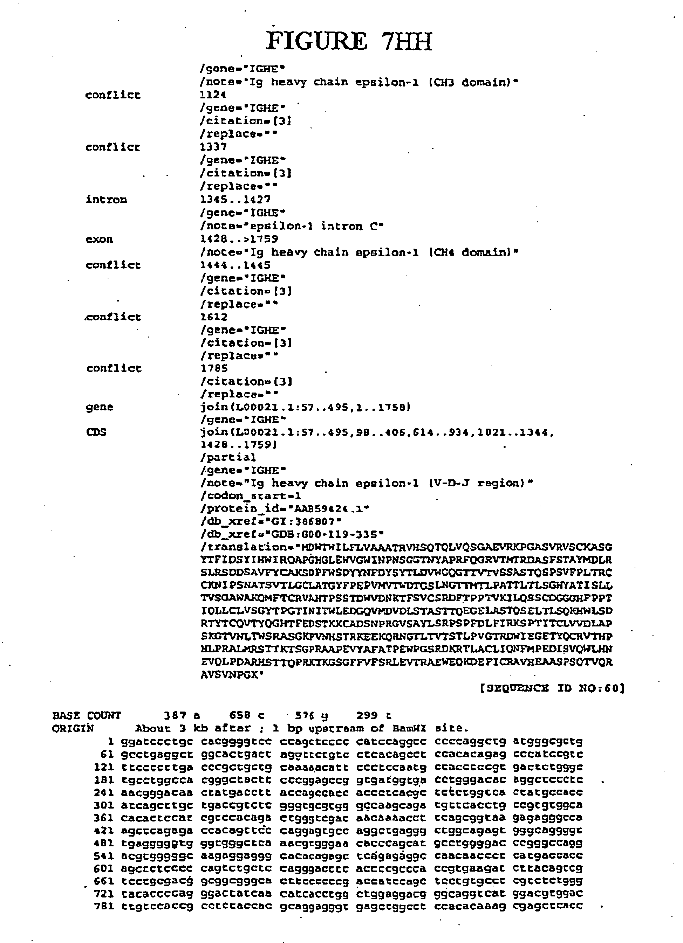

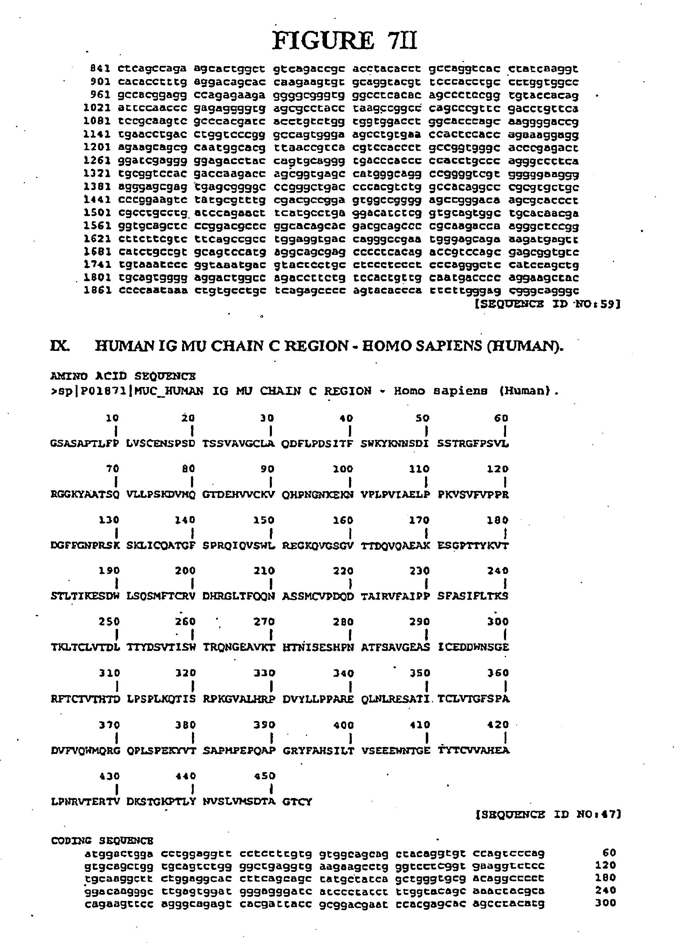

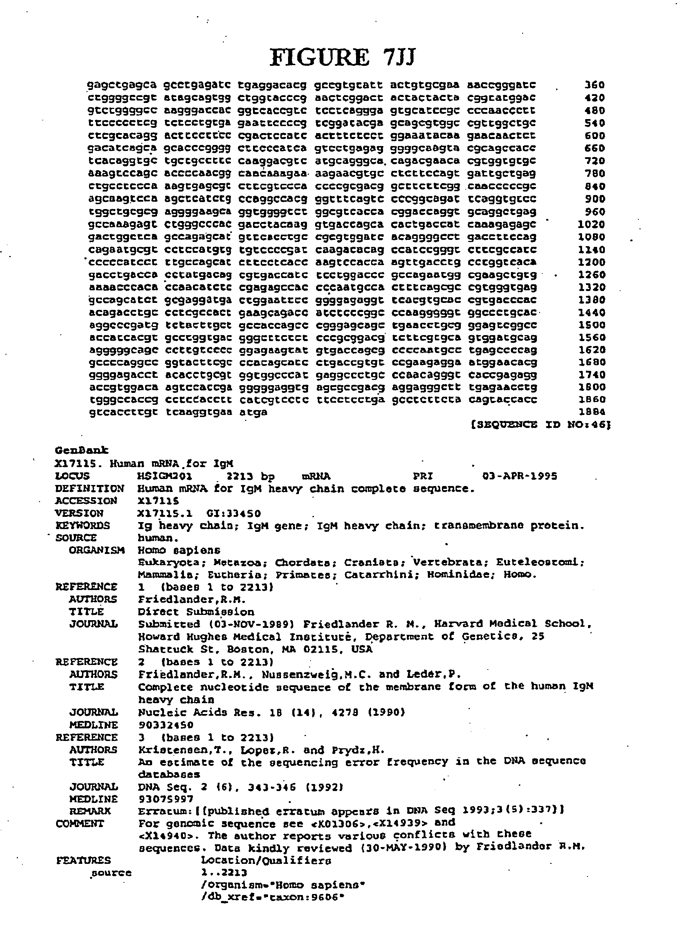

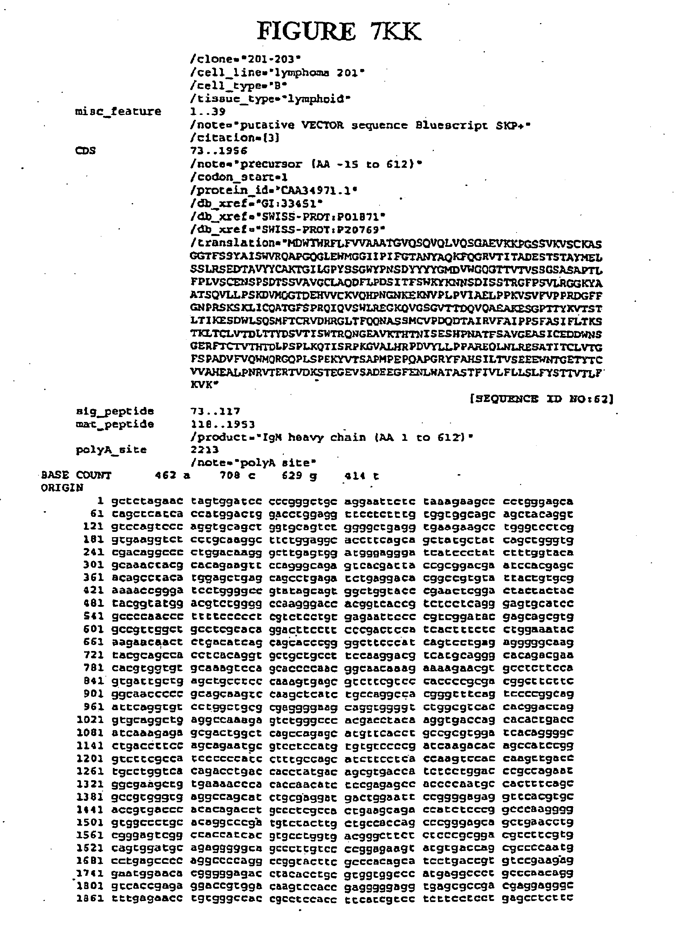

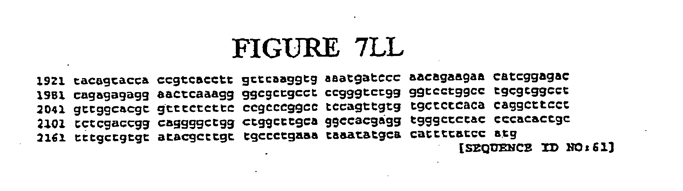

**FIG. 7** shows the coding and amino acid sequences of each of the immunoglobulin genes and proteins listed in **Table 2** [SEQ ID NO:15 through 47 and SEQ ID NO:52 through 62].

**FIG. 8** shows the sequences of plasmids used to transform plants, as described in Example 2, for use in studies of the expression of immunoadhesins of the present invention.

**FIG. 8A** shows the nucleotide [SEQ ID NO:9] and protein [SEQ ID NO:48] sequences for plasmids PSSpICAMHuA2

**FIG. 8B** shows the nucleotide and protein [SEQ ID NO:10] sequence for the bean legumin signal peptide.

**FIG. 8C** shows the nucleotide [SEQ ID NO:11] and amino acid [SEQ ID NO:50] sequence of the protein coding region of pSHuJ.

**FIG. 8D** shows the nucleotide [SEQ ID NO:12] and amino acid [SEQ ID NO:51] sequence of protein coding region of pSHuSC.

**FIG. 8E** shows the nucleotide sequence [SEQ ID NO: 13] of plasmid pBMSP-1.

**FIG. 8F** shows the nucleotide sequence [SEQ ID NO: 14] of plasmid pBMSP-1spJSC.

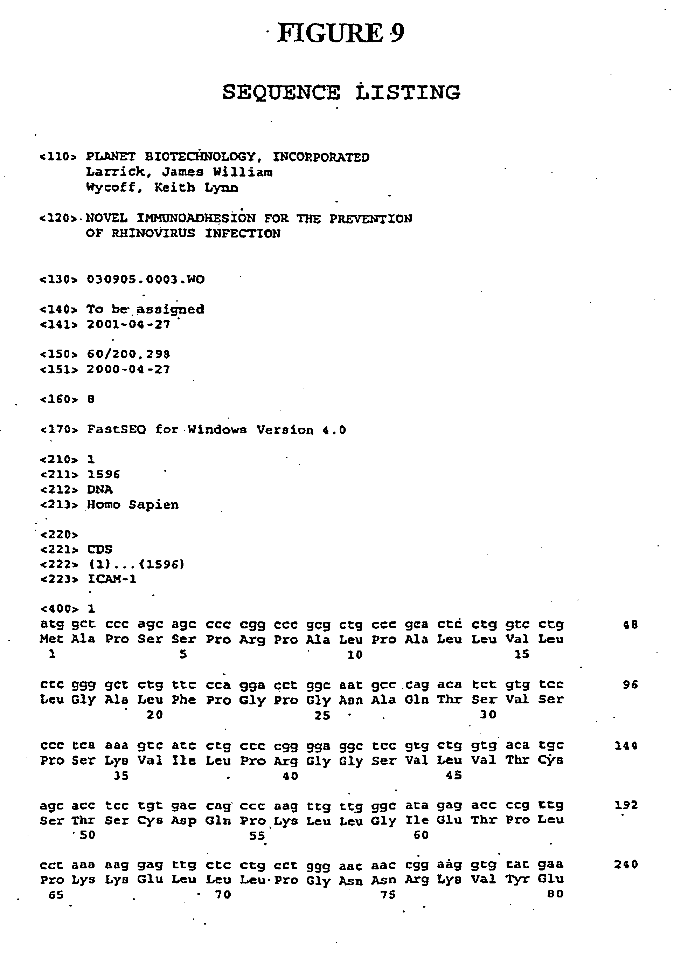

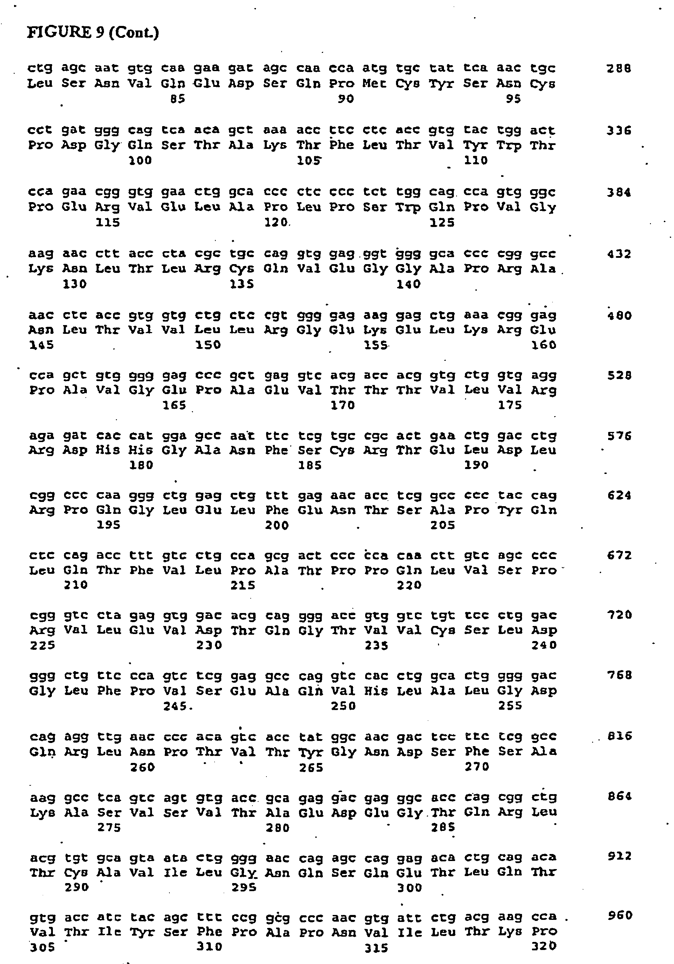

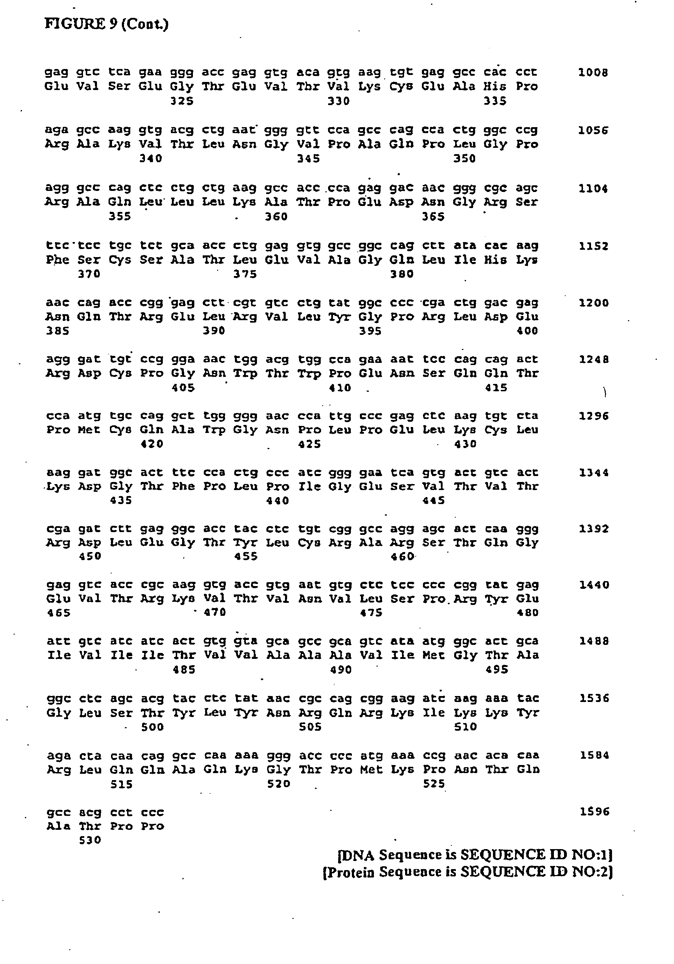

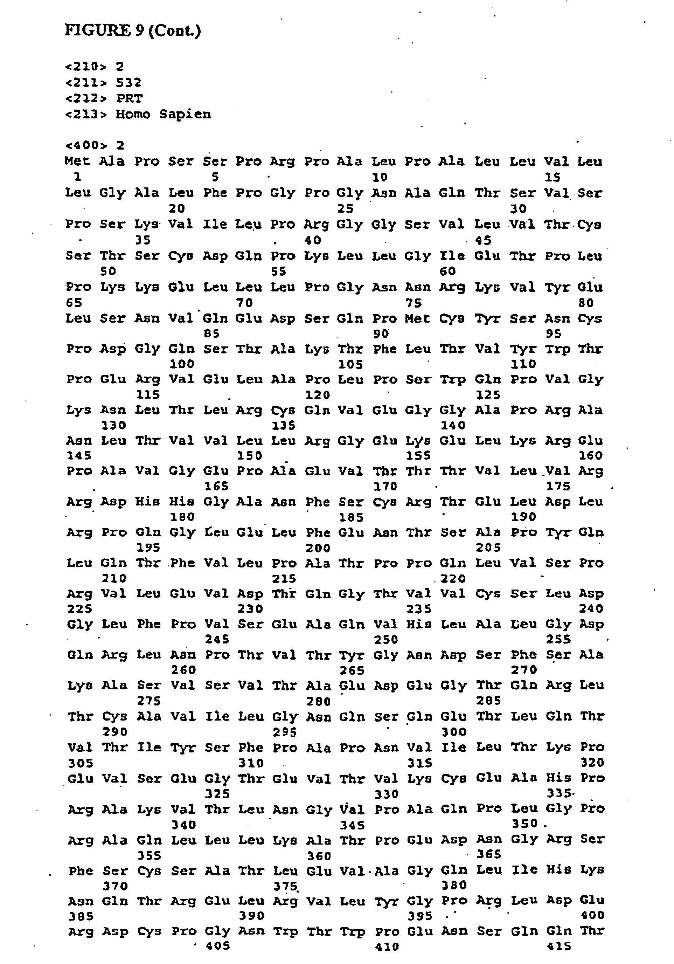

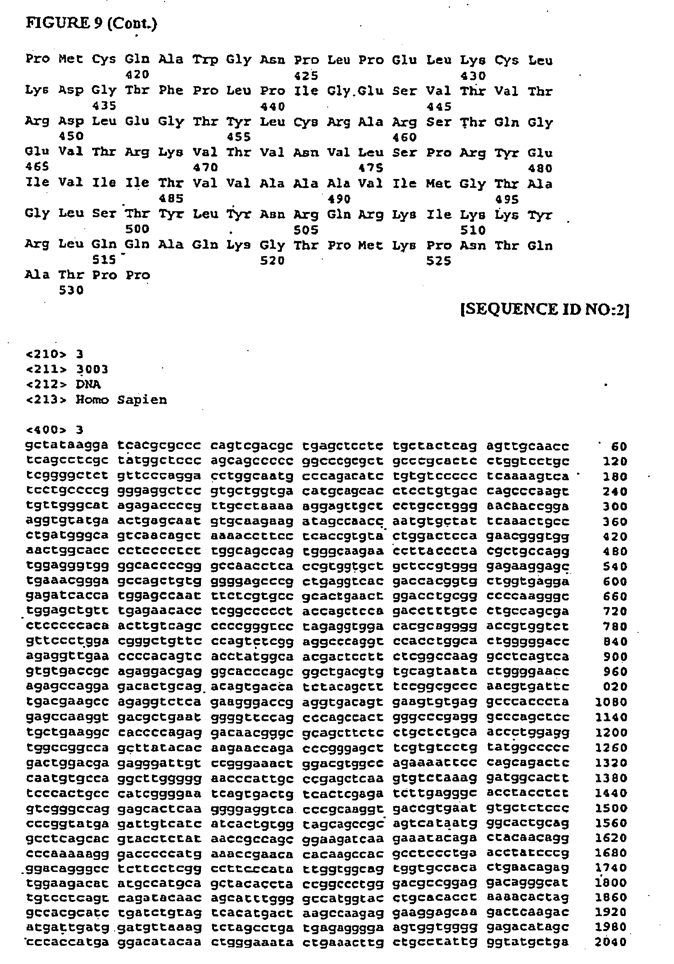

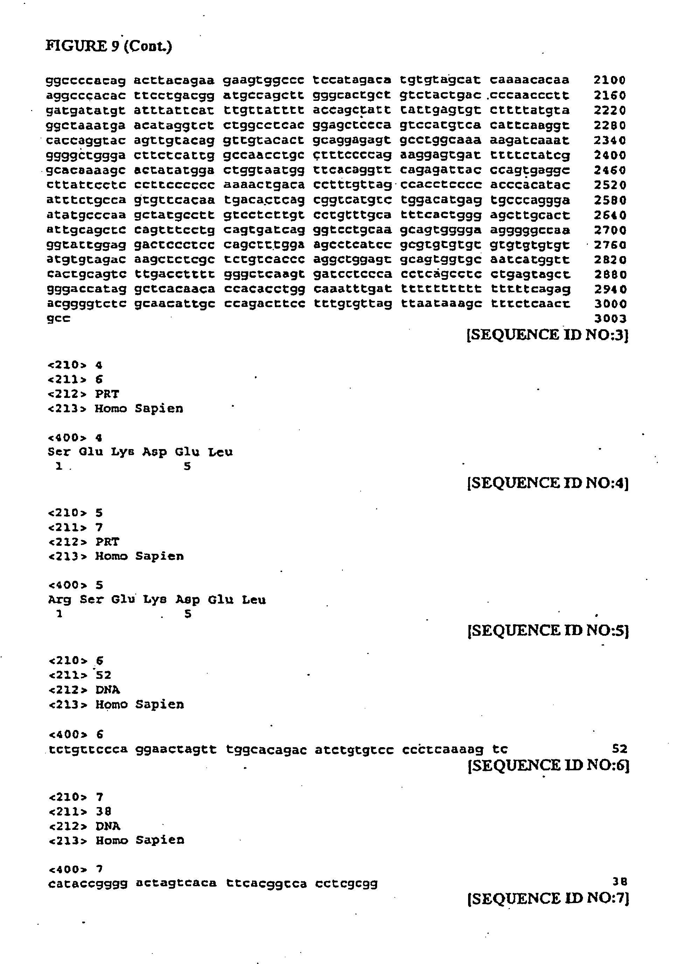

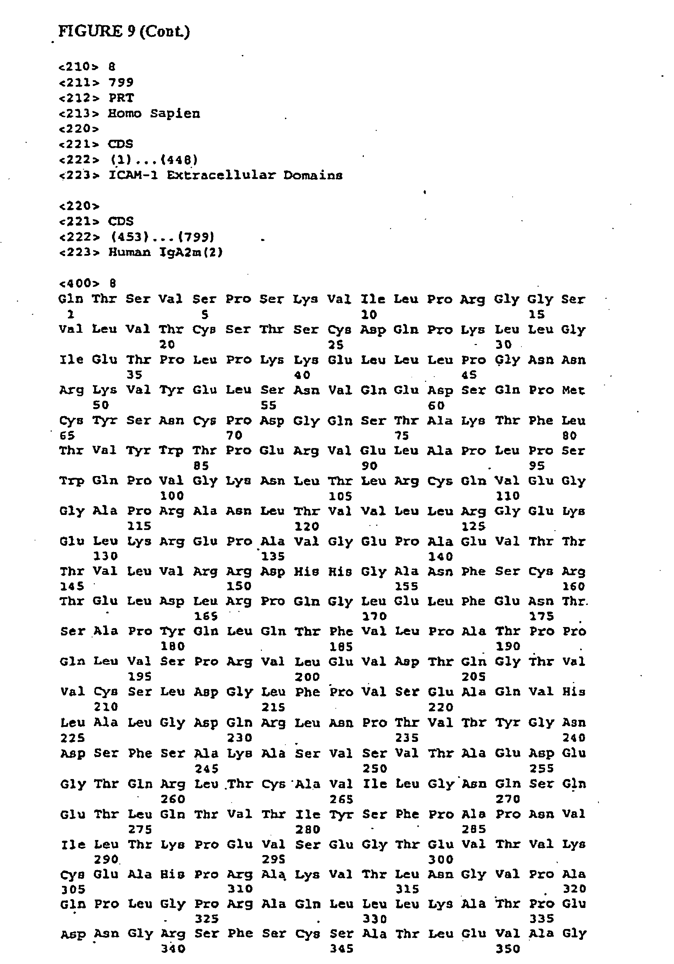

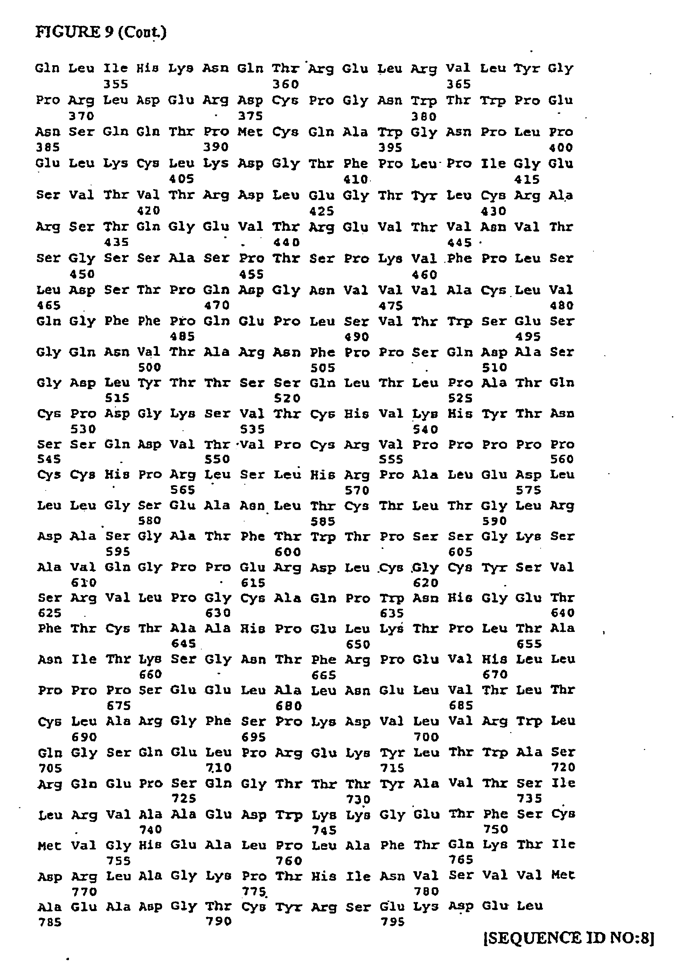

**FIG. 9** contains nucleotide and protein sequences SEQ ID NO: 1; SEQ ID NO:2; SEQ ID NO:3; SEQ ID NO:4; SEQ ID NO:5; SEQ ID NO:6; SEQ ID NO:7; SEQ ID NO:8, for ICAM-1, and human IgA2 and other nucleotide sequences.

**FIG. 10** shows the full nucleotide (SEQ ID NO: 98) and amino acid sequence (SEQ ID NO: 99) of the ATR-IgA2 fusion (an immunoadhesin).

**FIG. 11** shows the sequence (SEQ ID NO: 100) between the T-DNA borders of the plasmid pGPTV-kan-ocs-ATR-IgA2.

**FIG. 12** shows the sequence (SEQ ID NO: 101) between the T-DNA borders of the plasmid pGPTV-hpt-ocs-35SJ/SC.

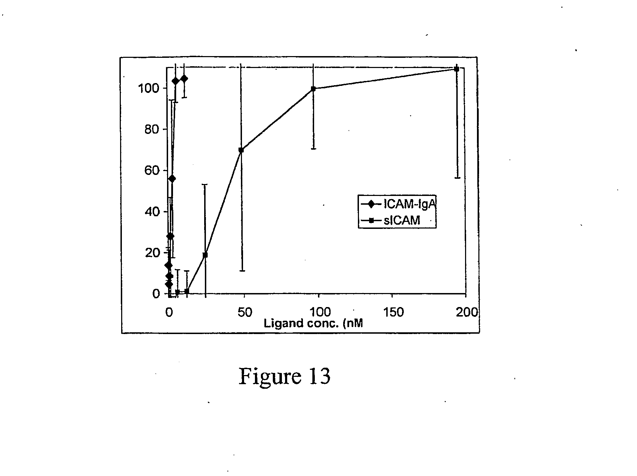

**FIG. 13** shows a cytopathic effect assay. ICAM-1/IgA2 (diamonds) and soluble ICAM-1 (squares) were mixed at various concentrations with human rhinovirus, and then added to HeLa cells. Inhibition of cell death was quantitated at 4 days.

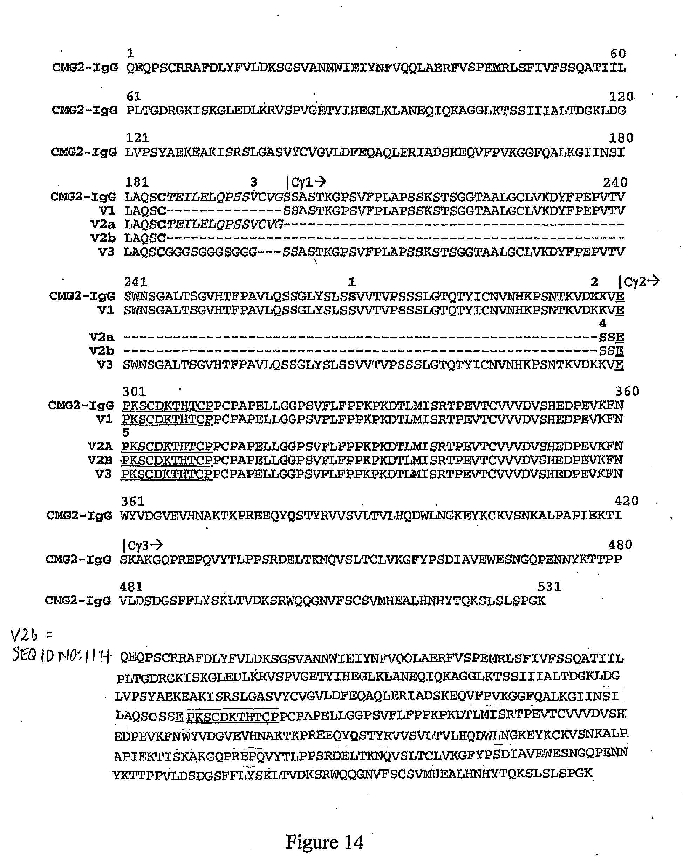

**FIG. 14** shows the amino acid sequence of CMG2-IgG construct and variants. The cysteine in bold type (Cys185 of the sequence above, and Cys218 of the published CMG2 sequence³¹) is the last amino acid of the VWA/I domain. The following 14 amino acids (italicized) are part of CMG2, but not the VWA/I domain. Labels above the sequence indicate the beginnings of IgG1 Cγ1, Cγ2 and Cγ3. Underlined sequence is the IgG1 hinge region. Sequences of variants (**V1**, **V2a, V2b** and **V3)** are indicated, with dashes representing deleted amino acids. Numbers over amino acids in bold type indicate amino termini of sequenced fragments (described in the text).

**FIG. 15** is a silver-stained gel of protein-A purified CMG2-IgG and 1H IgG. Lanes 1 and 4, protein size standards; lane 2, reduced CMG2-IgG; lane 3, reduced 1H; lanes 5 & 6, non-reduced CMG2-IgG from two separate preparations; lane 7, non-reduced 1H. Recombinant protein samples contain 50 ng of protein per lane. Cartoon representation of CMG2-IgG immunoadhesins and possible degradation products shown to the right.

**FIG. 16** is a Coomassie-stained SDS-PAGE of CMG2-IgG and variants. Lanes 1-7 run under non-reducing conditions. Lanes 8-13 run under reducing conditions (100 mM DTT). Lanes 1 and 8, original CMG2-IgG; Lanes 2 and 9, V1; Lanes 3 and 10, V2a; Lanes 4 and 11, V2b, Lanes 5 and 12, V3; Lanes 6 and 13, CMG2-IgG + kappa chain; Lane 7, molecular weight markers.

**FIG. 17** is an elution profile of the plant-derived CMG2-IgG V2b, fractionated by size exclusion chromatography. Protein-A-purified CMG2-IgG V2b from tobacco plants was applied to a Tosoh G4000SW column and eluted with PBS. Protein was monitored by absorbance at 280nm. The column was calibrated with molecular weight standards prior to the run. Retention times and calculated molecular weights are indicated on the profiles. The internal standard p-aminobenzoic acid (peak with a retention time of 15.3 minutes) was added to the sample prior to separation.

**FIG. 18** shows the neutralization of Lethal Toxin cytotoxicity by CMG2-IgG variants. RAW 264.7 mouse macrophage-like cells are exposed to PA + LF plus increasing concentrations (0 to 1.25 µg/ml) of either original CMG2-IgG (squares), CMG2-IgG V2b (diamonds), or an irrelevant plant-made IgG (circles). Each point is the mean of three replicates.

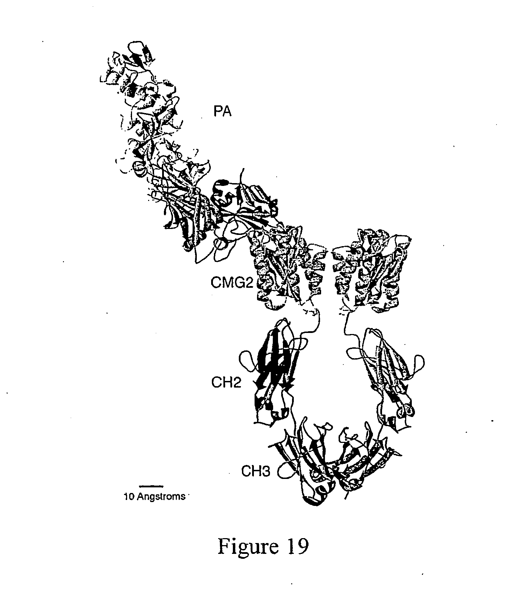

**FIG. 19** is a model of CMG2-IgG V2b. Hypothetical depiction of a homodimer of the antitoxin, with one molecule of PA bound. The antitoxin is depicted as held together by non-covalent interactions between Cγ3 domains, although there may be a disulfide bond in the hinge region (between CMG2 and Cγ2) in a portion of the molecules. Bar indicates scale. Structure files are from the Protein Data Base, and molecular graphics images were produced using the UCSF Chimera package from the Resource for Biocomputing, Visualization, and Informatics at the University of California, San Francisco (supported by NIH P41 RR-01081).⁵⁴

**FIG. 20** shows CMG2-kappa variants. CMG2-kappa was expressed in N. benthamiana with either CMG2-IgG V1 or CMG2-IgG V3 and proteins purified by Protein A were subject to SDS-PAGE and stained with Coomassie. Lanes 1 & 2 run under non-reducing conditions. Lanes 3 & 4 run under reducing conditions (100 mM DTT); Lane 5, molecular weight markers.

### BRIEF DESCRIPTION OF THE TABLES

Table 1 lists the boundaries for five extracellular domains of ICAM-1.

Table 2 shows the GenBank Accession numbers of immunoglobulin heavy chain genes and the proteins encoded by the genes.

Table 3 provides a list of the examples of molecules having ICAM-1 homology that can be used to create other chimeric rhinovirus toxin receptor proteins.

Table 4 shows the known botulinum receptors and putative binding domains.

Table 5 shows the study design for the randomized rhinovirus challenge studies performed in Example 9.

Table 6 shows chimeric anthrax toxin receptor production in transiently transfected N. benthamiana leaves as described in Example 16.

Table 7 shows the calculated IC₅₀ and molar ratio of antitoxin to PA at the IC₅₀ for Example 18.

Table 8 describes the structure of the various CMG-IgG chimera variants in Example 19.

### DETAILED DESCRIPTION

### Definitions

As used herein, the following abbreviations and terms include, but are not necessarily limited to, the following definitions.

The practice of the present invention will employ, unless otherwise indicated, conventional techniques of immunology, molecular biology, microbiology, cell biology and recombinant DNA, which are within the skill of the art. See, e.g., Sambrook, et al., Molecular Cloning: A Laboratory Manual, 2nd edition (1989); Current Protocols In Molecular Biology (F. M. Ausubel, et al. eds., (1987)); the series Methods In Enzymology (Academic Press, Inc.); M. J. MacPherson, et al., eds. Pcr 2: A Practical Approach (1995); Harlow and Lane, eds, Antibodies: A Laboratory Manual (1988), and H. Jones, Methods In Molecular Biology vol. 49, "Plant Gene Transfer And Expression Protocols" (1995).

Immunoadhesin: A complex containing a chimeric toxin receptor protein molecule fused to a portion of an immunoglobulin constant region, and optionally containing secretory component and J chain.

Chimeric toxin receptor protein: A toxin receptor-based protein having at least a portion of its amino acid sequence derived from a toxin receptor and at least a portion derived from an immunoglobulin complex.

Toxin receptor: As used herein, the term refers to any polypeptide that binds pathogen antigens as defined herein, toxins, or any proteins, lipoproteins, glycoproteins, polysaccharides or lipopolysaccharides that exert or lead to exertion of a pathogenic effect with an affinity and avidity sufficient to allow a chimeric toxin receptor protein to act as a receptor decoy. For example, a toxin receptor may be a viral attachment receptor such as ICAM-1, a receptor for human rhinovirus, or a receptor for a bacterial toxin, such as one of the receptors for anthrax protective antigen or botulinum toxin. The toxin receptors as used herein shall at a minimum contain the functional elements for binding of toxins/pathogen components but may optionally also include one or more additional polypeptides.

Immunoglobulin molecule or Antibody: A polypeptide or multimeric protein containing the immunologically active portions of an immunoglobulin heavy chain and immunoglobulin light chain covalently coupled together and capable of specifically combining with antigen. The immunoglobulins or antibody molecules are a large family of molecules that include several types of molecules such as IgM, IgD, IgG, IgA, secretory IgA (SIgA), and IgE.

Immunoglobulin complex: A polypeptide complex that can include a portion of an immunoglobulin heavy chain or both a portion of an immunoglobulin heavy chain and an immunoglobulin light chain. The two components can be associated with each other via a variety of different means, including covalent linkages such as disulfide bonds.

Portion of an Immunoglobulin heavy chain: As used herein, the term refers to that region of a heavy chain which is necessary for conferring at least one of the following properties on the chimeric toxin receptor proteins as described herein: ability to multimerize, effector functions, ability to be purified by Protein G or A, or improved pharmacokinetics. Typically, this includes at least a portion of the constant region.

Portion of an Immunoglobulin light chain: As used herein, the term refers to that region of a light chain which is necessary for increasing stability of the described chimeric toxin receptor protein and thus increasing production yield. Typically, this includes at least a portion of the constant region.

Heavy chain constant region: A polypeptide that contains at least a portion of the heavy chain immunoglobulin constant region. Typically, in its native form, IgG, IgD and IgA immunoglobulin heavy chain contain three constant regions joined to one variable region. IgM and IgE contain four constant regions joined to one variable region. As described herein, the constant regions are numbered sequentially from the region proximal to the variable domain. For example, in IgG, IgD, and IgA heavy chains, the regions are named as follows: variable region, constant region 1, constant region 2, constant region 3. For IgM and IgE, the regions are named as follows: variable region, constant region 1, constant region 2, constant region 3 and constant region 4.

Chimeric immunoglobulin heavy chain: An immunoglobulin derived heavy chain having at least a portion of its amino acid sequence derived from an immunoglobulin heavy chain of a different isotype or subtype or some other peptide, polypeptide or protein. Typically, a chimeric immunoglobulin heavy chain has its amino acid residue sequence derived from at least two different isotypes or subtypes of immunoglobulin heavy chain.

J chain: A polypeptide that is involved in the polymerization of immunoglobulins and transport of polymerized immunoglobulins through epithelial cells. See, The Immunoglobulin Helper: The J Chain in Immunoglobulin Genes, at pg. 345, Academic Press (1989). J chain is found in pentameric IgM and dimeric IgA and typically attached via disulfide bonds. J chain has been studied in both mouse and human.

Secretory component (SC): A component of secretory immunoglobulins that helps to protect the immunoglobulin against inactivating agents thereby increasing the biological effectiveness of secretory immunoglobulin. The secretory component may be from any mammal or rodent including mouse or human.

Linker: As used herein, the term refers to any polypeptide sequence used to facilitate the folding and stability of a recombinantly produced polypeptide. Preferably, this linker is a flexible linker, for example, one composed of a polypeptide sequence such as (Gly3Ser)3 or (Gly4Ser)3.

Transgenic plant: Genetically engineered plant or progeny of genetically engineered plants. The transgenic plant usually contains material from at least one unrelated organism, such as a virus, another plant or animal.

Monocots: Flowering plants whose embryos have one cotyledon or seed leaf. Examples of monocots are: lilies; grasses; corn; grains, including oats, wheat and barley; orchids; irises; onions and palms.

Dicots: Flowering plants whose embryos have two seed halves or cotyledons. Examples of dicots are: tobacco; tomato; the legumes including alfalfa; oaks; maples; roses; mints; squashes; daisies; walnuts; cacti; violets and buttercups.

Glycosylation: The modification of a protein by oligosaccharides. See, Marshall, Ann. Rev. Biochem., 41:673 (1972) and Marshall, Biochem. Soc. Symp., 40:17 (1974) for a general review of the polypeptide sequences that function as glycosylation signals. These signals are recognized in both mammalian and in plant cells.

Plant-specific glycosylation: The glycosylation pattern found on plant-expressed proteins, which is different from that found in proteins made in mammalian or insect cells. Proteins expressed in plants or plant cells have a different pattern of glycosylation than do proteins expressed in other types of cells, including mammalian cells and insect cells. Detailed studies characterizing plant-specific glycosylation and comparing it with glycosylation in other cell types have been performed by Cabanes-Macheteau et al., Glycobiology 9(4):365-372 (1999), Lerouge et al., Plant Molecular Biology 38:31-48 (1998) and Altmann, Glycoconjugate J. 14:643-646 (1997). Plant-specific glycosylation generates glycans that have xylose linked .beta.(1,2) to mannose. Neither mammalian nor insect glycosylation generate xylose linked .beta.(1,2) to mannose. Plants do not have a sialic acid linked to the terminus of the glycan, whereas mammalian cells do. In addition, plant-specific glycosylation results in a fucose linked .alpha.(1,3) to the proximal GlcNAc, while glycosylation in mammalian cells results in a fucose linked .alpha.(1,6) to the proximal GlcNAc.

Pathogen antigen: Any molecule, e.g., toxin, that exerts or leads to exertion of a pathogenic effect. The pathogen antigen may or may not be capable of stimulating the formation of antibodies.

Anthrax toxin receptor: As used herein, the term refers to any polypeptide which binds protective antigen (PA), one of the three interacting proteins used by Bacillus anthracis to destroy host cells, with an affinity and avidity sufficient to act to allow a chimeric anthrax toxin receptor protein to act as a receptor decoy. Two endogenous cellular anthrax toxin receptors have been identified in the art: Anthrax toxin receptor/Tumor endothelial marker 8 (ATR/TEM8) (Bradley et al., 2001) and Capillary morphogenesis factor 2 (CMG2) (Scobie et al., 2001). The extracellular domain of these ATRs contains a VWA/I domain, which is described below. The anthrax toxin receptor as used herein shall at a minimum contain the functional elements for binding of PA but may optionally also include one of more additional polypeptides (for example, ones that improve use of the anthrax toxin receptor as a receptor decoy) which may be generated by splicing the coding sequences for the one of more additional polypeptides into the anthrax toxin receptor coding sequences.

VWA/I domain: As used herein, the term refers to a Van Willebrand Factor type A domain (VWA domain), also commonly named integrin inserted domain (I domain), which is a domain in the extracellular domain of the two known anthrax toxin receptors, TEM8 and CMG2 and believed to contain a metal ion-dependent adhesion site (MIDAS) essential for proper PA binding to the receptor.

Chimeric Anthrax toxin receptor protein: An anthrax toxin receptor-based protein having at least a portion of its amino acid sequence derived from an anthrax toxin receptor and at least a portion derived from an immunoglobulin complex. The immunoglobulin complex may contain only a portion of an immunoglobulin heavy chain or it may contain both a portion of a heavy chain and a portion of a light chain.

Botulinum toxin receptor: Any polypeptide which binds a botulinum toxin with an affinity and avidity sufficient to allow a chimeric anthrax toxin receptor protein to act as a receptor decoy. Examples include SV2 and synaptotagmin.

Effective amount: An effective amount of an immunoadhesin of the present invention is sufficient to detectably inhibit viral or bacterial infection (as the case may be), cytotoxicity or replication; or to reduce the severity or length of infection.

Construct or Vector: An artificially assembled DNA segment to be transferred into a target plant tissue or cell. Typically, the construct will include the gene or genes of a particular interest, a marker gene and appropriate control sequences. The term "plasmid" refers to an autonomous, self-replicating extrachromosomal DNA molecule. Plasmid constructs containing suitable regulatory elements are also referred to as "expression cassettes." In a preferred embodiment, a plasmid construct also contains a screening or selectable marker, for example an antibiotic resistance gene.

Selectable marker: A gene that encodes a product that allows the growth of transgenic tissue on a selective medium. Non-limiting examples of selectable markers include genes encoding for antibiotic resistance, e.g., ampicillin, kanamycin, or the like. Other selectable markers will be known to those of skill in the art.

### Chimeric Toxin Receptor Proteins with Improved Stability

The chimeric toxin receptor proteins described herein have toxin receptor associated with an immunoglobulin complex containing an immunoglobulin heavy chain and an immunoglobulin light chain. A toxin receptor protein may be associated with the heavy chain, the light chain, or both. In some embodiments, a J chain, either alone or associated with secretory component is associated with the chimeric toxin receptor protein to form an immunoadhesin. In other embodiments, the chimeric toxin receptor protein has plant-specific glycosylation.

Without wishing to be bound by theory, it is believed that the chimeric toxin receptor proteins described herein will have significant advantages over existing monoclonal antibody or receptor decoy-based prophylactics or therapies including higher activity due to multivalency of the receptor, the effector functions of the immunoglobulin Fc, improved pharmacokinetics due to the Fc, and lower production cost when made in plants..

The advantage of multivalency has been demonstrated by fusions of the extracellular domains of human ICAM-1 with immunoglobulin heavy chains (Martin, S., Casasnovas, J. M., Staunton, D. E. & Springer, T. A. Efficient neutralization and disruption of rhinovirus by chimeric ICAM- 1/immunoglobulin molecules. J Virol 67, 3561-8 (1993)). ICAM-1 is the cell-surface receptor for human rhinovirus (HRV), a common cold virus. Chimeric immunoadhesins are much more effective than soluble ICAM-1 in inhibiting HRV binding to cells and disrupting the conformation of the virus capsid (Martin et al. J Virol 67, 3561-8 (1993)). An ICAM-1/IgA2 chimera (RhinoRx) that has more than 10 times the specific activity of soluble ICAM-1 in protecting cells from the cytopathic effect of HRV has been expressed in transgenic tobacco.

The presence of a Fc region should confer immunoglobulin effector functions, such as the ability to mediate the specific lysis of cells in the presence of complement. The heavy chain constant region domains of the immunoglobulins confer various properties known as antibody effector functions on a particular molecule containing that domain. Example effector functions include complement fixation, placental transfer, binding to staphyloccal protein, binding to streptococcal protein G, binding to mononuclear cells, neutrophils or mast cells and basophils. The association of particular domains and particular immunoglobulin isotypes with these effector functions is well known and for example, described in Immunology, Roitt et al., Mosby St. Louis, Mo. (1993 3rd Ed.)

In addition, binding of the Fc to the FcRn should allow the immunoadhesins to persist in the circulation much longer (Ober, R. J., Martinez, C., Vaccaro, C., Zhou, J. & Ward, E. S. Visualizing the Site and Dynamics of IgG Salvage by the MHC Class I-Related Receptor, FcRn. J Immunol 172, 2021-2029 (2004)). This may allow the antitoxin to be used as a prophylactic.

The increase in productivity of the chimeric toxin receptor protein and the immunoadhesin molecule or complex when produced in the host cell, host tissue or host organism, plant cell, plant tissue or intact plant, leads to higher yields of the molecules and lower production cost when processed. Such greater productivity may be the result of greater efficiency in producing the constituent chains of the chimeric toxin receptor protein and immunoadhesin or immunoadhesin complex and/or greater efficiency of assembly of the immunoadhesin complex. In the case especially of a chimeric toxin receptor protein or immunoadhesin comprising both a portion of an immunoglobulin heavy chain and a portion of a light chain, increases in apparent level of activity of the purified or partially purified immunoadhesin may be due to an increase in availability of constituent chains and/or greater efficiency in assembly of the complex, and/or multivalency of the receptor or a combination of all of the foregoing.

One of skill will understand that the chimeric toxin receptor proteins described herein can be modified as necessary to remove protease-sensitive domains and to include linkers when necessary to improve function and stability. Methods for introducing such modifications are well known in the art; however, the exact nature of such modifications (for example, which domains to remove, length and location of linkers) is not known *a priori.*

### Toxin Receptor

A toxin receptor is any polypeptide that binds pathogen antigens, toxins or any proteins that exert or lead to exertion of a pathogenic effect with an affinity and avidity sufficient to allow a chimeric toxin receptor protein to act as a receptor decoy. For example, in antiviral or antibacterial embodiments, one or more receptor proteins effective to bind a virus, bacterium or bacteria of interest or subcomponent thereof, such as a protein produced by the virus or bacteria and required for the virus or bacteria to initiate processes that exert or lead to exertion of its pathogenic effect, are used. Many such receptor proteins are known and can be implemented for use in appropriate aspects of the invention by those of ordinary skill in the art without exercising undue experimentation.

Anthrax receptors and suitable domains for use in this invention are described in more details in the section entitled "Anthrax Chimeric Toxin Receptors".

### Immunoglobulin Complex

The immunoglobulin complex can include a portion of an immunoglobulin heavy chain or a portion of both an immunoglobulin heavy chain and a light chain. The two components can be associated with each other via a variety of different means, including covalent linkages such as disulfide bonds. The immunoglobulin complexes are associated with at least one toxin receptor protein. The association can be via a covalent bond, an ionic interaction, a hydrogen bond, an ionic bond, a van der Waals force, a metal-ligand interaction, or any other type of interaction.

### Immunoglobulin Heavy Chain

The chimeric toxin receptor proteins contain at least a portion of an immunoglobulin heavy chain sufficient to confer either the ability to multimerize the attached anthrax receptor protein, confer antibody effector functions, stabilize the chimeric protein in the plant, confer the ability to be purified by Protein A or G, or to improve pharmacokinetics.

These properties are conferred by the constant regions of the heavy chains. If the chimeric toxin receptor protein contains only an immunoglobulin heavy chain, the portion of the heavy chain in the immunoglobulin complex preferably contains at least domains CH2 and CH3 and more preferably, only CH2 and CH3. If the chimeric toxin receptor protein contains both a heavy chain and a light chain, the portion of the heavy chain in the immunoglobulin complex preferably also contains a CH1 domain.

One of skill in the art will readily be able to identify immunoglobulin heavy chain constant region sequences. For example, a number of immunoglobulin DNA and protein sequences are available through GenBank. Table 2 shows the GenBank Accession numbers of immunoglobulin heavy chain genes and the proteins encoded by the genes.

**TABLE 2**

| GENBANK ACCESSION NO. | HUMAN IMMUNOGLOBULIN SEQUENCE NAME | SEQ NO. ID |
|---|---|---|
| J00220 | Ig_{α}1 Heavy Chain Constant Region Coding Sequence | 15, 52 |
| J00220 | Ig_{α}1 Heavy Chain Constant Region Amino | 16 |
| | Acid Sequence | |
| J00221 | IgA₂ Heavy Chain Constant Region Coding | 17, 53 |
| | Sequence | |
| J00221 | IgA₂ Chain Constant Region Amino Acid | 18 |
| | Sequence | |
| J00228 | Ig_{γ}1 Heavy Chain Constant Region Coding | 19, 54 |
| | Sequence | |
| J00228 | Igγ1 Heavy Chain Constant Region Amino Acid Sequence | 20 |
| J00230 | IgG₂ Heavy Chain Constant Region Coding | 21, 55 |
| V00554 | Sequence | |
| J00230 | IgG₂ Heavy Chain Constant Region | 22 |
| | Amino | |
| V00554 | Acid Sequence | |
| X03604 | IgG₃ Heavy Chain Constant Region Coding | 23, 57 |
| M12958 | Sequence | |
| X03604 | IgG₃ Heavy Chain Constant Region Amino | 24 |
| M12958 | Acid Sequence | |
| K01316 | IgG₄ Heavy Chain Constant Region Coding Sequence | 25 |
| K01316 | IgG₄ Heavy Chain Constant Region Amino Acid Sequence | 26 |
| K02876 | IgD Heavy Chain Constant Region Coding Sequence | 27 |
| K02876 | IgD Heavy Chain Constant Region Amino Acid Sequence | 28, 30, 32 |
| K02877 | IgD Heavy Chain Constant Region Coding Sequence | 29 |
| K02877 | IgD Heavy Chain Constant Region Amino Acid Sequence | 28, 30, 32 |
| K02878 | Germline IgD Heavy Chain Coding Sequence | 31 |
| K02878 | Germline IgD Heavy Chain Amino Acid Sequence | 28, 30, 32 |
| K02879 | Germline IgD Heavy Chain C-δ-3 Domain Coding Sequence | 33 |
| K02879 | Germline IgD Heavy Chain C-δ-3 Amino Acid Sequence | 28, 30, 32 |
| K01311 | Germline IgD Heavy Chain J-δ Region: C-δ | 58 |
| | CH1 Coding Sequence | |
| K01311 | Germline IgD Heavy Chain J-δ Region: C-δ | 28, 30, 32 |
| | CH1 Amino Acid Sequence | |
| K02880 | Germline IgD Heavy Chain Gene, C-Region, Secreted Terminus | 36 |
| | Coding Sequence | |
| K02880 | Germline IgD Heavy Chain Gene, C-Region, Secreted Terminus | 28, 30, 32 |
| | Amino Acid Sequence | |
| K02881 | Germline IgD-Heavy Chain Gene, C-Region, First Domain of Membrane Terminus Coding Sequence | 38 |
| K02881 | Germline IgD-Heavy Chain Gene, C-Region, First Domain of Membrane Terminus Amino Acid Sequence | 28, 30, 32 |
| K02882 | Germline IgD Heavy Chain Coding Sequence | 40 |
| K02882 | Germline IgD Heavy Chain Amino Acid Sequence | 28, 30, 32 |
| K02875 | Germline IgD Heavy Chain Gene, C-Region, C-δ-1 Domain Coding Sequence | 42 |
| K02875 | Germline IgD Heavy Chain Gene, C-Region, C-δ-1 Domain Amino Acid Sequence | 28, 30, 32 |
| L00022 | IgE Heavy Chain Constant Region Coding | 59 |
| J00227 | Sequence | |
| V00555 | | |
| L00022 | IgE Heavy Chain Constant Region Amino | 60 |
| J00227 | Acid Sequence | |
| V00555 | | |
| X17115 | IgM Heavy Chain Complete Sequence | 61 |
| | Coding Sequence | |
| X17115 | IgM Heavy Chain Complete Sequence | 62 |
| | Amino Acid Sequence | |

Therefore, in certain embodiments, the portion of the chimeric molecule derived from immunoglobulin heavy chain may contain less than an entire heavy chain as long as it still contains the portion sufficient to confer the properties described above.

The immunoglobulin heavy chain can be from any type of immunoglobuling, such as IgG₁, IgG₂, IgG₃ or IgG₄. The portion of the immunoglobulin heavy chain can contain CH1, CH2, or CH3 of the IgG heavy chains. Various combinations and subcombinations of these constant domains are also contemplated.

One skilled in the art will understand that immunoglobulins consist of domains which are approximately 100-110 amino acid residues. These various domains are well known in the art and have known boundaries. The removal of a single domain and its replacement with a domain of another antibody molecule is easily achieved with modem molecular biology. The domains are globular structures which are stabilized by intrachain disulfide bonds. This confers a discrete shape and makes the domains a self-contained unit that can be replaced or interchanged with other similarly shaped domains.

### Heavy Chain Chimeric Immunoglobulins

Various aspects and embodiments contemplate a chimeric toxin receptor molecule in which the immunoglobulin domains comprising the heavy chain are derived from different isotypes of heavy chain immunoglobulins. One skilled in the art will understand that using molecular techniques, these domains can be substituted for a similar domain and thus produce an immunoglobulin that is a hybrid between two different immunoglobulin molecules. These chimeric immunoglobulins allow immunoadhesins to be constructed that contain a variety of different and desirable properties that are conferred by different immunoglobulin domains.

In certain embodiments, chimeric toxin receptor molecules contain domains from two different isotypes of human immunoglobulin heavy chains.

Chimeric heavy chain immunoglobulins derived from species such as human, mouse or other mammals are contemplated. The present invention contemplates chimeric heavy chain immunoglobulin that contain immunoglobulin domains derived from at least two different isotypes of mammalian immunoglobulins. Generally, any of the mammalian immunoglobulins can be used in the preferred embodiments, such as the following isotypes: any isotype of IgG. The present invention also contemplates chimeric molecules that contain immunoglobulin domains derived from two different isotypes of rodent or primate immunoglobulin. The isotypes of rodent or primate immunoglobulin are well known in the art.

The chimeric molecules of the present invention may contain immunoglobulin derived heavy chains that include at least one of the following immunoglobulin domains: the CH1, CH2, or CH3 domains of a mouse IgG, IgG1, IgG2a, IgG2b or IgG3; the CH1 domain of a human IgG, IgG1, IgG2, IgG3 or IgG4; the CH1, CH2, or CH3 domain of an isotype of mammalian IgG; the CH1, CH2, or CH3 domain of an isotype of rodent IgG; the CH1, CH2, or CH3 domain of an isotype of animal IgG.

The present invention also contemplates the replacement or addition of protein domains derived from molecules that are members of the immunoglobulin superfamily into the chimeric molecules. The molecules that belong to the immunoglobulin superfamily have amino acid residue sequence and nucleic acid sequence homology to immunoglobulins. The molecules that are part of the immunoglobulin superfamily can be identified by amino acid or nucleic acid sequence homology. See, for example, p. 361 of Immunoglobulin Genes, Academic Press (1989).

### Light Chain Immunoglobulins

The portion of an immunoglobulin light chain described herein is that portion of the immunoglobulin light chain sufficient to increase stability of the expressed chimeric toxin receptors. Preferably, the light chains of this invention include at least a constant domain or at least a portion of a constant domain sufficient to become associated with any of the heavy chains described herein. Thus the chain can contain less than an entire domain. The light chain may either a lambda or kappa chain.

The identity of the toxin receptor covalently linked to the light chain can be adjusted to affect the affinity and avidity of the chimeric toxin receptor protein/immunoadhesin. For example, if the toxin receptor covalently linked to the light chain is the same toxin receptor as that associated with the heavy chain, the same toxin receptor but with a different amino acid sequence due to the presence of different receptor domains, or an alternative receptor for the same toxin, it can increase the avidity of the chimeric toxin receptor protein. Alternatively, the light chain can be covalently linked to a receptor for another toxin, thus creating a chimeric toxin receptor protein suitable for use as a decoy for multiple toxins.

The association between the domains of the light chains and the variable domain can be via any sort of linkage. For example, the linkage can be a flexible polypeptide linker, such as a (Gly3Ser)3 linker.

It is contemplated that the light chains as described herein can also occur in similar variations as those described for heavy chains of this invention. For example, the light chain constant domains may be derived from different organisms or species.

### Linkage between Toxin Receptor and Immunoglobulin Complex

The linkage between the immunoglobulin complex and the toxin receptor can either be via an association between the light chain and a toxin receptor, more preferably an association between the heavy chain and the toxin receptor, and most preferably both associations. These associations can be via any type of linkage that provides enough flexibility for the immunoadhesins described herein to act as receptor decoys. Preferably, the linkage is covalent, for example, a covalent peptide bond.

For example, the linkage can be a native amino acid sequence, an immunoglobulin hinge as found between the CH2 and CH3 of IgG or a flexible synthetic polypeptide linker, such as a (Gly3Ser)3 linker or a (Gly4Ser)3. One chimeric toxin receptor can contain any number of different types of linkers. Immunoadhesins

Immunoadhesins are polypeptides including at least one chimeric toxin receptor protein of the invention In some embodiments, the immunoadhesins described herein also include a J chain and/or a secretory component. In other embodiments, the immunoadhesins include more than one chimeric toxin receptor protein.

### J chain

The immunoadhesins of the present invention may, in addition to the chimeric toxin receptor proteins, contain J chain bound to the immunoglobulin derived heavy chains. In preferred embodiments, the immunoadhesin of the present invention comprises two or four chimeric anthrax toxin receptor proteins and an J chain bound to at least one of the chimeric proteins. The J chain is described and known in the art. See, for example, M. Koshland, The Immunoglobulin Helper: The J Chain, in Immunoglobulin Genes, Academic Press, London, pg. 345, (1989) and Matsuuchi et al., Proc. Natl. Acad. Sci. U.S.A., 83:456-460 (1986). The sequence of the J chain is available on various databases in the United States.

### Secretory Component

The immunoadhesin can have also a secretory component associated with the chimeric toxin receptor protein. This association may occur by hydrogen bonds, disulfide bonds, covalent bonds, ionic interactions or combinations of these various bonds.

The present invention contemplates the use of secretory component from a number of different species, including human, rat, rabbit, bovine and the like. The nucleotide sequences for these molecules are well known in the art. For example, U.S. Pat. No. 6,046,037 contains many of the sequences. The immunoadhesins of the present invention containing the secretory component, the chimeric anthrax receptor protein and J chain are typically bonded together by one of the following: hydrogen bonds, disulfide bonds, covalent bonds, ionic interactions or combinations of these bonds.

### Multimeric Inimunoadhesins

Immunoadhesins may comprise more than one receptor protein molecule, for example, they may contain chimeric toxin receptor molecules that are monomeric units and not disulfide bonded to other chimeric toxin receptor molecules. Immunoadhesins may contain chimeric toxin receptor molecules that are in association with other chimeric toxin receptor molecules to form dimers and other multivalent molecules. Typically, the chimeric toxin receptor molecule is present as a dimer because of the association of the immunoglobulin portion of the chimeric molecule. The immunoglobulin portion of the chimeric toxin receptor molecule allows the association of two chimeric Anthrax toxin receptor molecules to form a dimeric molecule having two active binding portions made up of the toxin receptor protein portion. In preferred embodiments, dimerization occurs via the disulfide bonding that normally occurs between the immunoglobulin domains as part of a naturally-occurring immunoglobulin molecule. One of skill in the art will understand that these disulfide bonds that are normally present in the native immunoglobulin molecule can be modified, moved and removed while still maintaining the ability to form a dimer of the chimeric toxin receptor molecules.

The multimeric form of the immunoadhesin may have four chimeric toxin receptor molecules, wherein two of the chimeric toxin receptor molecules, derived from the fusion of the toxin receptor and immunoglobulin heavy chain, are disulfide bonded to each other, and each such disulfide bonded chimeric toxin molecule is likewise disulfide bonded to another chimeric toxin receptor molecule derived from the fusion of the toxin receptor and an immunoglobulin light chain, thus forming a tetrameric structure.

In other preferred embodiments, the immunoadhesin contains multimeric forms of the chimeric toxin receptor molecule due to the association of J chain with the immunoglobulin portion of the chimeric toxin receptor molecule. The association of J chain with the dimer of two chimeric toxin receptor molecules allows the formation of tetrameric forms of the immunoadhesin.

### Plant-Specific Glycosylation

In certain embodiments of the present invention, the immunoadhesins and chimeric toxin receptor proteins described above are expressed by methods that generate proteins having plant-specific glycosylation. It is well-known in the art that glycosylation is a major modification of proteins in eukaryotic cells, including plant, mammalian and insect cells (Lerouge et al., Plant Molecular Biology 38:31-48, 1998). The glycosylation process starts in the endoplasmic reticulum by the co-translational transfer of a precursor oligosaccharide to specific residues of the nascent polypeptide chain. Processing of this oligosaccharide into different types of glycans, which differ in the types of residues present and the nature of the linkages between the residues, occurs in the secretory pathway as the glycoprotein moves from the endoplasmic reticulum to its final destination. One of skill in the art will understand that at the end of their maturation, proteins expressed in plants or plant cells have a different pattern of glycosylation than do proteins expressed in other types of cells, including mammalian cells and insect cells. Detailed studies characterizing plant-specific glycosylation and comparing it with glycosylation in other cell types have been performed, for example, in studies described by Cabanes-Macheteau et al., Glycobiology 9(4):365-372 (1999), and Altmann, Glycoconjugate J. 14:643-646 (1997). These groups and others have shown that plant-specific glycosylation generates glycans that have xylose linked β(1,2) to mannose, but xylose is not linked β(1,2) to mannose as a result of glycosylation in mammalian and insect cells. Plant-specific glycosylation results in a fucose linked a(1,3) to the proximal GlcNAc, while glycosylation in mammalian cells results in a fucose linked α(1,6) to the proximal GlcNAc. Furthermore, plant-specific glycosylation does not result in the addition of a sialic acid to the terminus of the protein glycan, whereas in glycosylation in mammalian cells, sialic acid is added.

The amino acid sequences of the all of the polypeptides forming the immunoadhesins and chimeric toxin receptor protein of this invention are known. One of skill in the art will know that the glycosylation sites are the tripeptide Asn-X-Ser/Thr where X can be any amino acid except proline and aspartic acid (Kornfeld and Kornfeld, Annu Rev Biochem 54:631-664, 1985). It will therefore be known to one of skill in the art that which amino acids of the polypeptides forming the proteins described herein will have plant-specific glycosylation.

In other preferred aspects and embodiments of the present invention, the immunoadhesin having plant-specific glycosylation further comprises a J chain and secretory component associated with said chimeric molecule. As was true with respect to the chimeric molecule, one of skill in the art will be able to identify the sites for plant-specific glycosylation in the J chain and secretory component sequences.

If desired, plant specific glycosylation may be modified by the addition of sequences that direct the post-translational processing of the plant-produced polypeptide forming the chimeric toxin receptor proteins and immunoadhesins to sites other than the endoplasmic reticulum leading to a modified post-translational glycosylation in which xylose and fucose linkages are substantially reduced or eliminated. The addition of KDEL to the sequence encoding the C-terminus of the immunoglobulin leads to this type of modified glycosylation.

In other preferred aspects and embodiments of the invention it may be desirable to eliminate either N-linked or O-linked glycosylation or both of these forms of glycosylation, especially if Fc receptor interaction is not required for a functional chimeric toxin receptor protein/immunoadhesin product to prevent, or provide therapy for, the pathological effects of a virus, bacteria, fungus or a toxic molecule produced thereby or otherwise present in the subject to which the product is administered.

### Functional Derivatives

Also provided herein are immunoadhesin and chimeric toxin receptor protein functional derivatives. By "functional derivative" is meant a "chemical derivative," "fragment," or "variant," of the polypeptide or nucleic acid of the invention which retains at least a portion of the function of the protein, for example reactivity with an antibody specific for the protein, enzymatic activity or binding activity, which permits its utility in accordance with the present invention. It is well known in the art that due to the degeneracy of the genetic code numerous different nucleic acid sequences can code for the same amino acid sequence. It is also well known in the art that conservative changes in amino acid can be made to arrive at a protein or polypeptide that retains the functionality of the original. In both cases, all permutations are intended to be covered by this disclosure.

The derivatives may also be engineered according to routine methods to include an affinity purification tag such that large quantities and/or relatively pure or isolated quantities of immunoadhesin may be produced. Many different versions of tag exist that can be incorporated into one or more components of the immunoadhesin, preferably not destroying the desired binding activity of the immunoadhesin in the absence of tag. Such tags can be engineered as expressible encoded nucleic acid sequence fused with nucleic acid sequences encoding the immunoadhesins of the invention. The tags may further be engineered to be removable, e.g., with a commercially available enzyme.

Further, it is possible to delete codons or to substitute one or more codons with codons other than degenerate codons to produce a structurally modified polypeptide, but one which has substantially the same utility or activity as the polypeptide produced by the unmodified nucleic acid molecule. As recognized in the art, the two polypeptides can be functionally equivalent, as are the two nucleic acid molecules that give rise to their production, even though the differences between the nucleic acid molecules are not related to the degeneracy of the genetic code.

Manipulations of this sort, and post-production chemical derivatization may be implemented, e.g., to improve stability, solubility, absorption, biological or therapeutic effect, and/or biological half-life. Moieties capable of mediating such effects are disclosed, for example, in Remington's Pharmaceutical Sciences, 18th ed., Mack Publishing Co., Easton, Pa. (1990). A functional derivative intended to be within the scope of the present invention is a "variant" polypeptide which either lacks one or more amino acids or contains additional or substituted amino acids relative to the native polypeptide. The variant may be derived from a naturally occurring complex component by appropriately modifying the protein DNA coding sequence to add, remove, and/or to modify codons for one or more amino acids at one or more sites of the C-terminus, N-terminus, and/or within the native sequence. It is understood that such variants having added, substituted and/or additional amino acids retain one or more characterizing portions of the native protein, as described above.

A functional derivative of a protein with deleted, inserted and/or substituted amino acid residues may be prepared using standard techniques well-known to those of ordinary skill in the art. For example, the modified components of the functional derivatives may be produced using site-directed mutagenesis techniques (as exemplified by Adelman et. al., 1983, DNA 2:183) wherein nucleotides in the DNA coding sequence are modified such that a modified coding sequence is produced, and thereafter expressing this recombinant DNA in a prokaryotic or eukaryotic host cell, using techniques such as those described above. Alternatively, proteins with amino acid deletions, insertions and/or substitutions may be conveniently prepared by direct chemical synthesis, using methods well-known in the art. The functional derivatives of the proteins typically exhibit the same qualitative biological activity as the native proteins.

In addition, the immunoadhesins of the invention may be not just chimeric receptor protein/Ig immunoadhesins, but may also embrace other native receptor protein family members, isotypes, and/or other homologous amino acid sequences, e.g., human, primate, rodent, canine, feline, bovine, avian, etc. Furthermore, the Ig type used in the immunoadhesins can vary, e.g., may assume a different Ig family member identity, within or without a given species. Igs are diverse and have well-known sequences that one of ordinary skill can exploit to create different immunoadhesins having more or less different utility in a given organism to undergo treatment.

### Anthrax Chimeric Toxin Receptor Proteins and Immunoadhesins

The invention also includes chimeric anthrax toxin receptor proteins as set forth in claim 1. Optionally, an immunoglobulin light chain is included in the complex to improve stability. Alternatively, an anthrax toxin receptor associated with an immunoglobulin light chain or fragment of such immunoglobulin light chain, in either case preferably at the N-terminus thereof, is included in the complex to improve stability and/or provide additional anthrax toxin receptors to improve binding to anthrax toxin.

An anthrax toxin receptor protein may be associated with the heavy chain, the light chain, or both. In some embodiments, a J chain and secretory component is associated with the chimeric anthrax toxin receptor protein to form an immunoadhesin. In other embodiments, the chimeric toxin receptor protein has plant-specific glycosylation.

### Chimeric Anthrax Toxin Receptor Protein

The chimeric anthrax toxin receptor proteins described herein contain at least one anthrax toxin receptor protein associated with an immunoglobulin complex containing an immunoglobulin heavy chain.

### Anthrax Toxin Receptor

The Anthrax toxin receptor is any polypeptide which binds protective antigen (PA) with an affinity sufficient to allow a chimeric anthrax toxin receptor protein to act as a receptor decoy.

The Anthrax toxin receptor as described herein is Capillary morphogenesis factor 2 (CMG2) (Scobie et al., 2001). The receptor functions as type 1 transmembrane protein and has as a signal peptide, an extracellular domain and a single-pass transmembrane region. Several protein isoforms have been predicted through alternative splicing including soluble variants.

The Anthrax toxin receptor described herein shall at a minimum contain the functional elements for binding of PA and may optionally also include one of more additional polypeptides (for example, ones that improve use of the anthrax toxin receptor as a receptor decoy). The domains which mediate binding of the ATR to PA have been well characterized (32-27). The Van Willebrand Factor type A domain (VWA domain), also known as the integrin inserted domain (I domain), contains a metal ion-dependent adhesion site (MIDAS) that appears to be essential for proper PA binding to this receptor. See Scobie HM, Rainey GJ, Bradley KA, Young JA (2003) Human capillary morphogenesis protein 2 functions as an anthrax toxin receptor. Proc Natl Acad Sci USA 100: 5170-5174. Bradley KA, Mogridge J, Mourez M, Collier RJ, Young JA (2001) Identification of the cellular receptor for anthrax toxin. Nature 414: 225-229. In preferred embodiments, the Anthrax toxin receptor protein includes the entire extracellular domain of the receptor.

The Anthrax toxin receptor in the present invention lacks the 14 amino acids which are not part of the VWA/I domain. This 14 amino acid sequence and its corresponding nucleotide sequence are provided below:
TEILELQPSSVCVG (SEQ ID NO: 102)
act gaa atc cta gaa ttg cag ccc tca agt gtc tgt gtg ggg (SEQ ID NO: 103)

### Vectors, Cells and Plants Containing Chimeric Toxin Receptor Proteins and Immunoadhesins

The present invention also contemplates expression and cloning vectors, cells and plants containing the chimeric toxin receptors and immunoadhesins of the present invention. Technology for isolating the genes encoding the various portions of the proteins are well-known to one of skill in the art and can be applied to insert the various required genes into expression vectors and cloning vectors such as those vectors can be introduced into eukaryotic cells including plant cells, plants and transgenic plants.

The present invention contemplates a method of assembling an immunoadhesin comprising the steps of: introducing into an organism a DNA segment encoding a chimeric receptor protein molecule, J chain, and introducing into the same organism a DNA encoding a secretory component. The preferred secretory component contains at least a segment of the amino acid residues 1 to residue about 606 of the human polyimmunoglobulin receptor (pIgR) amino acid residue sequence or analogous amino acid residues from other species (Mostov, Ann Dev. Immu. 12:63-84 1994). Among the secretory component sequences that may be used in addition to the foregoing human pIgR are those containing at least a segment of the amino acid residue 1 to about 627 of the human pIgR.

The present invention contemplates eukaryotic cells, including plant cells, containing chimeric toxin receptors and immunoadhesins of the present invention. The present invention also contemplates plant cells that contain nucleotide sequences encoding the various components of the chimeric toxin receptors and immunoadhesins of the present invention. One skilled in the art will understand that the nucleotide sequences that encode the secretory component protection protein and the chimeric receptor protein molecule and J chain will typically be operably linked to a promoter and present as part of an expression vector or cassette. Typically, if the eukaryotic cell used is a plant cell then the promoter used will be a promoter that is able to operate in a plant cell. After the chimeric receptor protein genes, secretory component genes and J chain genes are isolated, they are typically operatively linked to a transcriptional promoter in an expression vector. The present invention also contemplates expression vectors containing a nucleotide sequence encoding a chimeric receptor protein molecule which has been operatively linked to a regulatory sequence for expression. These expression vectors place the nucleotide sequence to be expressed in a particular cell 3' of a promoter sequence which causes the nucleotide sequence to be transcribed and expressed. The expression vector may also contain various enhancer sequences which improve the efficiency of this transcription. In addition, such sequences as terminators, polyadenylation (poly A) sites and other 3' end processing signals may be included to enhance the amount of nucleotide sequence transcribed within a particular cell.

Expression of the components in the organism of choice can be derived from an independently replicating plasmid, or from a permanent component of the chromosome, or from any piece of DNA which may transiently give rise to transcripts encoding the components. Organisms suitable for transformation can be either prokaryotic or eukaryotic. Introduction of the components of the complex can be by direct DNA transformation, by biolistic delivery into the organism, or mediated by another organism as for example by the action of recombinant *Agrobacterium* on plant cells. Expression of proteins in transgenic organisms usually requires co-introduction of an appropriate promoter element and polyadenylation signal. In one embodiment of the invention, the promoter element potentially results in the constitutive expression of the components in all of the cells of a plant. Constitutive expression occurring in most or all of the cells will ensure that precursors can occupy the same cellular endomembrane system as might be required for assembly to occur.

### Expression Vectors

Expression vectors compatible with the host cells, preferably those compatible with plant cells are used to express the genes of the present invention. Typical expression vectors useful for expression of genes in plants are well known in the art and include vectors derived from the tumor-inducing (Ti) plasmid of *Agrobacterium tumefaciens* described by Rogers et al., Meth. in Enzymol., 153:253-277 (1987). However, several other expression vector systems are known to function in plants. See for example, Verma et al., PCT Publication No. WO87/00551; and Cocking and Davey, Science, 236:1259-1262 (1987).

The expression vectors described above contain expression control elements including the promoter. The genes to be expressed are operatively linked to the expression vector to allow the promoter sequence to direct RNA polymerase binding and synthesis of the desired polypeptide coding gene. Useful in expressing the genes are promoters which are inducible, viral, synthetic, constitutive, and regulated. The choice of which expression vector is used and ultimately to which promoter a nucleotide sequence encoding part of the immunoadhesin of the present invention is operatively linked depends directly, as is well known in the art, on the functional properties desired, e.g. the location and timing of protein expression, and the host cell to be transformed, these being limitations inherent in the art of constructing recombinant DNA molecules. However, an expression vector useful in practicing the present invention is at least capable of directing the replication, and preferably also the expression of the polypeptide coding gene included in the DNA segment to which it is operatively linked.

In preferred embodiments, the expression vector used to express the genes includes a selection marker that is effective in a plant cell, preferably a drug resistance selection marker. A preferred drug resistance marker is the gene whose expression results in kanamycin resistance, i.e., the chimeric gene containing the nopaline synthase promoter, Tn5 neomycin phosphotransferase II and nopaline synthase 3' nontranslated region described by Rogers et al., in Methods For Plant Molecular Biology, a Weissbach and H. Weissbach, eds., Academic Press Inc., San Diego; Calif. (1988). A useful plant expression vector is commercially available from Pharmacia, Piscataway, N.J. Expression vectors and promoters for expressing foreign proteins in plants have been described in U.S. Pat. Nos. 5,188,642; 5,349,124; 5,352,605, and 5,034,322

### Construction of Expression Vectors

A variety of methods have been developed to operatively link DNA to vectors via complementary cohesive termini. For instance, complementary homopolymer tracks can be added to the DNA segment to be inserted into the vector DNA. The vector and DNA segment are then joined by hydrogen bonding between the complementary homopolymeric tails to form recombinant DNA molecules. Alternatively, synthetic linkers containing one or more restriction endonuclease sites can be used to join the DNA segment to the expression vector. The synthetic linkers are attached to blunt-ended DNA segments by incubating the blunt-ended DNA segments with a large excess of synthetic linker molecules in the presence of an enzyme that is able to catalyze the ligation of blunt-ended DNA molecules, such as bacteriophage T4 DNA ligase. Thus, the products of the reaction are DNA segments carrying synthetic linker sequences at their ends. These DNA segments are then cleaved with the appropriate restriction endonuclease and ligated into an expression vector that has been cleaved with an enzyme that produces termini compatible with those of the synthetic linker. Synthetic linkers containing a variety of restriction endonuclease sites are commercially available from a number of sources including New England BioLabs, Beverly, Mass.

The nucleotide sequences encoding the secretory component, J chain, and the chimeric receptor protein molecule of the present invention are introduced into the same plant cell either directly or by introducing each of the components into a plant cell and regenerating a plant and cross-hybridizing the various components to produce the final plant cell containing all the required components.

### Expression of Chimeric Toxin Receptors and Immunoadhesins

Any method may be used to introduce the nucleotide sequences encoding the components of the chimeric toxin receptors and immunoadhesins of the present invention into a eukaryotic cell. For example, methods for introducing genes into plants include *Agrobacterium-mediated* plant transformation, protoplast transformation, gene transfer into pollen, injection into reproductive organs and injection into immature embryos. Each of these methods has distinct advantages and disadvantages. Thus, one particular method of introducing genes into a particular eukaryotic cell or plant species may not necessarily be the most effective for another eukaryotic cell or plant species.

### Plants

For example, methods for introducing genes into plants include *Agrobacterium-mediated* plant transformation, protoplast transformation, gene transfer into pollen, injection into reproductive organs and injection into immature embryos. Each of these methods has distinct advantages and disadvantages. Thus, one particular method of introducing genes into a particular eukaryotic cell or plant species may not necessarily be the most effective for another eukaryotic cell or plant species.

Genes can be introduced into plant cells in a transient manner designed to rapidly produce a protein of interest without permanently or stably altering the genome of a plant or its offspring. One example of how this transient expression can be accomplished is through the infiltration of the intercellular air spaces in leaves of an intact plant with a suspension of Agrobacterium tumefaciens containing the gene or genes of interest in a binary vector, and harvesting said leaves for extraction of protein at some later time. Voinnet O, Rivas S, Mestre P, Baulcombe D (2003) An enhanced transient expression system in plants based on suppression of gene silencing by the p19 protein of tomato bushy stunt virus. The Plant Journal 33: 949-956.

*Agrobacteriuin tumefaciens-*mediated transfer is a widely applicable system for introducing genes into plant cells because the DNA can be introduced into whole plant tissues, bypassing the need for regeneration of an intact plant from a protoplast. The use of *Agrobacterium-*mediated expression vectors to introduce DNA into plant cells is well known in the art. See, for example, the methods described by Fraley et al., Biotechnology, 3:629 (1985) and Rogers et al., Methods in Enzymology, 153:253-277 (1987). Further, the integration of the Ti-DNA is a relatively precise process resulting in few rearrangements. The region of DNA to be transferred is defined by the border sequences and intervening DNA is usually inserted into the plant genome as described by Spielmann et al., Mol. Gen. Genet., 205:34 (1986) and Jorgensen et al., Mol. Gen. Genet., 207:471 (1987). Modem *Agrobacterium* transformation vectors are capable of replication in Escherichia coli as well as *Agrobacterium,* allowing for convenient manipulations as described by Klee et al., in Plant DNA Infectious Agents, T. Hohn and J. Schell, eds., Springer-Verlag, New York, pp. 179-203 (1985). Further recent technological advances in vectors for *Agrobacterium-*mediated gene transfer have improved the arrangement of genes and restriction sites in the vectors to facilitate construction of vectors capable of expressing various polypeptide coding genes. The vectors described by Rogers et al., Methods in Enzymology, 153:253 (1987), have convenient multi-linker regions flanked by a promoter and a polyadenylation site for direct expression of inserted polypeptide coding genes and are suitable for present purposes.

In those plant species where *Agrobacterium-*mediated transformation is efficient, it is the method of choice because of the facile and defined nature of the gene transfer. *Agrobacterium-*mediated transformation of leaf disks and other tissues appears to be limited to plant species that *Agrobacterium tumefaciens* naturally infects. Thus, *Agrobacterium-*mediated transformation is most efficient in dicotyledonous plants.

Few monocots appear to be natural hosts for *Agrobacterium,* although transgenic plants have been produced in asparagus using *Agrobacterium* vectors as described by Bytebier et al., Proc. Natl. Acad. Sci. U.S.A., 84:5345 (1987). Therefore, commercially important cereal grains such as rice, corn, and wheat must be transformed using alternative methods. Transformation of plant protoplasts can be achieved using methods based on calcium phosphate precipitation, polyethylene glycol treatment, electroporation, and combinations of these treatments. See, for example, Potrykus et al., Mol. Gen. Genet., 199:183 (1985); Lorz et al., Mol. Gen. Genet., 199:178 (1985); Fromm et al., Nature, 319:791 (1986); Uchimiya et al., Mol. Gen. Genet., 204:204 (1986); Callis et al., Genes and Development, 1:1183 (1987); and Marcotte et al., Nature, 335:454 (1988).

Application of these methods to different plant species depends upon the ability to regenerate that particular plant species from protoplasts. Illustrative methods for the regeneration of cereals from protoplasts are described in Fujimura et al., Plant Tissue Culture Letters, 2:74 (1985); Toriyama et al., Theor Appl. Genet., 73:16 (1986); Yamada et al., Plant Cell Rep., 4:85 (1986); Abdullah et al., Biotechnology, 4:1087 (1986).

To transform plant species that cannot be successfully regenerated from protoplasts, other ways to introduce DNA into intact cells or tissues can be utilized. For example, regeneration of cereals from immature embryos or explants can be effected as described by Vasil, Biotechnology, 6:397 (1988). In addition, "particle gun" or high-velocity microprojectile technology can be utilized. Using such technology, DNA is carried through the cell wall and into the cytoplasm on the surface of small (0.525 *µ*m) metal particles that have been accelerated to speeds of one to several hundred meters per second as described in Klein et al., Nature, 327:70 (1987); Klein et al., Proc. Natl. Acad. Sci. U.S.A., 85:8502 (1988); and McCabe et al., Biotechnology, 6:923 (1988). The metal particles penetrate through several layers of cells and thus allow the transformation of cells within tissue explants. Metal particles have been used to successfully transform corn cells and to produce fertile, stably transformed tobacco and soybean plants. Transformation of tissue explants eliminates the need for passage through a protoplast stage and thus speeds the production of transgenic plants.

DNA can also be introduced into plants by direct DNA transfer into pollen as described by Zhou et al., Methods in Enzymology, 101:433 (1983); D. Hess, Intern Rev. Cytol., 107:367 (1987); Luo et al., Plant Mol. Biol. Reporter, 6:165 (1988). Expression of polypeptide coding genes can be obtained by injection of the DNA into reproductive organs of a plant as described by Pena et al., Nature, 325:274 (1987). DNA can also be injected directly into the cells of immature embryos and the rehydration of desiccated embryos as described by Neuhaus et al., Theor. Appl. Genet., 75:30 (1987); and Benbrook et al., in Proceedings Bio Expo 1986, Butterworth, Stoneham, Mass., pp. 27-54 (1986).

The regeneration of plants from either single plant protoplasts or various explants is well known in the art. See, for example, Methods for Plant Molecular Biology, A. Weissbach and H. Weissbach, eds., Academic Press, Inc., San Diego, Calif. (1988). This regeneration and growth process includes the steps of selection of transformant cells and shoots, rooting the transformant shoots and growth of the plantlets in soil.

The regeneration of plants containing the foreign gene introduced by *Agrobacterium tumefaciens* from leaf explants can be achieved as described by Horsch et al., Science, 227:1229-1231 (1985). In this procedure, transformants are grown in the presence of a selection agent and in a medium that induces the regeneration of shoots in the plant species being transformed as described by Fraley et al., Proc. Natl. Acad. Sci. U.S.A., 80:4803 (1983). This procedure typically produces shoots within two to four weeks and these transformant shoots are then transferred to an appropriate root-inducing medium containing the selective agent and an antibiotic to prevent bacterial growth. Transformant shoots that rooted in the presence of the selective agent to form plantlets are then transplanted to soil to allow the production of roots. These procedures will vary depending upon the particular plant species employed, such variations being well known in the art.

The immunoadhesins of the present invention may be produced in any plant cell including plant cells derived from plants that are dicotyledonous or monocotyledonous, solanaceous, alfalfa, legumes, oil seeds such as safflower, or tobacco.

Transgenic plants of the present invention can be produced from any sexually crossable plant species that can be transformed using any method known to those skilled in the art. Useful plant species are dicotyledons including tobacco, tomato, the legumes, alfalfa, oaks, and maples; monocotyledons including grasses, corn, grains, oats, wheat, and barley; and lower plants including gymnosperms, conifers, horsetails, club mosses, liverworts, hornworts, mosses, algaes, gametophytes, sporophytes or pteridophytes.

### Eukaryotic Cells

The present invention also contemplates expressing the chimeric toxin receptors and immunoadhesins within eukaryotic cells including mammalian cells. One of skill in the art will understand the various systems available for expression in mammalian cells and can readily modify those systems to express the immunoadhesins and chimeric protein receptor molecules.

### Compositions Containing Chimeric Toxin Receptors and Immunoadhesins

The present invention also contemplates compositions containing a chimeric toxin receptor or immunoadhesin of the present invention together with plant macromolecules or material. Typically these plant macromolecules or plant materials are derived from any plant useful in the present invention. The plant macromolecules are present together with a chimeric toxin receptor or immunoadhesin of the present invention for example, in a plant cell, in an extract of a plant cell, or in a plant. Typical plant macromolecules in a composition are ribulose bisphosphate carboxylase, light harvesting complex pigments (LHCP), secondary metabolites or chlorophyll. The compositions of the present invention have plant material or plant macromolecules in a concentration of between 0.01% and 99% mass excluding water. Other compositions include compositions having the chimeric toxin receptors or immunoadhesins of the present invention present at a concentration of between 1% and 99% mass excluding water. Other compositions include chimeric toxin receptors or immunoadhesins at a concentration of 50% to 90% mass excluding water.

The compositions of the present invention may contain plant macromolecules at a concentration of between 0.1 % and 5% mass excluding water. Typically the mass present in the composition will consist of plant macromolecules and chimeric toxin receptors or immunoadhesins of the present invention. When the proteins of the present invention are present at a higher or lower concentration the concentration of plant macromolecules present in the composition will vary inversely. In other embodiments the composition of plant macromolecules are present in a concentration of between 0.12% and 1% mass excluding water.

The present invention contemplates a composition of matter comprising all or part of the following: a chimeric protein receptor molecule, a J chain or a secretory component. These components form a complex and are associated as was previously described. Typically, the composition also contains molecules derived from a plant. This composition may also be obtained after an extraction process yielding functional chimeric toxin receptor or immunoadhesin and plant-derived molecules.

The extraction method comprises the steps of applying a force to a plant containing the complex whereby the apoplastic compartment of the plant is ruptured releasing said complex. The force involves shearing as the primary method of releasing the apoplastic liquid. The whole plant or plant extract contains an admixture of chimeric toxin receptor/immunoadhesin and various other macromolecules of the plant. Among the macromolecules contained in the admixture is ribulose bisphosphate carboxylase (RuBisCo) or fragments of RuBisCo. Another macromolecule is LHCP. Another molecule is chlorophyll. Other useful methods for preparing compositions include extraction with various solvents and application of vacuum to the plant material. The compositions of the present invention may contain plant macromolecules in a concentration of between about 0.1% and 5% mass excluding water.

The present invention also contemplates that the relative proportion of plant-derived molecules and animal-derived molecules can vary. Quantities of specific plant proteins, such as RuBisCo or chlorophyll may be as little as 0.01 % of the mass or as much as 99.9% of the mass of the extract, excluding water.

The present invention also contemplates therapeutic compositions which may be used in the treatment of a patient or animal. Administration of the therapeutic composition can be before or after extraction from the plant or other transgenic organism. Once extracted the chimeric toxin receptors/immunoadhesins may also be further purified by conventional techniques such as size exclusion, ion exchange, or affinity chromatography. Plant molecules may be co-administered with the complex.

The present invention also contemplates the direct use of the therapeutic plant extract containing chimeric toxin receptors/immunoadhesins without any further purification of the specific therapeutic component. Administration may be by topical application, oral ingestion, nasal spray or any other method appropriate for delivering the chimeric toxin receptors/immunoadhesins to the mucosal target pathogen.

### Pharmaceutical Compositions, Formulations, and Routes of Administration

The chimeric toxin receptors/immunoadhesins described herein can be administered to a patient, preferably in the form of a suitable pharmaceutical composition. Such composition may include components in addition to, or in lieu of, those described above. The composition preferably exhibits either or both of a therapeutic and prophylactic property when administered. The preparation of such compositions can be done according to routine methodologies in the art, and may assume any of a variety of forms, e.g., liquid solutions, suspensions or emulsifications, and solid forms suitable for inclusion in a liquid prior to ingestion. Techniques for the formulation and administration of polypeptides and proteins may be found in Remington's Pharmaceutical Sciences, Mack Publishing Co., Easton, Pa., latest edition. Using these procedures, one of ordinary skill can utilize the immunoadhesins of the invention to achieve success without undue experimentation.

### Administration Routes

Suitable routes of administration for the invention include, e.g., oral, nasal, inhalation, intraocular, pharyngeal, bronchial, transmucosal, intravenous, intramuscular, intraperitoneal or intestinal administration. Alternatively, one may administer the compound in a local manner, e.g., via injection or other application of the compound to a preferred site of action.

### Formulations

The pharmaceutical compositions of the present invention may be manufactured in a manner that is itself known, e.g., by means of conventional mixing, dissolving, granulating, dragee-making, levigating, emulsifying, encapsulating, entrapping or lyophilizing processes. One or more physiologically acceptable carriers comprising excipients and/or other auxiliaries can be used to facilitate processing of the active compounds into pharmaceutical preparations. Proper formulation is dependent upon the particular route of administration chosen.

For injection, the agents of the invention may be formulated in aqueous solutions, preferably in physiologically compatible buffers such as Hanks's solution, Ringer's solution, or physiological saline buffer.

For transmucosal administration, penetrants appropriate to the barrier to be permeated are used in the formulation. Such penetrants are generally known in the art.

For oral administration, the compounds can be formulated readily by combining the active compounds with pharmaceutically acceptable carriers well known in the art. Such carriers enable the compounds of the invention to be formulated as tablets, pills, dragees, capsules, liquids, gels, syrups, slurries, suspensions and the like, for oral ingestion by a patient to be treated. Suitable carriers include excipients such as, e.g., fillers such as sugars, including lactose, sucrose, mannitol, and/or sorbitol; cellulose preparations such as, e.g., maize starch, wheat starch, rice starch, potato starch, gelatin, gum tragacanth, methyl cellulose, hydroxypropylmethyl- cellulose, sodium carboxymethylcellulose, and/or polyvinylpyrrolidone (PVP). If desired, disintegrating agents may be added, such as the cross-linked polyvinyl pyrrolidone, agar, or alginic acid or a salt thereof such as sodium alginate.

Dragee cores are provided with suitable coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses.

Pharmaceutical preparations which can be used orally include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added. All formulations for oral administration should be in dosages suitable for such administration.

For buccal administration, the compositions may take the form of tablets or lozenges formulated in conventional manner or in the form of a water soluble gel.

For administration by inhalation, the compounds for use according to the present invention may be conveniently delivered in the form of an aerosol spray presentation from pressurized packs or a nebuliser, with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of e.g. gelatin for use in an inhaler or insufflator may be formulated containing a powder mix of the compound and a suitable powder base such as lactose or starch.

Alternatively, the active ingredient may be in powder form for constitution with a suitable vehicle, e.g., sterile pyrogen-free water, before use.

In addition, the compounds may also be formulated as a depot preparation. Such long acting formulations may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

Additionally, the compounds may be delivered using a sustained-release system, such as semipermeable matrices of solid hydrophobic polymers containing the therapeutic agent. Various sustained-release materials have been established and are well known by those skilled in the art. Sustained-release capsules may, depending on their chemical nature, release the compounds for a few weeks up to over 100 days. Depending on the chemical nature and the biological stability of the therapeutic reagent, additional strategies for protein stabilization may be employed.

The pharmaceutical compositions also may comprise suitable solid or gel phase carriers or excipients. Examples of such carriers or excipients include but are not limited to calcium carbonate, calcium phosphate, various sugars, starches, cellulose derivatives, gelatin, and polymers such as polyethylene glycols.

Pharmaceutically compatible salts may be formed with many acids, including but not limited to hydrochloric, sulfuric, acetic, lactic, tartaric, malic, succinic, citric, etc. Salts tend to be more soluble in aqueous or other protonic solvents that are the corresponding free base forms. In solutions, manipulation of pH is also routinely employed for optimizing desired properties.

### Determining Effective Dosages and Dosage Regimens

Pharmaceutical compositions suitable for use in the present invention include compositions where the active ingredients are contained in an amount effective to achieve an intended purpose, e.g., a therapeutic and/or prophylactic use. A pharmaceutically effective amount means an amount of compound effective to prevent, alleviate or ameliorate symptoms of disease or prolong the survival of the subject being treated. Determination of a pharmaceutically effective amount is well within the capability of those skilled in the art, and will typically assume an amount of between about 0.5 pg/kg/day and about 500 g/kg/day, with individual dosages typically comprising between about 1 nanogram and several grams of immunoadhesin.

For any compound used in the methods of the invention, the therapeutically effective dose can be estimated initially from cell culture assays. For example, varying dosages can be administered to different animals or cell cultures and compared for effect. In this way, one can identify a desired concentration range, and prepare and administer such amount accordingly. Optimization is routine for one of ordinary skill in the art.

The person of skill, in addition to considering pharmaceutical efficacy, also considers toxicity according to standard pharmaceutical procedures in cell cultures or experimental animals, e.g., for determining the LD50 (the dose lethal to 50% of the population) and the ED₅₀ (the dose therapeutically effective in 50% of the population). The dose ratio between toxic and therapeutic effects is the therapeutic index and it can be expressed as the ratio between LD₅₀ and ED₅₀. Compounds which exhibit high therapeutic indices are preferred. The data obtained from these cell culture assays and animal studies can be used in formulating a range of dosage for use in humans. The dosage of such compounds lies preferably within a range of concentrations that include the ED₅₀ with little or no toxicity. The dosage may vary within this range depending upon the dosage form employed and the route of administration utilized. The exact formulation, route of administration and dosage can be chosen by the individual physician in view of the patient's condition. (See e.g., Fingl et al., 1975, in "The Pharmacological Basis of Therapeutics,".Ch. 1 p.1).

Dosage amount and frequency may be adjusted to provide circulatory levels of a chimeric toxin receptor or immunoadhesin sufficient to maintain or provide a pharmaceutical effect, e.g., therapeutic and/or prophylactic. The minimal effective concentration (MEC) will vary for each formulation, but can be estimated from in vitro and/or in vivo data. Dosages necessary to achieve MEC will depend on individual characteristics and route of administration. However, assays as described herein can be used to determine circulatory concentrations, which can then be further optimized in amount and precise formulation.

Dosage intervals can also be determined using MEC value. Compounds can be administered using a regimen which maintains circulatory levels above the MEC for 10-90% of the time, 30-90% of the time, or, most preferably, 50-90% of the time.

The compositions may, if desired, be presented in a pack or dispenser device which may contain one or more unit dosage forms containing the active ingredient. The pack may for example comprise metal or plastic foil, such as a blister pack. The pack or dispenser device may be accompanied by instructions for administration. The pack or dispenser may also be accompanied with a notice associated with the container in form prescribed by a governmental agency regulating the manufacture, use, or sale of pharmaceuticals, which notice is reflective of approval by the agency of the form of the immunoadhesin for human or veterinary administration. Such notice, for example, may be the labeling approved by the U.S. Food and Drug Administration for prescription drugs, or the approved product insert. Compositions comprising a compound of the invention formulated in a compatible pharmaceutical carrier may also be prepared, placed in an appropriate container, and labeled for treatment of an indicated condition, e.g. treatment or prophylaxis of a disease mediated by host organism/patient protein receptor molecules.

### Methods of Treatment and Prevention of Infection

A patient in need of therapeutic and/or prophylactic chimeric toxin receptors/immunoadhesins of the invention, e.g., to counter anthrax infection, can be administered a pharmaceutically effective amount of desired chimeric toxin receptors/immunoadhesin, preferably as part of a pharmaceutical composition determined, produced, and administered as described above. These formulations and delivery modalities can vary widely. Described following are preliminary procedures that can be used to deduce effective amounts and toxicity, and which can then be conveniently used to determine treatment and prophylaxis parameters and regimens, both in humans and other animals. These procedures are illustrative only and are not intended to be limiting of the invention. Further, these procedures are routine for one of ordinary skill in the art.

### Ability of the Chimeric Toxin Receptor/Immunoadhesin to Reduce Infectivity in Humans: Dose Escalation Tolerance Study

Chimeric toxin receptors/immunoadhesins of the invention may be tested, e.g., using randomized controlled trials to determine the effect of administration, e.g., intravenous or intramuscular on infection. Other administration routes can be used. Various assays exist that can be used to monitor effect. These studies can evaluate the extent to which chimeric toxin receptors/immunoadhesin taken by a patient subjects can treat or prevent infection. For example, infected and exposed but uninfected subjects can be administered the chimeric toxin receptors/immunoadhesin and evaluated over a time course to assess illness progression as compared to similar infected or exposed but untreated subjects.

The chimeric toxin receptors/immunoadhesin of the present invention may be formulated as a buffered saline with varying amounts of chimeric toxin receptors/immunoadhesin within and administered at various intervals to a patient. Single ascending dose and multiple ascending dose studies can be used to evaluate the safety of the immunoadhesin. In each case, one or more subjects may be evaluated at each dosage level, some receiving the immunoadhesin and one or more optionally receiving placebo. In either study, multiple dosage levels may be evaluated. Dosage levels can vary, but are typically in the nanogram to gram range.

Dosages may be administered over seconds, minutes, hours, weeks, and months, and evaluated for toxicity and/or pharmaceutical effect.

Safety and toxicity may be assessed, e.g., by visual examination of the nasal mucosa for signs of irritation or inflammation. Blood safety evaluations can also be employed according to routine methods and using sensitive assays such as ELISA to determine various blood components, including circulating immunoadhesin and rhinovirus quantities. Nasal lavage testing may similarly be done according to routine methodologies.

Routine statistical analyses and calculations may be employed to determine efficacy and toxicity predicted over time courses for single patients and/or for populations of patient-recipients.

If the pathogen targeted by the chimeric toxin receptor protein, e.g., anthrax bacteria and anthrax spores, cannot be ethically administered to human subjects in a controlled clinical trial, projected dose efficacy in human beings can be experimentally determined in appropriate animal species which may include primate species exposed to anthrax bacterial or spores. Effective dose can be determined by survival of treated and control animals at various concentrations and routes of chimeric toxin receptor protein administration. Serum, mucus, and/or tissue samples can be taken from the subject to determine the pharmacokinetics and pharmacodynamics of the administered chimeric toxin receptor protein and from this data the minimum effective concentration and route of administration of the chimeric toxin receptor protein protective of the animal subject can be determined. To establish the minimum effective concentration for human subjects, normal male and female subjects can be dosed with the chimeric toxin receptor protein by the same route of administration determined to be effective in the animal subject trial described above and serum, mucus and or tissue samples would be taken from these human subjects. The concentration of administered chimeric toxin receptor protein in the human subject can be adjusted to obtain the same concentration previously shown to be effective or protective in the animal tests and samples.

Guidelines for animal and human trials the establishment of safety and efficacy of products for the treatment and/or prevention of life-threatening exposure to pathogens such as anthrax, Botulinum toxin, tularemia and the like, have been promulgated by the United States Food and Drug Administration and are available to the public in various publications including Federal Register: May 31, 2002 (Volume 67, Number 105), Rules and Regulations, Page 37988-37998. This rule is available from the Federal Register Online via GPO Access [wais.access.gpo.gov and may be found in the Code of Federal regulations, Food and Drug Administration, 21 CFR Parts 314 and 601.

The following examples illustrate various aspects and embodiments of the disclosed invention. These examples in no way limit the scope of the claimed invention.

### EXAMPLES

### Example 1: Construction of ICAM-1 Immunoadhesin Expression Cassettes (not of the invention)

A cassette encoding ICAM-1 extracellular domains D1 through D5 was prepared by PCR cloning. Specifically, a fragment containing all five extracellular Ig-like domains of ICAM-1 was amplified from plasmid pCDIC1-SD/IgA (Martin, et al. J. Virol. 67:3561-8, 1993) using the following oligonucleotide primers:
5'-TCTGTTCCCAGGAACTAGTTTGGCACAGACATC (SEQ ID NO: 6)
TGTGTCCCCCTCAAAAGTC-3'
5'-CATACCGGGGACTAGTCACATTCACGGTCACCT (SEQ ID NO: 7)
CGCGG-3'

These two primers were designed to introduce Spel sites at the 5' and 3' ends of the PCR fragment (underlined nucleotides). PCR was performed with Pfu polymerase (Stratagene) to reduce accumulation of errors. The PCR fragment was cloned into the vector PCRScript (Stratagene), and sequenced before fusing to the human IgA2 cassettes (with and without SEKDEL [SEQ ID NO:4] at the carboxy-terminus).

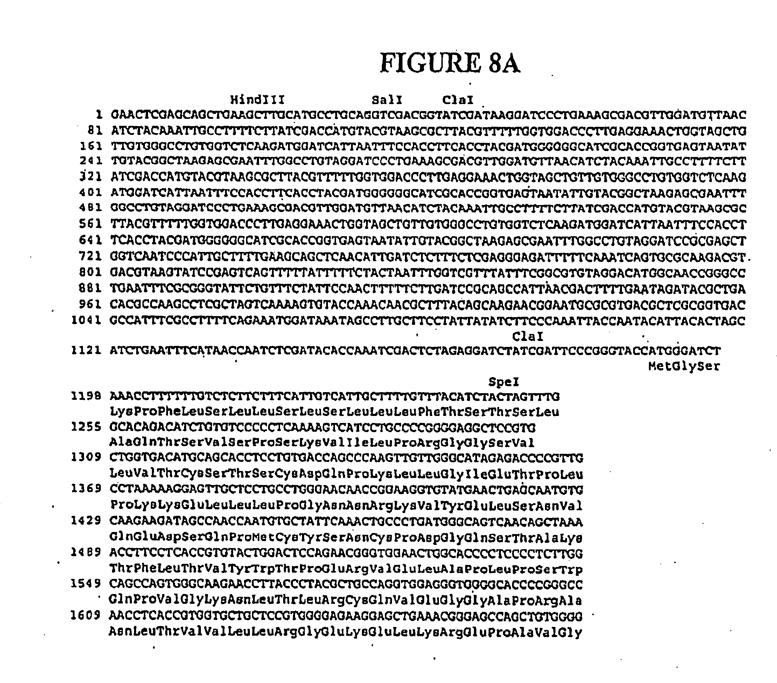

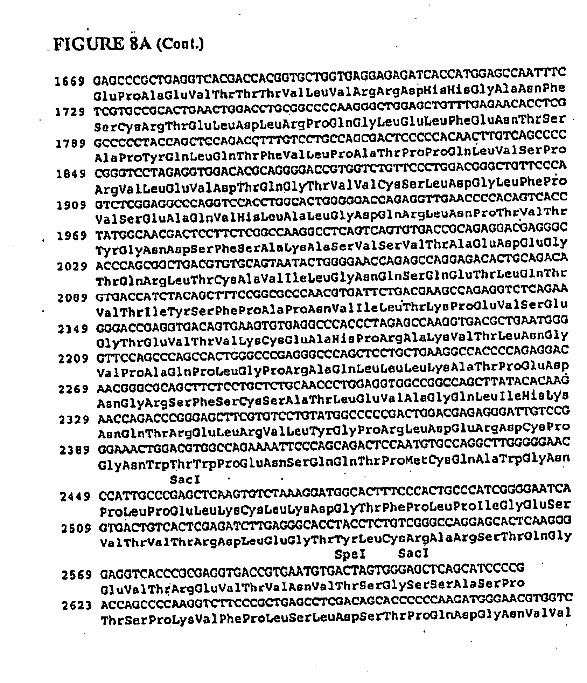

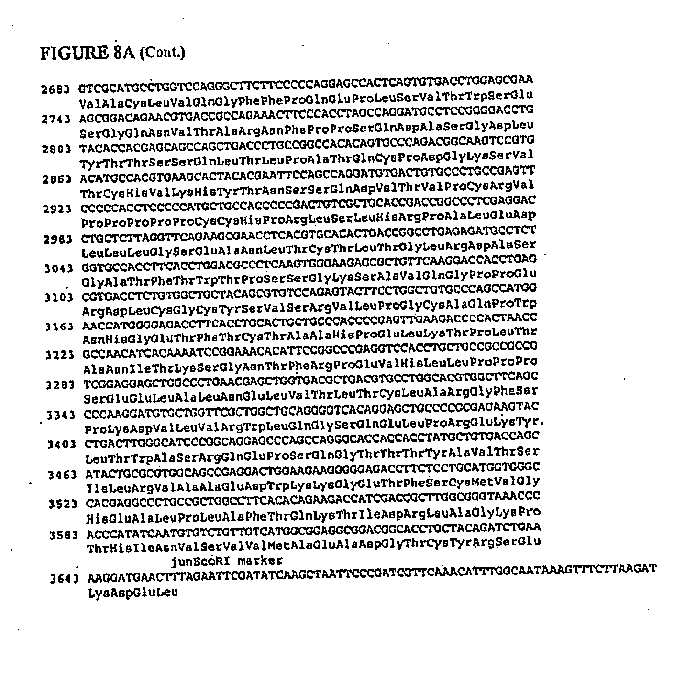

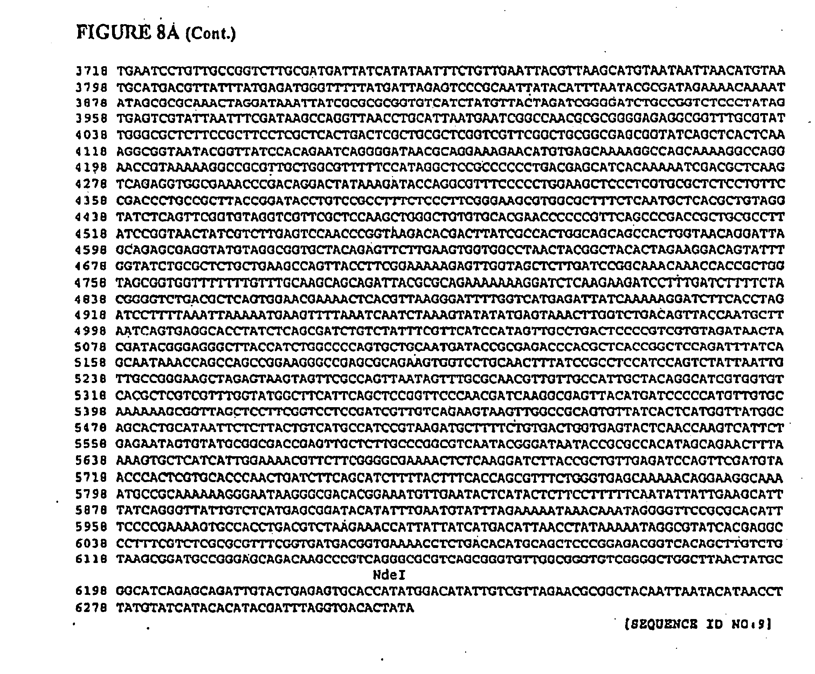

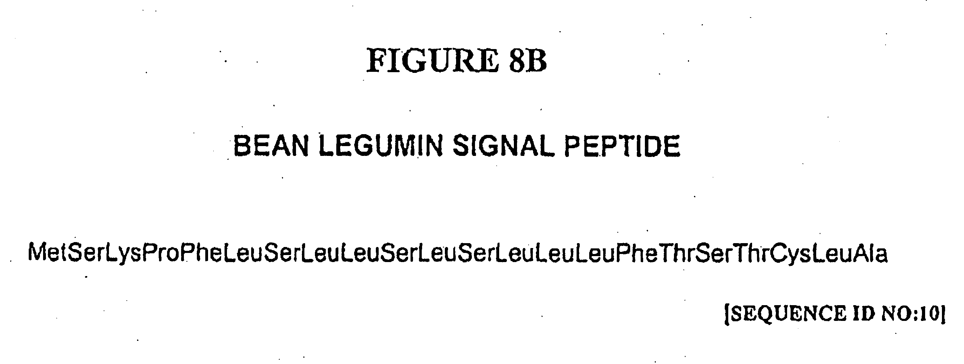

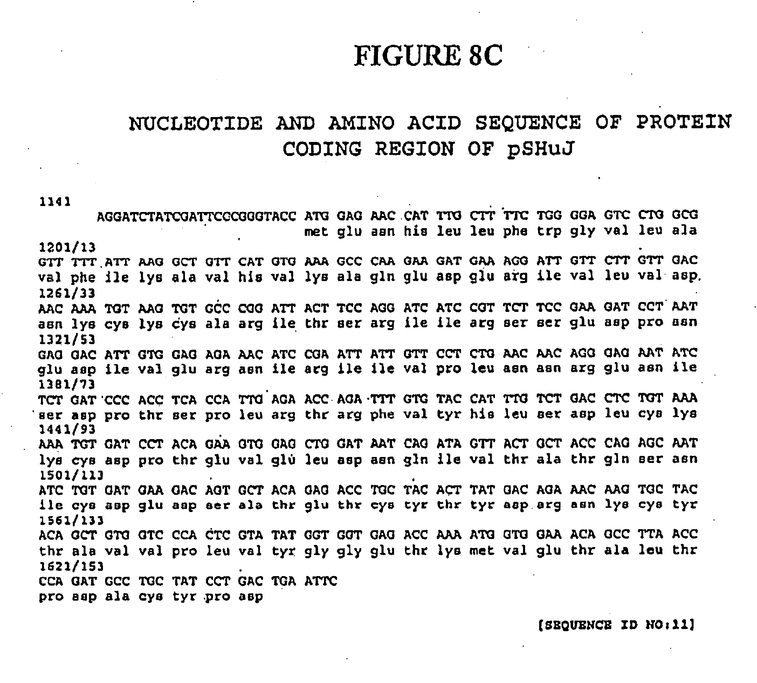

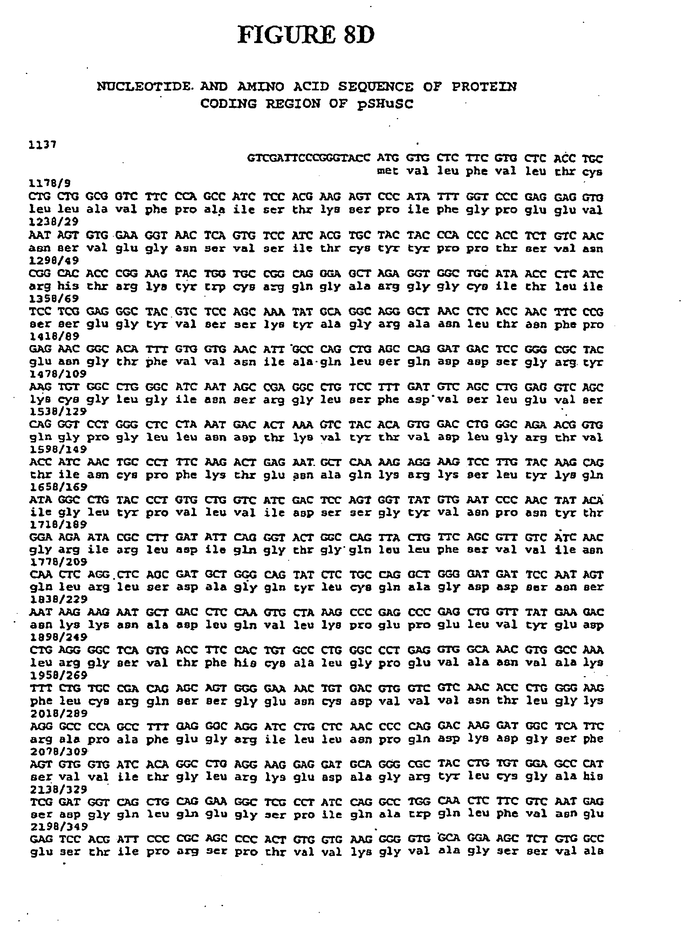

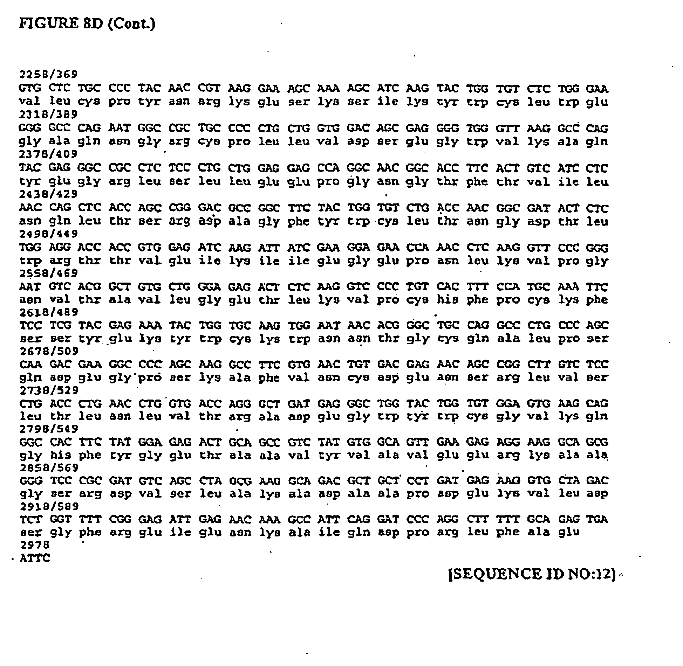

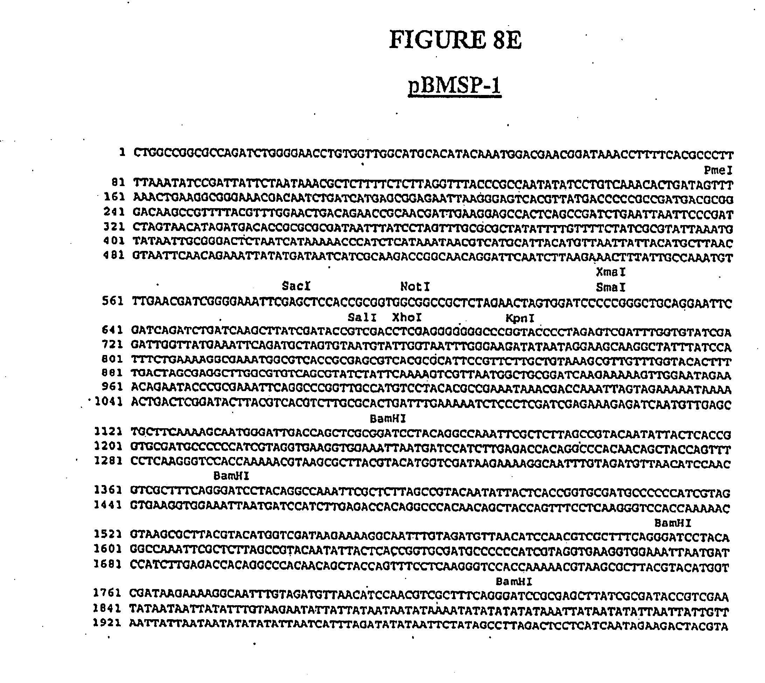

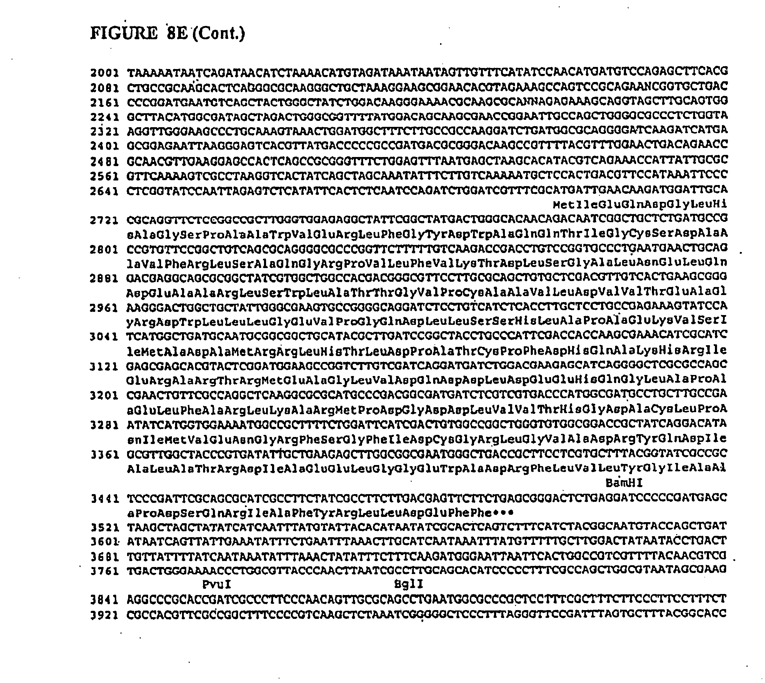

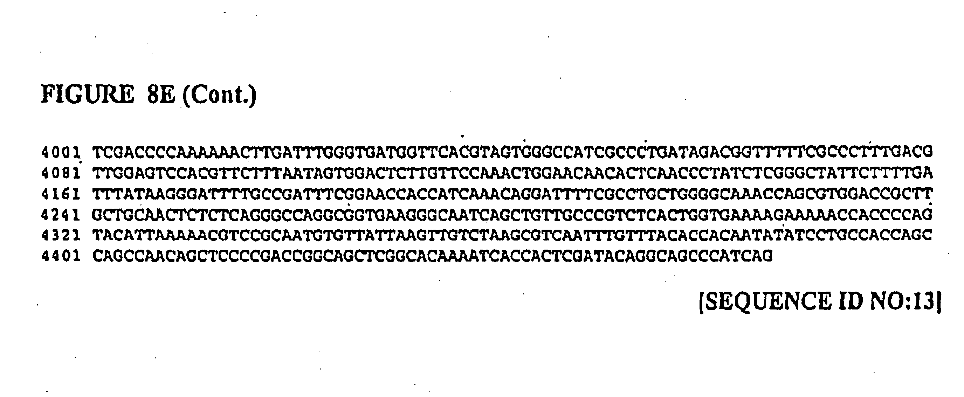

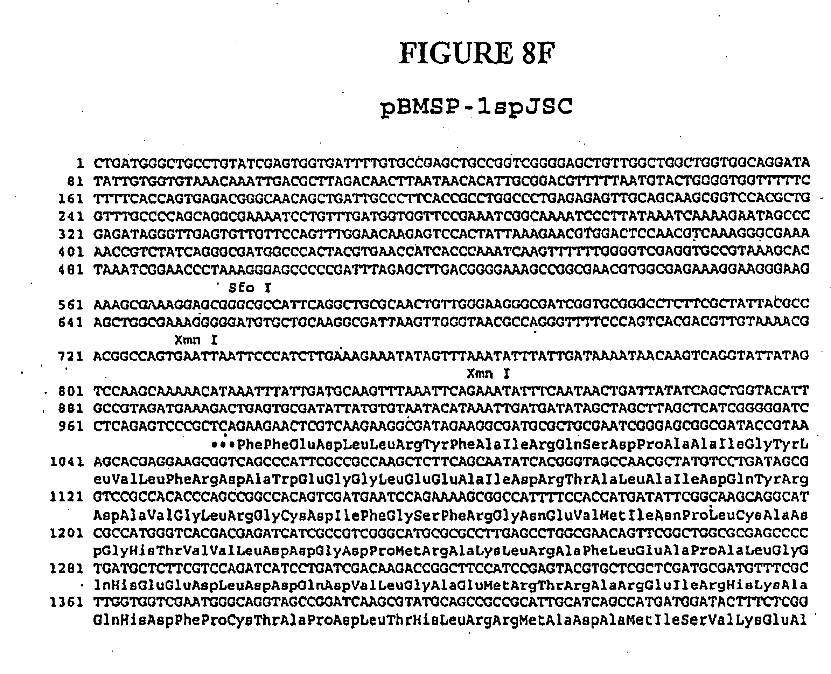

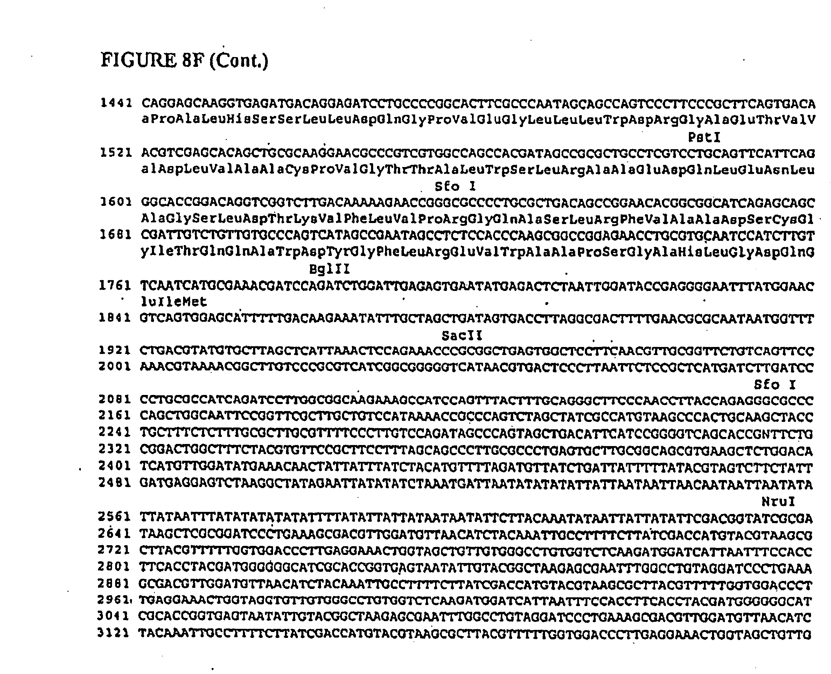

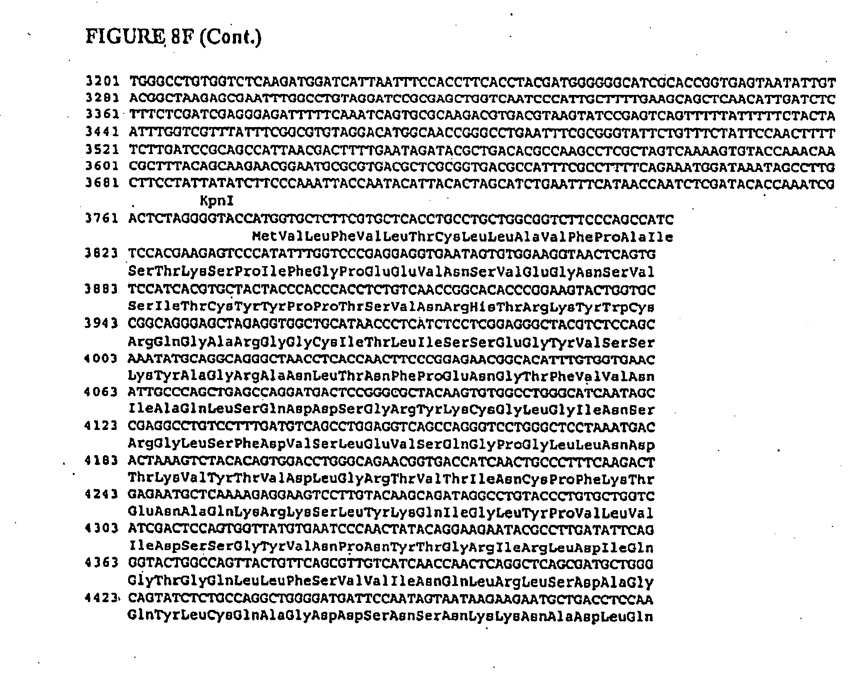

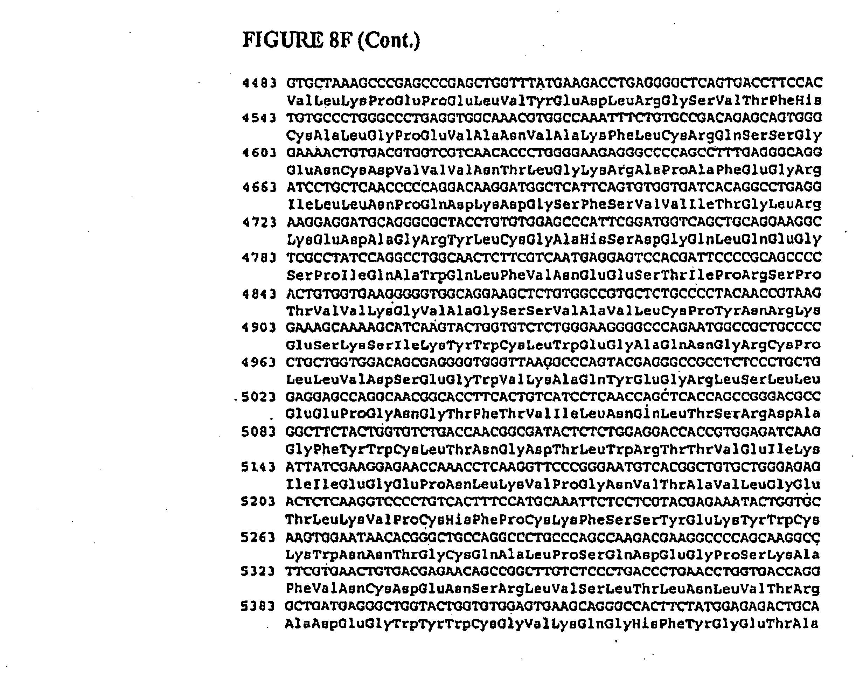

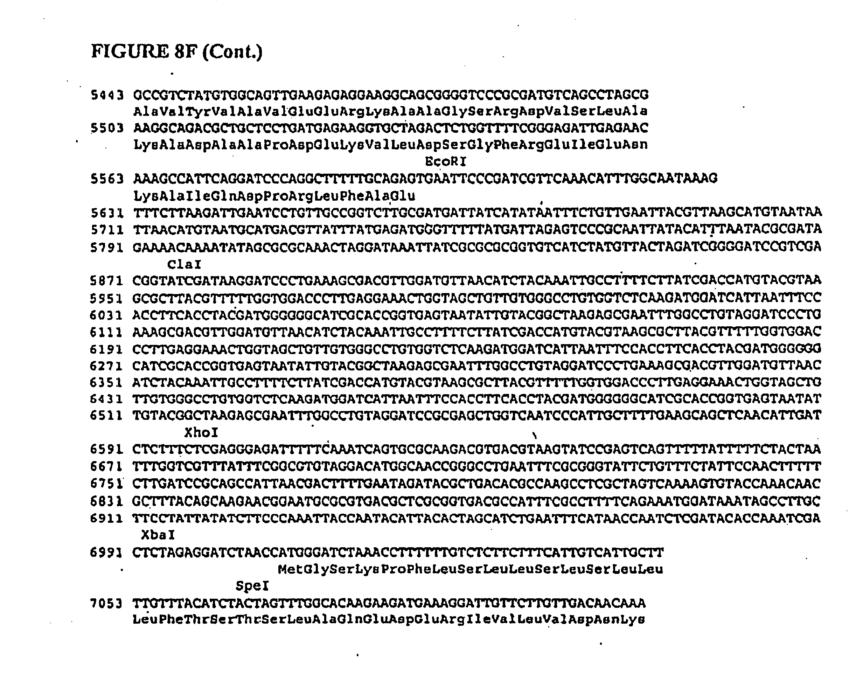

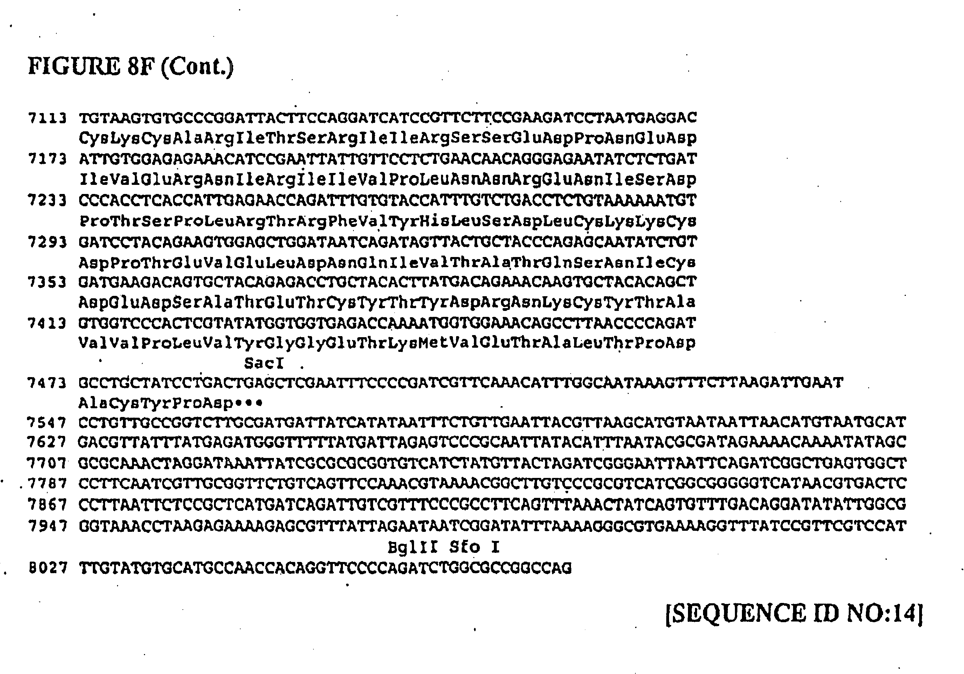

Constructs for the expression in plants of human J chain and secretory component, as well as a human IgA2 heavy chain, were developed. A heavy chain expression cassette vector was made and called pSSpHuA2 (See **FIG.1**). It contains sequence encoding a bean legumin signal peptide (Baumlein et al., Nucleic Acids Res. 14 (6), 2707-2720, 1986). The sequence of bean legumin is provided as GenBank Accession No. X03677, and the sequence of the bean legumin signal peptide is SEQ ID NO: 10 (also see **FIG. 8****)** and the IgA2m(2) constant region with SpeI and SacI sites in between, and the SuperMas promoter for driving the expression of a signal peptide and the constant regions of the human IgA2m(2) heavy-chain.

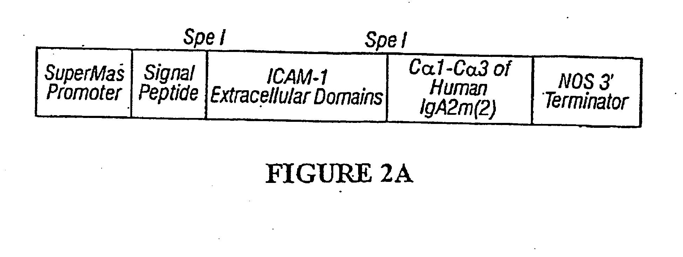

The amplified DNAs encoding the first five domains of human ICAM-1, and the Fc region of human IgA2m(2) were fused in a plant-expression cassette to make a chimeric ICAM-1 molecule expression construct, shown in **FIG. 2A****.** This was done by cloning the fragment encoding the five extracellular domains of ICAM-1 into vector pSSPHuA2 to generate pSSPICAMHuA2. The convenient restriction sites (5' SpeI and 3' Spe I) allowed the amplified fragment to be inserted between the signal peptide and the Cα1 domain. In the resulting construct, expression of the chimeric ICAM-1 molecule is under the control of the constitutive promoter "superMAS" (Ni et. al., 1995) and the nos 3' terminator region.

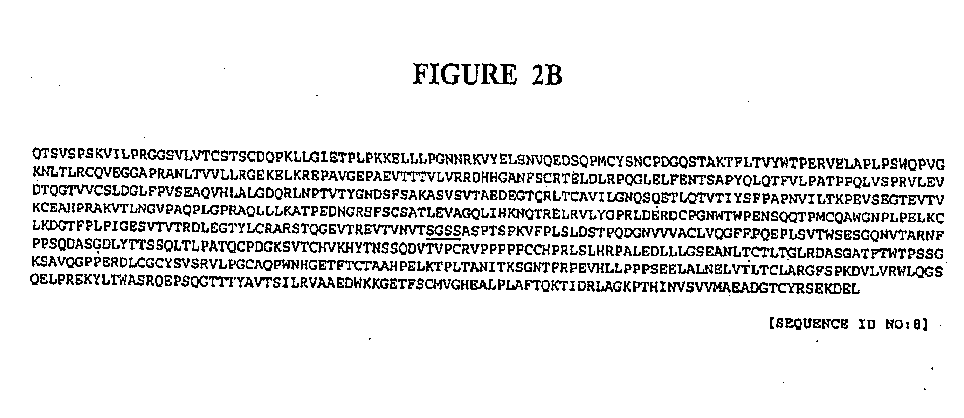

The resulting chimeric ICAM-1 molecule construct contains no variable region. Upon translation of the mRNA, signal peptide cleavage is predicted to deposit the ICAM-1-heavy chain fusion into the plant cell's endoplasmic reticulum (ER). DNA encoding an ER retention signal (RSEKDEL, SEQ ID NO: 5) was appended to the 3' end of the heavy-chain in order to boost the expression level of the construct. The amino acid sequence SEKDEL (SEQ ID NO: 4) is the consensus signal sequence for retention of proteins in the endoplasmic reticulum in plant cells. This sequence has been shown to enhance accumulation levels of antibodies in plants (Schouten et al., Plant Molecular Biology 30:781-793,1996). The amino acid sequence of the chimeric ICAM-1 molecule construct is shown in **FIG. 2****B.** The DNA sequence and translational frame of the construct was verified before it was used for particles bombardment.

It has been shown recently that assembly of J chain with IgA takes place in the Golgi apparatus (Yoo et al., J. Biol. Chem. 274:33771-33777, 1999), and so constructions of heavy chain without SEKDEL (SEQ ID NO: 4) have been made as well. The ICAM-1 fragment was cloned into an expression cassette containing the IgA2m(2) constant region without SEKDEL (SEQ ID NO: 4).

### Example 2: Expression of Assembled ICAM-1 Immunoadhesin in Plants (not of the invention)

### A. Immunoadhesin Expression Vectors

The plasmid pSSPICAMHuA2 [SEQ ID NO:9 and **FIG. 8A**] is 6313 bp in length. Nucleotides 49-1165 represent the Superpromoter (Ni et al., Plant Journal 7:661-676, 1995). Nucleotides 1166-3662 comprise a sequence encoding a human ICAM-1/human IgA2m(2) constant hybrid with linker sequences. A consensus Kozak sequence (Kozak, Cell 44(2):283-92, 1986) is included (nt 1186-1192) to enhance translation initiation, as well as the signal peptide from *V. faba* legumin (nt 1189-1257; Bäumlein et al., Nucleic Acids Reg. 14(6):2707-2720 (1986). The sequence of the human IgA2m(2) constant region (nt 3663-3633) has been previously published (Chintalacharuvu, et al., J. Imm. 152: 5299-5304, 1994). A sequence encoding the endoplasmic reticulum retention signal SEKDEL [SEQ ID NO:4] is appended to the end of the heavy chain (nt 3634-3654). Nucleotides 3663-3933 derive from the nopaline synthase 3' end (transcription termination and polyadenylation signal; Depicker et al., 1982). The remainder of the plasmid derives from the vector pSP72 (Promega).

The plasmid pSHuJ (**FIG. 8C**) is 4283 bp in length. Nucleotides 14-1136 represent the Superpromoter (Ni et al., Plant Journal 7:661-676, 1995) and nucleotides 1137-1648 are shown in **FIG. 8** [SEQ ID NO:11] and comprise a sequence encoding the human J Chain including the native signal peptide (Max and Korsmeyer, J Imm. 152:5299-5304, 1985) along with linker sequences. A consensus Kozak sequence (Kozak, Cell 44(2):283-92, 1986) is included (nt 1162-1168) to enhance translation initiation. Nucleotides 1649-1902 derive from the nopaline synthase 3' end (transcription termination and polyadenylation signal; Depicker et al., J Mol Appl Genet 1(6):561-73, 1982). The remainder of the plasmid derives from the vector pSP72 (Promega).

The plasmid pSHuSC (**FIG. 8D**) is 5650 bp in length. Nucleotides 13-1136 are derived from the Superpromoter (Ni et al., Plant Journal 7:661-676, 1995), and nucleotides 1137-2981 comprise a sequence encoding the human Secretory Component including the native signal peptide (Krajci, et al., Biochem. and Biophys. Res. Comm 158:783, 1994) along with linker sequences [SEQ ID NO: 12]. A consensus Kozak sequence (Kozak, Cell 44(2):283-92, 1986) is included (nt 1151-1157) to enhance translation initiation. Nucleotides 2982-3236 derive from the nopaline synthase 3' end, providing a transcription termination and polyadenlyation signal, described in Depicker et al., J Mol Appl Genet 1(6):561-73 (1982). The remainder of the plasmid derives from the vector pSP72 (Promega).

The plasmid pBMSP-1 [SEQ ID NO: 13 and **FIG. 8E**] is derived from pGPTV-KAN. Becker et al., in Plant Molecular Biology 20, 1195-1197, (1992), describe new plant binary vectors with selectable markers located proximal to the left T-DNA border, and the sequences outside of the left and right borders. Nucleotides 18-187 of pBMSP-1 represent the right T-DNA border, and nucleotides 1811-775 represent the superMAS promoter. Nucleotides 2393-2663 represent the NOS promoter (Depicker et al., J Mol Appl Genet 1(6):561-73, 1982), nucleotides 2698-, 3492 encode the NPTII gene (conferring resistance to kanamycin), and nucleotides 3511-3733 are the polyadenylation signal from *A. tumefaciens* gene 7 (Gielen et al., Embo J 3:835-46, 1984). Nucleotides 1768-976 encode the NPTII gene, and nucleotides 4317-4464 represent the left T-DNA border.

The plasmid pBMSP-1spJSC [SEQ ID NO: 14 and **FIG. 8F**] is a derivative of pBMSP, containing both J and SC under control of superpromoter. In this plasmid, nucleotides 1-149 represent the left T-DNA border. Nucleotides 955-733 are the polyadenylation signal from *A. tumefaciens* gene, nucleotides 1768-976 encode the NPTII gene (conferring resistance to kanamycin), and nucleotides 2073-1803 represent the NOS promoter. Nucleotides 2635-3768 represent the superMAS promoter, nucleotides 3774-5595 encode the Human Secretory component, and nucleotides 5603-5857 represent the NOS polyadenylation signal. Nucleotides 5880-6991 represent the superMAS promoter, nucleotides 7007-7490 encode the Human Joining Chain, and nucleotides 7504-7757 represent the NOS polyadenylation signal. Nucleotides 7886-8057 represent the right T-DNA border.

The plasmid pGPTV-HPT, encoding the enzyme conferring hygromycin resistance, is available commercially from the Max-Planck-Institut für Zuchtungsforschung (Germany). It is described by Becker in Plant Molecular Biology 20, 1195-1197 (1992).

### B. Plant Transformation and ICAM-1 Immunoadhesin Expression in Plants

The expression cassettes described above were used to produce the assembled immunoadhesin in plants. Plasmids pSSPICAMHuA2, pSHuJ, pSHuSC and pBMSP-1 were co-bombarded into tobacco leaf tissue (*N. tabacum* cultivar Xanthi) and transformed microcalli were selected on nutrient agar in the presence of kanamycin. Individual microcalli, indicative of independent transformation events, were dissected from the parent tissue and propagated on nutrient agar with kanamycin.

The callus tissues were screened for transgene expression. Callus #7132 was shown to express a chimeric ICAM-1 immunoadhesin and J chain by immunoblotting and PCR (data not shown). This callus did not possess DNA encoding the SC. The callus grew well in culture and, upon accumulation of sufficient mass, #7132 was bombarded again, this time with two of the plasmids described above, PBMSP-1 SpJSC, containing expression cassettes for both the J chain and SC and pGPTV-HPT, containing an expression cassette for the hpt I gene which confers hygromycin resistance. After a period of selection and growth on nutrient agar, several independent transformants were identified, by immunoblotting that expressed the chimeric ICAM-1 molecule, the J chain and SC in several states of assembly.

**FIG. 3** illustrates the expression of the chimeric ICAM-1 molecule in independently transformed tobacco calli. **FIG. 3A** shows immunoblots of non-reducing SDS-polyacrylamide gels on which samples containing different transformed tobacco calli (C) and aqueous extracts (Aq) were run and probed for the presence of human ICAM. The solubility of the immunoadhesin assured us that extraction could be easily performed, and the similarity of signals leads us to believe in the reproducibility of expression. **FIG. 3B** shows immunoblots of nonreducing SDS-polyacrylamide gels containing various fractions of partially purified immunoadhesin from callus Rhi107-11. The blots were probed with antibodies against human ICAM (∼ICAM), human IgA heavy chain (∼α), human secretory component (∼SC) and human J chain (∼J). Secondary, enzyme-conjugated antibodies were employed as necessary to label immuno-positive bands with alkaline phosphatase. The specificity of immuno-blotting was further verified by a failure to detect immuno-positive bands in extracts of non-expressing calli (not shown). The expected MW for a dimerized chimeric ICAM-1 molecule, without glycosylation, is 173,318; this form is likely present in the band migrating just below the 250 kD marker since it is immuno-positive for ICAM-1 and heavy-chain. This band is also immune-positive for SC (total expected MW of ∼248 kD) but not for J chain which is somewhat unexpected given the canonical pathway for SIgA assembly, which involves 2 cell types (in mammalian) and requires the presence of J chain prior to assembly of SC. A tetrameric immunoadhesin, containing a single molecule of J chain and a single molecule of SC, has an expected MW of ∼440 kD, prior to glycosylation. Several species with molecular weights well in excess of 200 kD, immuno-positive with all four probes, are readily apparent.

Bombardment with DNA-coated microprojectiles is used to produce stable transformants in both plants and animals (reviewed by Sanford et al., Meth. Enz. 217:483-509,1993). Particle-mediated transformation with the vectors encoding the immunoadhesin of the present invention was performed using the PDS-1000/He particle acceleration device, manufactured by Bio-Rad. The PDS-1000/He particle acceleration device system uses Helium pressure to accelerate DNA-coated microparticles toward target cells. The physical nature of the technique makes it extremely versatile and easy to use. We have successfully transformed tobacco with all four components of a secretory IgA simultaneously.

The basic biolistic procedure was performed as follows: A stock suspension of microprojectiles was prepared by mixing 60 mg of particles in 1 ml of absolute ethanol. This suspension was vortexed and 25-50 *µ*l was removed and added to a sterile microcentrifuge tube. After microcentrifuging for 30 seconds the ethanol was removed and the pellet resuspended in 1 ml sterile water and centrifuged for 5 minutes. The water was then removed and the pellet resuspended in 25-50 *µ*l of DNA solution containing a mixture of plasmid DNAs, usually, but not always in equimolar amounts. The amount of plasmid added varied between 0.5 ng and 1 *µ*g per preparation. The following were added sequentially: 220 *µ*l of sterile water, 250 *µ*l of 2.5M CaCl2, and 50 *µ*l of 0.1M spermidine. This mixture was vortexed for at least 10 min and then centrifuged for 5 min. The supernatant was removed and the DNA/microprojectile precipitated in 600 *µ*l of absolute ethanol, mixed and centrifuged 1 min. The ethanol was removed and the pellet resuspended in 36 *µ*l of ethanol. Ten *µ*l of the suspension was applied as evenly as possible onto the center of a macrocarrier sheet made of Kapton (DuPont) and the ethanol was evaporated. The macrocarrier sheet and a rupture disk were placed in the unit. A petri dish containing surface-sterilized tobacco leaves was placed below the stopping screen. The chamber was evacuated to 28-29 mm Hg and the target was bombarded once. The protocol has been optimized for tobacco, but is optimized for other plants as well by varying parameters such as He pressure, quantity of coated particles, distance between the macrocarrier and the stopping screen and flying distance from the stopping screen to the tissue.

Expression cassettes for chimeric ICAM-1 molecules were also assembled in binary vectors for use in *Agrobacterium-*mediated transformation. An *Agrobacterium* binary vector designed for expression of both human J chain and human secretory component, as well as kanamycin resistance, was introduced into A. *tumefaciens* strain LBA4404. The chimeric ICAM/IgA molecule in another binary vector was also used to transform LBA4404. Overnight cultures of both strains were used for simultaneous "co-cultivation" with leaf pieces of tobacco, according to a standard protocol (Horsch et al., Science 227:1229-1231, 1985).

A standard protocol for regeneration of both bombarded and *Agrobacterium*-transformed tobacco leaf disks was used (Horsch et al., Science 227:1229-1231, 1985). Because transformed plants, regenerated from bombarded tissue, frequently undergo gene-silencing upon maturation, transgenic tobacco plants were prepared via *Agrobacterium-*mediated transfonnation, which gives a higher yield of expressing, mature plants.

### Example 3: Purification of Assembled ICAM-1 Immunoadhesin (not of the invention)

The immunoadhesin expressed according to Example 2 was purified. Calli were grown in large amounts to facilitate the development of extraction procedures. A partial purification schedule provided a stable concentrate, available in a variety of buffer conditions, for investigation of subsequent chromatographic techniques for the further purification of the immunoadhesin (See FIG. 3). Calli were extracted in a juicer, which crushes tissue between two stainless-steel gears, while bathed in a buffer containing sodium citrate (0.6 M, pH 7.4) and urea (final concentration of 2 M). The juice (∼1 ml/g fresh weight) was precipitated, after coarse filtration through cheesecloth, with 0.67 volumes of saturated ammonium sulfate. A green pellet was collected after centrifugation and thoroughly extracted, in a small volume of 50 mM sodium citrate (pH 6.6), with a Dounce homogenizer. After additional centrifugation, a clear brown supernatant was collected and partially purified, during buffer exchange in a de-salting mode, by passage through a Sephadex G-100 column. The desalting/buffer exchange step has allowed preparation of a partially purified concentrate (∼0.2 ml/g fresh weight callus) in a desirable buffer; the G-100 column was eluted with 0.25× phosphate buffered saline. This eluate appeared to be stable for at least 10 days at 2-8° C.

### Example 4: The ICAM-1 Immunoadhesin Inhibits Human Rhinovirus Infectivity (not of the invention)

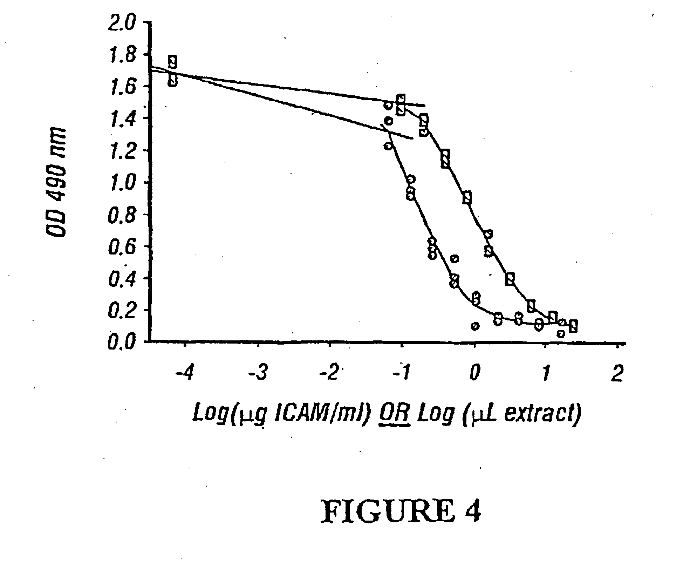

The infectivity of cells by human rhinovirus was demonstrated to be inhibited by the immunoadhesin prepared according to Example 3. Callus extract prepared according to Example 3 successfully competed for binding of an anti-ICAM monoclonal antibody to soluble ICAM-1. **FIG. 4** shows the data from an enzyme-linked immunosorbent assay (ELISA). For the assay, 96-well plates were coated with 0.25 *µ*g soluble ICAM-1/ml. The squares represent the increasing concentrations of sICAM and the circles represent the increasing amounts of callus extract (sterile filtered fraction from G-100) used to compete with the adhered ICAM for a constant amount of a mouse (anti-human ICAM) antibody. After washing the wells, adherent mouse antibody was detected with an anti-mouse antibody conjugated to horseradish peroxidase. Adherent enzyme activity was measured at 490 nm, with ortho-phenylene diamine as a substrate. The data (squares, sICAM; circles, Extract) are well described by siginoids of the form OD490=y=y0+a/[1+e -{(x-x0)/b}], where a=y max, y0=y min, b=the slope of the rapidly changing portion of the curve and x0=the value of x at the 50% response level. Relative to soluble ICAM-1, the immunoadhesin extract tested here contains the equivalent of 250 *µ*g ICAM/ml; this is an overestimate due to expected avidity effects of the dimeric and tetrameric assemblies of the ICAM-1-heavy-chain fusions. Thus, this ELISA demonstrated that the immunoadhesin competes with soluble ICAM-1 for binding to an anti-ICAM mAb.

The competitive ELISA allows for quantitative assessment of the recovery of activity by comparing the normalized amounts of various fractions required to give a 50% response. Upon purification, the titer of an immunoadhesin preparation may be expressed as a reciprocal dilution, or the number of milliliters to which a milligram of immunoadhesin must be diluted in order to give a 50% response. This ELISA will facilitate the development of a purification process for the immunoadhesin.

A cytopathic effect assay (CPE) demonstrated the specific ability of the partially purified immunoadhesin to inhibit the infectivity of human cells by human rhinovirus (FIG. 5). Rhinovirus serotype HRV-39 was pre-incubated with human ICAM-1, an ICAM/IgA fusion (gift of Dr. Tim Springer), or with extracts from calli either expressing our ICAM-1/SIgA immunoadhesin or another, different, antibody before plating each of the mixtures with HeLa S3 cells at 33° C. After 3 days, viable cells were fixed and stained with a methanolic solution of Crystal Violet; the optical density at 570 nm provides a proportional measure of cell viability.

Two extracts derived from Rhi107-11, containing the immunoadhesin, clearly inhibited the virus' ability to infect and kill HeLa S3 cells (**FIG. 5A**, right side-up and upside-down triangles). Because the extracts were only partially purified, we also assayed a similarly prepared extract that contained a human IgA2m(2) directed against Doxorubicin, a chemotherapeutic agent. That extract, containing a similar immunoglobulin with an unrelated binding specificity, was unable to inhibit the infectivity of the rhinovirus and demonstrates that expression of the ICAM-1-heavy-chain fusion confers specificity to the inhibition. The CPE assay demonstrated, as expected, the differential ability of soluble ICAM-1 and an (IC1-5/IgA; Martin, et al., 1993) to inhibit viral infectivity (**FIG. 5B**). The insert in **FIG. 5B** is the scale expansion in the range of the IC₅₀ for soluble ICAM-1 (1.35 *µ*g/ml) and for the IC1-5/IgA (0.12 *µ*g/ml; 11.3 fold less).

### Example 5: Production and Purification of Immunoadhesins for Clinical and Toxicological Studies (not of the invention)

Production of sufficient inununoadhesin for the proposed clinical and toxicological needs is performed by making transgenic tobacco plants. The transgenic plants which express the immunoadhesin (without an ER retention signal) are generated by *Agrobacterium-*mediated transformation. The absence of an ER retention signal is anticipated to enhance assembly since the nascent SIGA is processed through the entire Golgi apparatus, including, in particular, the trans-Golgi, where SC is covalently linked to dIgA as suggested by pulse-chase experiments (Chintalacharuvu & Morrison, Immunotechnology 4:165-174, 1999). Because *Agrobacterium-*mediated transformation is much more likely to generate plants with consistent levels of transgene expression, it is likely that progeny of these plants will be used for the production of clinical grade immunoadhesin.

In order to maximize expression levels, and create a true-breeding line, it is desirable to create homozygous plants. The highest producing plants (generation T0) can self-fertilize in the greenhouse before seed is collected. One quarter of the T1 plants are expected to be homozygous. These are grown in the greenhouse and seed samples from several plants are separately germinated on medium containing kanamycin. All the progeny (T2) from homozygous positive plants are expected to be green. Some of the progeny of heterozygous plants are expected to be white or yellowish. Homozygosity is confirmed by back-crossing to wild-type and immunoblotting extracts of the progeny.

Harvesting and processing may be continuously meshed during a production campaign, especially since multiple harvests may be obtained from a single planting, i.e. plants cut to soil level for one harvest are regrown for subsequent harvests. In developing a sense of scale for the production of immunoadhesin it is necessary to decide on the required amount of finished immunoadhesin, account for expression levels (mg immunoadhesin present/kg fresh weight tobacco), know the growth rate of the plants and the expected weight of the average plant, and the overall yield of the purification schedule (set at 20%). Setting the overall need at 3 g of finished immunoadhesin requires preparing for 4 harvests, each with an expected yield of 1 g of finished immunoadhesin.

Given these targets and parameters, the necessary number of plants and hence the space requirements for plant growth is determined. **FIG. 6** shows an evaluation of the production necessities for making 1 gram of finished immunoadhesin. In this diagram, the number of plants needed for 1 g of immunoadhesin, at 20% yield, at expected levels of expression and plant weight is illustrated. At different levels of immunoadhesin expression (mg/kg fresh weight) and overall recovery (set at 20%), the weight of each plant, and so the total number of plants, may be determined for a specified production target (1 g/harvest) within a window (dotted square) of reasonable possibilities. The number of required plants decreases, inversely, with the number of specified growth and re-growth periods. The expected biomass production, a function of time and growth conditions, influences the time to harvest and the time between harvests. These growth periods can be adjusted to the realities of the purification schedule by staggering planting and harvesting dates. For example, 1 g of finished immunoadhesin from plants with a reasonable expression level, of 100 mg of immunoadhesin/kg fresh weight, require 250 plants when harvested at a weight of 200 g/plant (∼80 days post germination). At this scale, these plants require about 10 m² of growing space and are harvested twice over 150 days.

Processing 50+ kg of biomass at a time requires several moderately large-scale operations which all have counter-parts in the food-processing industry. These include bulk materials handling, size reduction, juicing and filtration. A Vincent Press and a Durco filtration system are used to efficiently process these quantities. The juicing step employs a proven and simple buffer of sodium citrate and urea. These components buffer the extract, help prevent the oxidation of phenolics and their association with proteins (Gegenheimer, Methods in Enzymology 182:174-193, 1990; Loomis, Methods in Enzymology, 31:528-544, 1974; Van Sumere, et al., The Chemistry and Biochemistry of Plant Proteins, 1975.) and ensure the solubility of the immunoadhesin during a subsequent acid precipitation.

Filtration of acid-insoluble lipid and protein (∼90% of the total) is followed by tangential flow ultrafiltration to concentrate the immunoadhesin and to remove small proteins, especially phenolics. Diafiltration enhances the removal of small molecules and exchanges the buffer in preparation for short-term storage and subsequent chromatography. Either SP-Sepharose (binding at pH 5.0 or below) or Q-Sepharose (binding at pH 5.5 or above) are among the ion-exchanges that can be used for filtering immunoadhesin. They are readily available, scalable, robust and have high capacities. In particular, they are available for expanded-bed formats, which reduce the stringency of prior filtration steps. Cation-exchange chromatography, which can be more selective than anion-exchange chromatography, is used first. The immunoadhesin is purified from the several species of protein potentially present, to the point where at least 95% of the protein is in the form of ICAM-1/IgA, ICAM-1/dIgA or ICAM-1/SIgA, as the presence of di- and tetra-valent ICAM-1 domains are critical for potent anti-viral activity. Purified immunoadhesin is then tested for acceptable levels of endotoxin, alkaloids such as nicotine and for bio-burden. In addition, potency levels (defined by ELISA and CPE assays), protein concentration, pH and appearance are monitored. Subsequently, the stability of the clinical lots of immunoadhesin is determined, to ensure that patients receive fully potent immunoadhesin. Even partially purified extracts have been found to be stable for 10 days when refrigerated. The titer and potency of clinically formulated immunoadhesin (in phosphate-buffered saline), when stored at -20° C, 2-8° C, and at 37° C, over a period of 3 to 6 months, is also tested.

### Example 6: The Immunoadhesins Have Plant-Specific Glycosylation (not of the invention)

The immunoadhesins produced are analyzed to determine the pattern of glycosylation present. Cabanes-Macheteau et al.,Glycobiology 9(4):365-372 (1999), demonstrated the presence of several glycosyl moieties, typical of plants, on a plant-expressed antibody construct. Their methods are used to demonstrate that the immunoadhesins produced according to Example 1, 2 and 3 have a plant-specific glycosylation pattern. We anticipate that this diversity will also be a source of variability for immunoadhesin. Since crude extracts have been shown to have anti-viral activity in vitro (data not shown), glycosylation, as such, does not appear to affect potency. N-linIced glycosylation (**FIG. 2** shows that there are fifteen potential sites on the chimeric ICAM-1 molecule alone) probably contributes to the diversity of bands seen in immuno-blots. Immunoadhesin preparations are digested with N-Glycosidase A, before blotting, showing that the difference in banding patterns collapse into fewer, discrete bands. In this way, glycoforms are initially characterized with reducing and non-reducing polyacrylamide gels. In addition, digested and mock-digested fractions are tested in the CPE assay and competition ELISA, demonstrating the effect of N-linked glycosylation on potency and titer in vitro.

### Example 7: The ICAM-1 Immunoadhesin Inactivates Human Rhinovirus (not of the invention)

The immunoadhesin prepared according to Examples 1, 2 and 3 is assayed for its ability and to inactivate HRV by binding to the virus, blocking virus, entry, and inducing the formation of empty virus capsids. To measure binding of the immunoadhesin to HRV, the immunoadhesin is incubated with [³H]leucine-labeled HRV-39 for 30 min and then added to HeLa cells for 1 hr. After washing, cells and bound virus are detached with Triton X-100 and [³H] measured in a scintillation counter.

Inactivation of HRV-39 by incubation with the immunoadhesin is compared with HRV inactivation by sICAM-1. HRV-39 is not directly inactivated to a significant extent (<0.5 log 10 reduction in infectivity) by incubation with monomeric sICAM-1, while incubation with IC1-SD/IgA reduced infectivity approximately 1.0 log10 (Arruda, et al., Antimicrob. Agents Chemother. 36:1186-1191, 1992; Crump, et al., Antimicrob. Agents Chemother. 38:1425-7, 1994). In order to test the ability of the immunoadhesin to inactivate HRV-39,106 50% tissue culture infective doses (TCID₅₀) of HRV-39 are incubated in medium containing a concentration of sICAM-1 or immunoadhesin equal to ten times the IC50 of each molecule for that virus, or in plain medium, for 1 hr at 33° C. on a rocker platform. Each virus-immunoadhesin or virus-medium mixture are then diluted serially in ten-fold dilutions, and the titer determined on HeLa cells in 96-well plates.

The effect of the immunoadhesin on HRV attachment to host cells is tested by inoculating HeLa cells with HRV-39 at a MOI of 0.3 in the presence or absence of the immunoadhesin. Absorbance proceeds for one hour at 4° C., the cells are washed, and media is replaced plus or minus the immunoadhesin. Cells are incubated for ten hours at 33° C. (to allow one round of replication), and virus are harvested by freeze/thawing the cells. The virus is titered on HeLa cells.

ICAM-IGA (IC1-5D/IgA) is more efficient than Sicam-1 at inducing conformational changes in HRV, leading to the formation of empty, non-infectious viral particles (Martin, et al. J. Virol. 67:3561-8, 1993). To examine the ability of the immunoadhesin produced according to Examples 1, 2 and 3 to induce conformational changes in HRV, causing release of viral RNA, purified immunoadhesin is incubated with [³H]leucine-labeled HRV-39 for 30 min and then the virus is overlayed onto a 5 to 30% sucrose gradient. Following centrifugation for 90 min at 40,000 rpm, fractions are collected, [³H] measured, and fractions assessed for infectivity. (Intact HRV sediments at 149S on a sucrose gradient while empty capsids lacking RNA sediments at 75S (Martin, et al. J. Virol. 67:3561-8, 1993)). Due to its increased valence, we expect the ICAM/SIgA immunoadhesin is more efficient at inducing empty non-infectious particles than ICAM-IgA.

The inhibitory effect of purified immunoadhesin on a panel of both major and minor (that do not use ICAM-1 as a receptor) HRV serotypes will be examined using the CPE assay. The ability of ICAM-1 to inhibit HRV infection varies among viral isolates. It has been shown (Crump, et al., Antimicrob. Agents Chemother. 38:1425-7, 1994) that the EC₅₀ for sICAM-1 varies from 0.6 *µ*g/ml to >32 *µ*g/ml when tested on a panel of HRV major receptor serotypes assay using HeLa cells. Our panel includes nine major serotypes (HRV-3, -13, -14, -16, -23, -39, -68, -73, and -80) and the minor receptor serotype HRV-1A.

### Example 8: Clinical Studies Demonstrating the Ability of the ICAM-1 Immunoadhesin to Reduce Infectivity in Humans: Dose Escalation Tolerance Study (not of the invention)

The immunoadhesin of the present invention is tested in two randomized controlled trials to determine the effect of intranasal administration of the immunoadhesin on infection, IL-8 response, and illness in experimental rhinovirus colds. These two studies evaluate the immunoadhesin taken by subjects before or after rhinovirus inoculation. The clinical protocols used here are based on protocols previously used by in evaluation of a recombinant soluble ICAM-1 molecule for efficacy against rhinovirus infection (Turner, et al., JAMA 281:1797-804, 1999).

### A. Subjects

Subjects are recruited from university communities at the University of Virginia, Charlottesville. Subjects are required to be in good health, non-smokers, and between the ages of 18 and 60 years. Subjects are excluded if they have a history of allergic disease or nonallergic rhinitis, abnormal nasal anatomy or mucosa, or a respiratory tract infection in the previous 2 weeks. Pregnant or lactating women or women not taking medically approved birth control are also excluded. In the experimental virus challenge study (Phase I/II, see below), subjects are required to be susceptible to the study virus as evidenced by a serum neutralizing antibody titer of 1:4 or less to the virus, determined within 90 days of the start of the trial.

### B. Study Medication

The immunoadhesin of the present invention is formulated as a phosphate-buffered saline (PBS) spray solution containing 2.6 mg/ml. The placebo consists of PBS and is identical in appearance to the active preparation. The solutions are administered using a medication bottle equipped with a metered nasal spray pump. The pump delivers 70 *µ*l of solution containing 183 *µ*g of the immunoadhesin with each spray. The medication is administered as two sprays per nostril, six times daily (at 3-hour intervals) for a total daily dose of 4.4 mg. This is the same dose, in mg protein/day, as was used for soluble ICAM-1 in the tremacamra study infection (Turner, et al., JAMA 281:1797-804,1999). A mole of the immunoadhesin has about twice the mass as a mole of sICAM-1. However, given the differences in *in vitro* activity between sICAM-1 and ICAM/IgA fusions, the immunoadhesin is many-fold more effective on a molar basis than sICAM-1. Thus, this amount is a conservative calculation of what is necessary. This amount is used, except in the event that the dose escalation study reveals problems at this dose.

### C. Study Design

Single ascending dose and multiple ascending dose studies are used to evaluate the safety of the immunoadhesin. In each case, three subjects are evaluated at each dosage level, two receiving the immunoadhesin and one receiving placebo. In the single ascending dose study, four dosage levels are evaluated. The lowest individual dose is half the anticipated dose to be used in the challenge study, and the highest individual dose is twice the anticipated challenge study dose. The dosage levels are as follows: one spray in each nostril (366 *µ*g total), two sprays in each nostril (732 *µ*g total), three sprays in each nostril (1098 *µ*g total), four sprays in each nostril (1464 *µ*g total).

The same dosage levels are used in the multiple ascending dose study. Subjects receive doses every three hours (six times per day) for five days. In both studies subjects are evaluated at each dosage level, staggering the start of each subsequent level until it is clear that there is no acute toxicity at the previous level. All subjects return for a single dose 21 days after the first dose, and then for a follow-up at six weeks (for determination of serum antibody against the immunoadhesin).

A separate group of twelve subjects is given one dose of two sprays in each nostril (732 *µ*g total), and nasal lavage is done at 1, 2, 4, 8 and 16 hours (two subjects at each time point). Washings are assayed at Panorama Research by ELISA for the immunoadhesin in order to calculate its in vivo half-life. The total amount of the immunoadhesin to be used in the dose escalation and half-life determination studies (on a total of 28 subjects) will be approximately 270 mg.

### D. Safety Evaluations

In addition to routine adverse event recording, the safety of the immunoadhesin is assessed in three ways. First, prior to the first dose and after the last dose the investigators perform a visual examination of the nasal mucosa, in particular looking for signs of irritation or inflammation. Any visible changes are noted. Second, standard blood safety evaluations are done on samples collected prior to treatment and after the last dose on study days 1, 4, and 8 (and 21 in the multiple ascending dose study). Third, serum samples are saved, frozen, and used to determine if the immunoadhesin is able to pass through the nasal mucosa into the blood. This is accomplished in two ways. First, the presence the immunoadhesin in serum samples is measured by ELISA. In this assay, anti-human IgA antibodies adsorbed to microtiter plates capture any the immunoadhesin in the serum, which are detected by an anti-ICAM antibody. The sensitivity of the assay is determined using normal human serum samples spiked with known concentrations of the immunoadhesin. Alternatively, anti-ICAM antibodies can be adsorbed to plates to capture the immunoadhesin in the serum that would be detected by anti-IgA. Second, the presence of an immune response to the immunoadhesin is assayed with an ELISA method that uses the immunoadhesin adsorbed to microtiter plates. Any anti-immunoadhesin antibodies in the serum bind, and are detected with anti-human IgG or anti-human IgM. Pretreatment and post-treatment serum samples are compared, and any change in titer is considered evidence of uptake of the immunoadhesin. If there is any positive evidence of anti-immunoadhesin antibodies, additional assays will be done to distinguish between anti-ICAM-1 and anti-IgA activity.

Patients are screened for the development of an allergic reaction to the immunoadhesin. (In previous studies, there were no episodes of adverse reactions with soluble ICAM applied topically in the nose or plantibodies applied topically in the oral cavity.) Individuals exhibiting symptoms of nasal allergy are tested for anti-immunoadhesin-specific IgE antibodies in nasal lavage fluids using a sensitive two-step ELISA (R & D Systems).

### E. Statistical Analysis.

The sample size for these studies is based on previous studies using the rhinovirus challenge model. The sample size planned for the protection studies should be adequate to detect a reduction in the incidence of clinical colds from 75% in the placebo groups to 25% in the active treatment groups at 1-sided levels of α=0.05 and 1-β=0.80. In addition, the sample size should be adequate to detect a change in the total symptom score of 5 units assuming an SD of 5.8 units.

### Example 9: Clinical Studies Demonstrating the Ability of the Immunoadhesin to Reduce Infectivity in Humans: Challenge Studies (not of the invention)

Challenge studies are used to demonstrate that treatment with the immunoadhesin of the present invention protect against clinical colds or reduce cold symptoms after viral challenge.

### A. Challenge Virus

The challenge virus used for this study is rhinovirus 39 (HRV-39). Rhinovirus type 39 is a major group of rhinovirus that requires ICAM-1 for attachment to cells. The challenge virus pool is safety-tested according to consensus guidelines (Gwaltney, et al., Prog. Med. Virol. 39:256-263,1992). All subjects are inoculated with approximately 200 median tissue culture infective dose (TCID₅₀). The virus are administered as drops in two inocula of 250 *µ*l per nostril given approximately 15 minutes apart while the subjects are supine.

**Table 5**

| | Day | | | | | | | | |
|---|---|---|---|---|---|---|---|---|---|
| | 0 | 1 | 2 | 3 | 4 | 5 | 6 | 7-14 | 21 |
| | Pre-inoculation study timetable | | | | | | | | |
| Medications | | 6 doses | 6 doses | 6 doses | 6 doses | 6 doses | | | |
| Inoculation | | hour 4 | | | | | | | |
| Symptom scores | | m/e | m/e | m/e | m/e | m/e | m/e | e | |
| Nasal lavage | | m | m | m | m | m | m | | |
| Serum sample | | X | | | | | | | X |

| | Pre-inoculation study timetable | | | | | | | | |
|---|---|---|---|---|---|---|---|---|---|
| Medications | | 6 doses | 6 doses | 6 doses | 6 doses | 6 doses | | | |
| Inoculation | | hour 0 | | | | | | | |
| Symptom scores | | m/e | m/e | m/e | m/e | m/e | m/e | e | |
| Nasal lavage | | m | M | m | m | m | m | | |
| Serum sample | | X | | | | | | | X |

| | | | | | | | | | |
|---|---|---|---|---|---|---|---|---|---|
| Note In both studies on days 1-5, doses are given hours 0, 3, 6, 9, 12, and 15 m = morning e = evening | | | | | | | | | |

### B. Study Design

Two randomized rhinovirus challenge studies are performed (see Table 5). The same formulation of the immunoadhesin of the present invention is evaluated in pre-inoculation and post-inoculation studies. In both studies, medication is administered as six doses each day for five days. Subjects are randomly assigned to receive either the immunoadhesin or matching placebo at the time of enrollment into each study. The study is blinded and all clinical trial personnel, subjects, and employees of Panorama Research remain blinded until all data are collected.

In the pre-inoculation study, medications are started four hours (two doses) prior to viral challenge. The virus challenge is administered one hour after the second dose of the immunoadhesin (or placebo) and the four remaining doses of study medication for the first day are given as scheduled. In this study eighteen subjects receive the active treatment and eighteen subjects receive placebo.

In the post-inoculation study, medications begin 24 hours after virus challenge. This timepoint was chosen because it has been used in other studies of protection from virus challenge, and because cold symptoms are clearly present (Harris & Gwaltney, Clin. Infect. Dis. 23:1287-90, 1996). Virus challenge in this study is administered in the morning of study day 0 approximately 24 hours prior to the first dose of study medication on the morning of study day 1. In this study, 36 subjects receive the active treatment and 18 subjects receive placebo.

Subjects are isolated in individual hotel rooms from study day 0 (the day of virus challenge) to study day 6. On each of these days a symptom score and a nasal lavage for virus isolation are done in the morning prior to the first dose of medication and a second symptom score is done each evening. On study day 6, subjects are released from isolation but continue to record symptom scores each evening through day 14. The subjects return to the study site on study day 21, when a final serum sample for detection of anti-immunoadhesin antibodies will be collected. The total amount of immunoadhesin to be used in the two virus challenge studies (on a total of 54 subjects) is approximately 1200 mg.

### C. Viral Isolation

Virus shedding is detected by virus isolation in cell culture. Nasal wash specimens are collected by instillation of 5 ml of 0.9% saline into each nostril. This wash is then expelled into a plastic cup and kept chilled for one to two hours until it is processed for viral cultures. Immunoadhesin is removed from the specimens by treatment with anti-ICAM-1 antibody adsorbed to an agarose support (Affi-Gel 10, Bio-Rad Laboratories, Hercules, Calif.). A portion of each processed specimen is stored at -80° C., and another portion is inoculated into two tubes of HeLa-1 cells, a HeLa cell line enriched for the production of ICAM-1 Arruda, et al., J. Clin. Microb. 34:1277-1279, 1996). Rhinovirus are identified by the development of typical cytopathic effect. Subjects with a positive viral culture on any of the postchallenge study days are considered infected. Viral titers in the specimens stored at -80° C. are determined by culturing serial ten-fold dilutions in microtiter plates of HeLa-1 cells.

Antibody to the challenge virus are detected by serum neutralizing titers done using standard methods Gwaltney, et al, Diagnostic Procedures for Viral Rickettsial and Chlamydial Infections, p. 579-614, American Public Health Association). Serum specimens for antibody testing are collected during screening, immediately prior to virus challenge (acute), and again 21 days later (convalescent). Subjects with at least a four-fold rise in antibody titer to the challenge virus when the convalescent serum sample is compared with the acute serum sample are considered infected.

### D. Evaluation of Illness Severity

Illness severity is assessed as previously described (Turner, et al., JAMA 281:1797-804, 1999). Symptom scores are recorded prior to virus challenge (baseline) and twice each day at approximately twelve-hour intervals for the next 6 days. On study days 7 through 14 each subject records his/her symptom score once per day in the evening. At each evaluation, subjects are asked to judge the maximum severity of the following eight symptoms in the interval since the last symptom evaluation: sneezing, rhinorrhea, nasal obstruction, sore throat, cough, headache, malaise, and chilliness. Each symptom is assigned a severity score of 0 to 3 corresponding to a report of symptom severity of absent, mild, moderate, or severe. If symptoms are present at baseline, the baseline symptom score will be subtracted from the reported symptom score. The higher of the two daily evaluations are taken as the daily symptom score for each symptom. The daily symptom scores for the eight individual symptoms are summed to yield the total daily symptom score. The total daily symptom scores for the first 5 days after virus challenge (study days 1-5) are summed and on the evening of study day 5, all subjects are asked, "Do you feel you have had a cold?" Subjects who had a total symptom score of at least 6 and either at least three days of rhinorrhea or the subjective impression that they had a cold are defined as having a clinical cold.

The weight of expelled nasal secretions is determined on days 1-7 by providing all subjects with packets of preweighed nasal tissues. After the tissues are used they are stored in an airtight plastic bag. Each morning the used tissues, together with any unused tissues from the original packet, are collected and weighed.

### E. IL-8 Assay

Recent studies have suggested that the host inflammatory response, particularly interleukin 8 (IL-8), may play a role in the pathogenesis of common cold symptoms due to rhinovirus infection. Concentrations of IL-8 in nasal lavage are determined with a commercially available ELISA (R&D Systems, Minneapolis, Minn.) as previously described (Turner, et al., JAMA 281:1797-804,1999).

### F. Safety Evaluations

The same evaluations are, done in the challenge study as in the dose escalation study described in Example 8.

### G. Statistical Analysis

Statistical analysis is performed similarly as to that described for the dose escalation study described in Example 8.

The foregoing examples and discussion, while predominantly addressed to ICAM-1 immunoadhesins, can be readily adapted by one of skill to achieve and implement the use of other types of immunoadhesins active against other types or subtypes of virus and bacterial pathogens. The following examples illustrate antibacterial immunoadhesin embodiments making use of the anthrax toxin receptor (ATR) as receptor protein.

### Example 10: Construction of ATR Immunoadhesin Expression Cassettes (not of the invention)

A cassette encoding a portion of the extracellular domains of human anthrax toxin receptor (ATR) is prepared by PCR cloning. Specifically, a fragment of 523 bp, encoding amino acids 44-216 (the so-called von Willebrand factor type A domain) is amplified from plasmid ATR (Bradley et al., 2001), or from plasmid TEM8 (St Croix et al., 2000) using the following oligonucleotide primers:
5'-GACCTGTACTTCATTTTGGACAAATCAGG-3' (SEQ ID NO: 91)

5'-GAGCTCAAAATTGAGTGGATGATGCCT (SEQ ID NO: 92)
TGCAGAG -3'

The second primer (SEQ ID NO: 92) is designed to introduce a Sac I site at the 3' end of the coding region of the ATR extracellular domain (solid underline). PCR is performed with Pfu polymerase (Stratagene) to reduce accumulation of errors. A second fragment of 124 bp, which includes a 5' untranslated region and a plant signal peptide, is amplified from plasmid 8ATG-TOPO#4 (which is a PCR clone of a plant-optimized 5' untranslated region and signal peptide in the Invitrogen cloning vector pCR4-TOPO), using the following oligonucleotide primers:
5'-GGTACCACTTCTCTCAATCCAACTTTC-3' (SEQ ID NO: 93)

5'-GTCCAAAATGAAGTACAGGTCAGCCAA (SEQ ID NO: 94)
ACTAGTAGAGGTGAACAAAAGC-3'

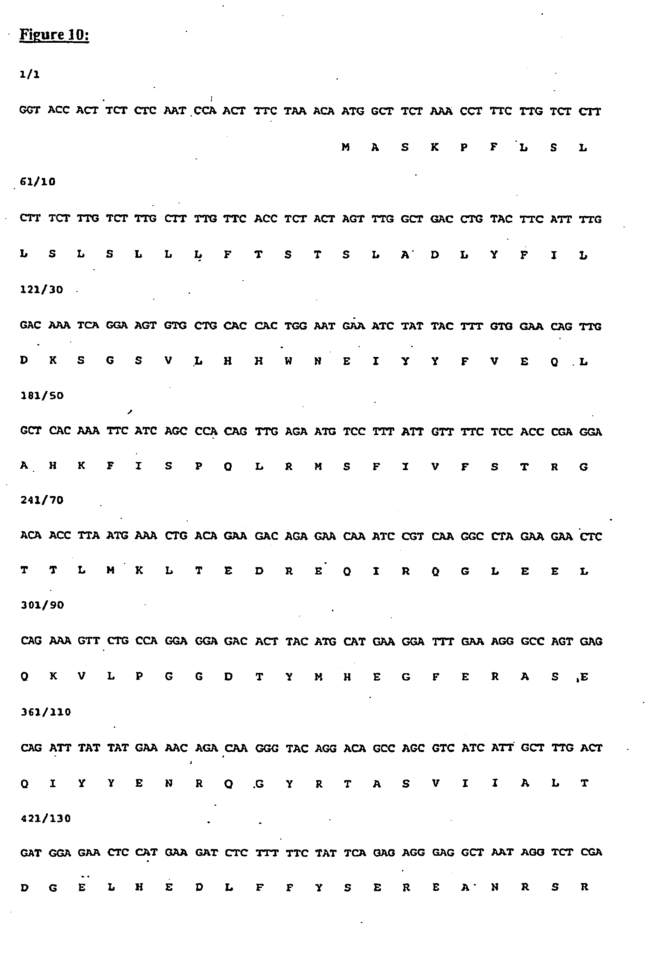

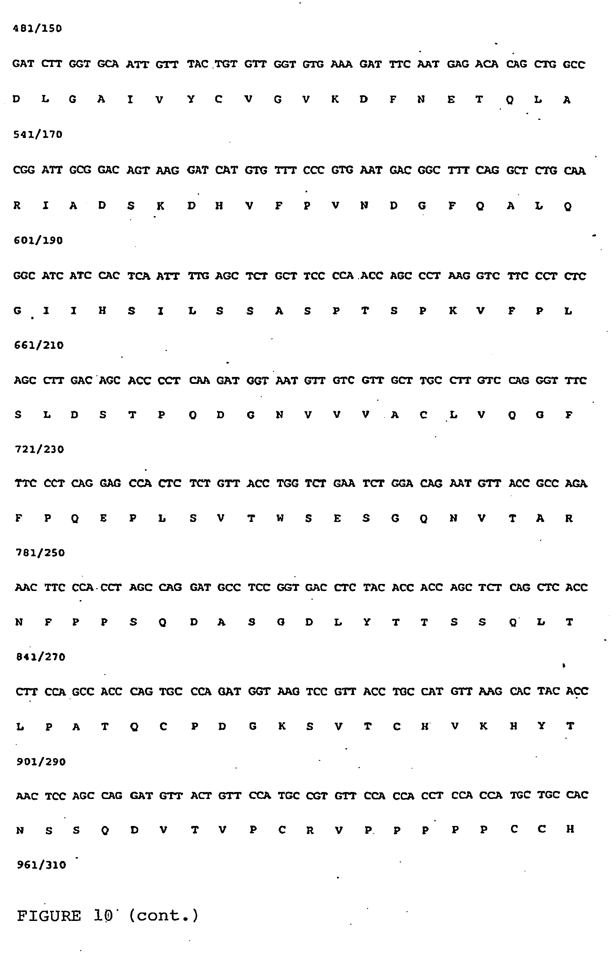

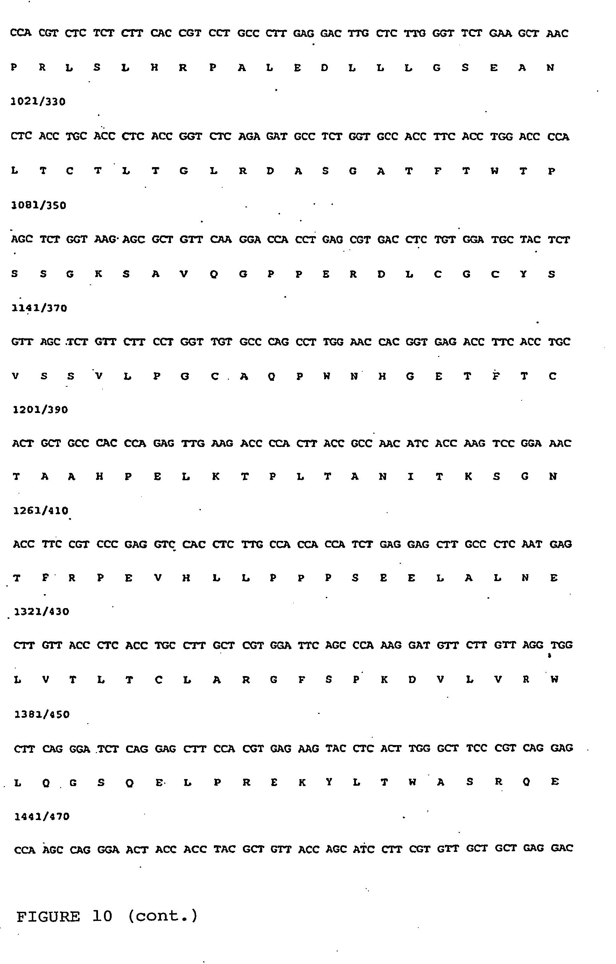

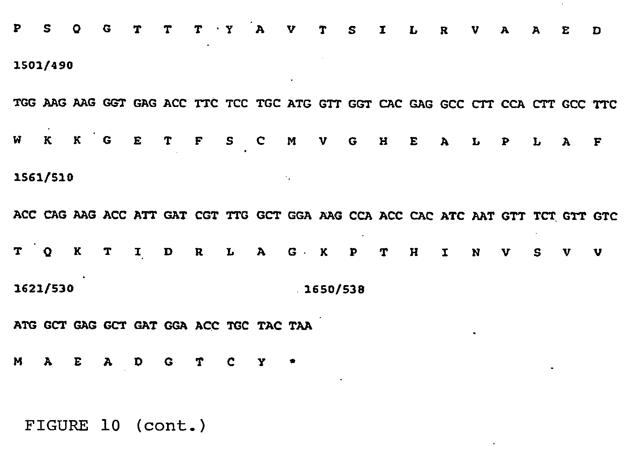

The first primer (SEQ ID NO: 93) is designed to introduce a Kpn I site at the 5' end of the PCR fragment (solid underline). The two PCR fragments have 20 nt of complementary sequence (dotted underlines). The two PCR fragments are mixed together, and a fragment of 626 bp is amplified using SEQ ID NO: 93 and SEQ ID NO: 92. The resulting PCR fragment is cloned into the vector PCRScript (Stratagene), and sequenced before cloning between Kpn I and Sac I sites in the vector pMSP-coICAM, resulting in plasmid pMSP-ATR-IgA2. This results in a genetic fusion of the extracellular domain of ATR and the constant region of human IgA2. This human IgA2 constant region has been synthesized to use codons optimal for expression in tobacco cells. The full nucleotide and amino acid sequence of the ATR-IgA2 fusion (the immunoadhesin) is shown in **FIG. 10****.** In the resulting construct, expression of the chimeric ATR-IgA2 molecule is under the control of the constitutive promoter "superMAS" (Ni et al., 1995) and the ags 3' terminator region.

The entire expression cassette (promoter+ATR-IgA2+terminator) is removed from pMSP-ATR-IgA2 with the restriction enzyme Asc I, and cloned into the binary *Agrobacterium* Ti plasmid vector pGPTV-kan-ocs, resulting in plasmid pGPTV-kan-ocs-ATR-IgA2. The vector pGPTV-kan-ocs is derived from pGPTV-kan (Becker et al., 1992), which was modified in the following manner. The sequence between the Eco RI and Hind III sites of pGPTV-kan, including the entire uid A gene, was removed and replaced with the ocs 3' terminator region (MacDonald et al., 1991) oriented toward the npt II gene, plus the restriction sites for Asc I and Sac I. The purpose of this terminator adjacent to the right border of the T-DNA is to eliminate transcriptional interference with the transgene due to transcription originating in the plant DNA outside of the right border (Ingelbrecht et al., 1991).

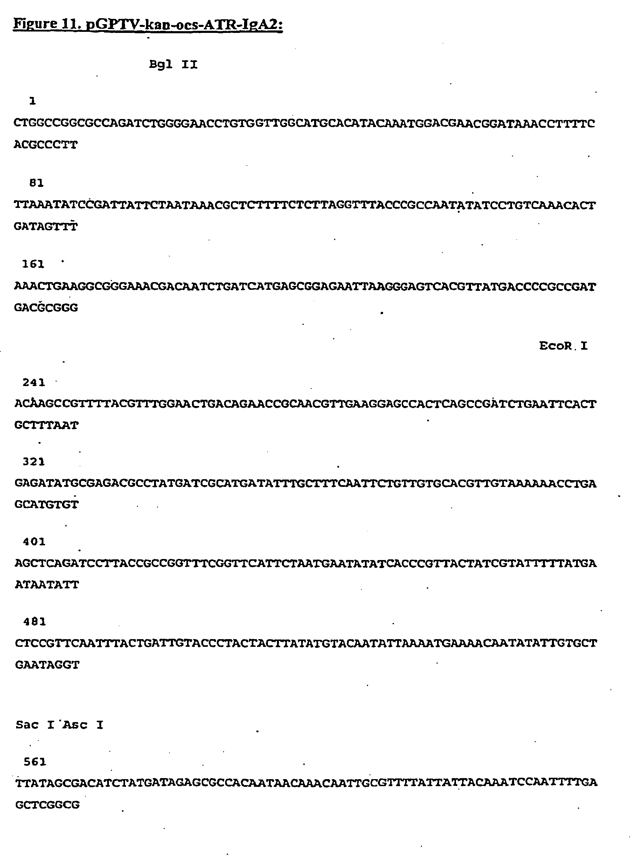

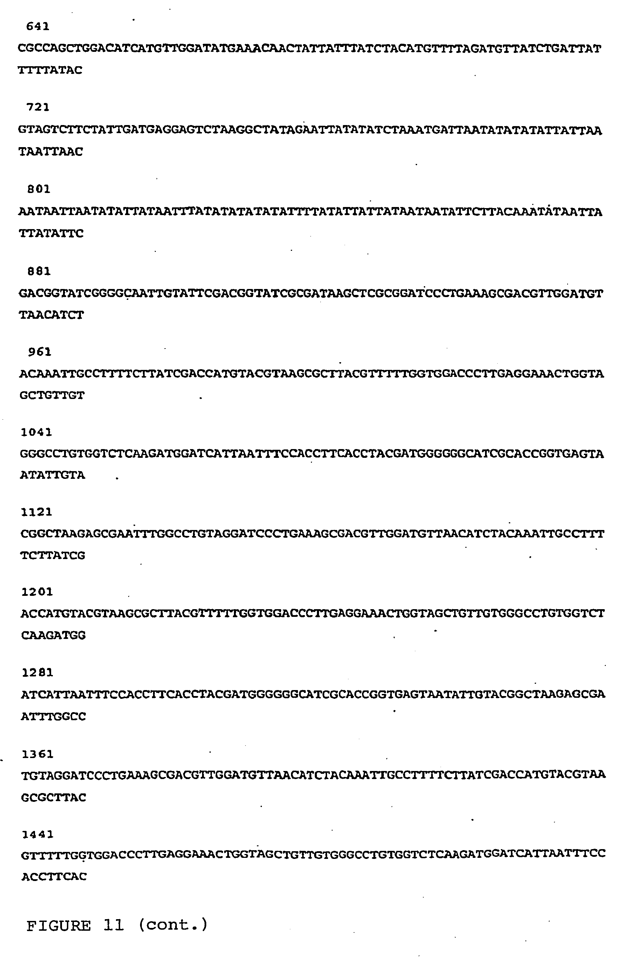

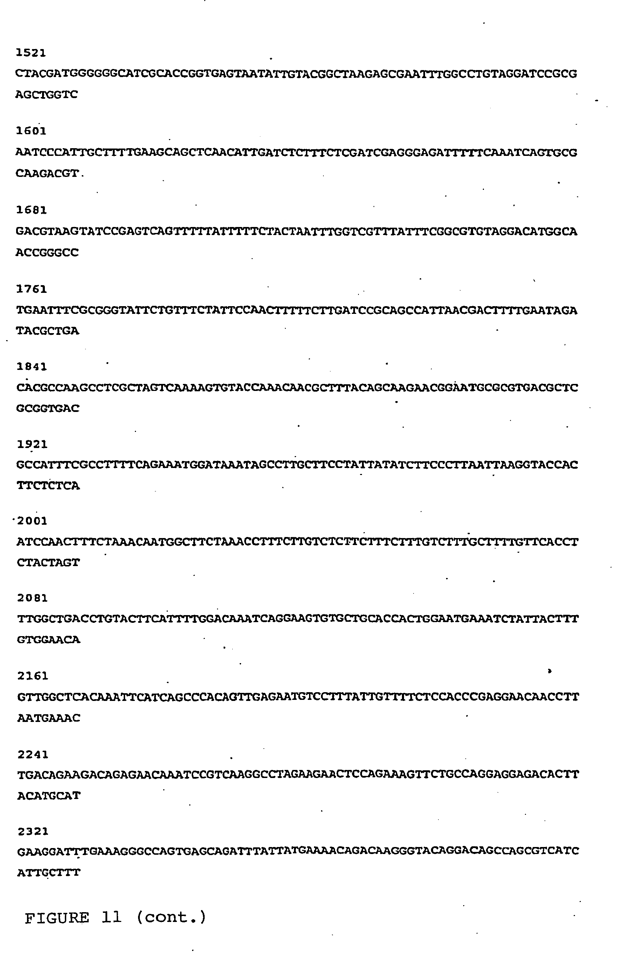

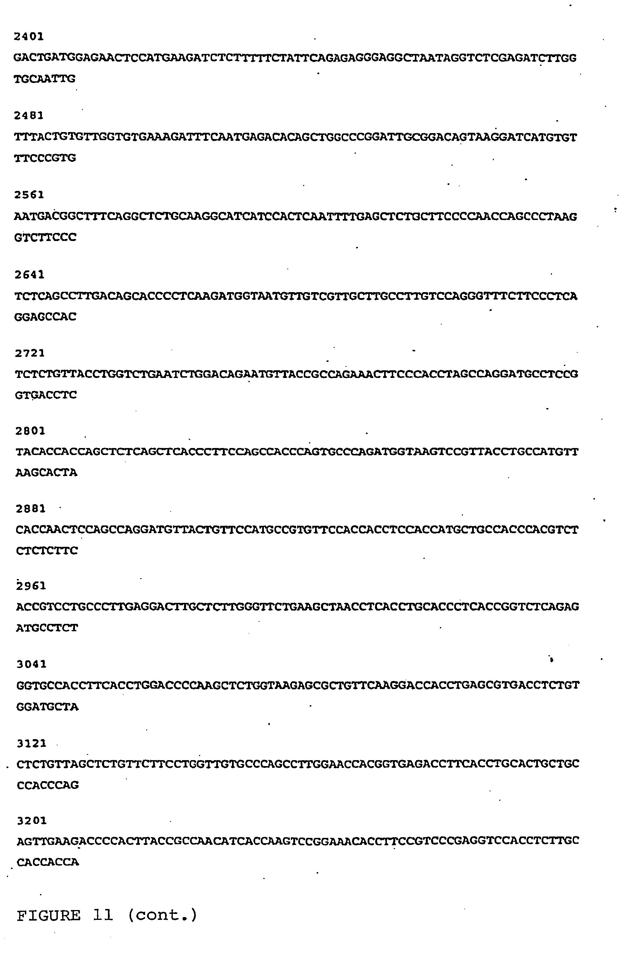

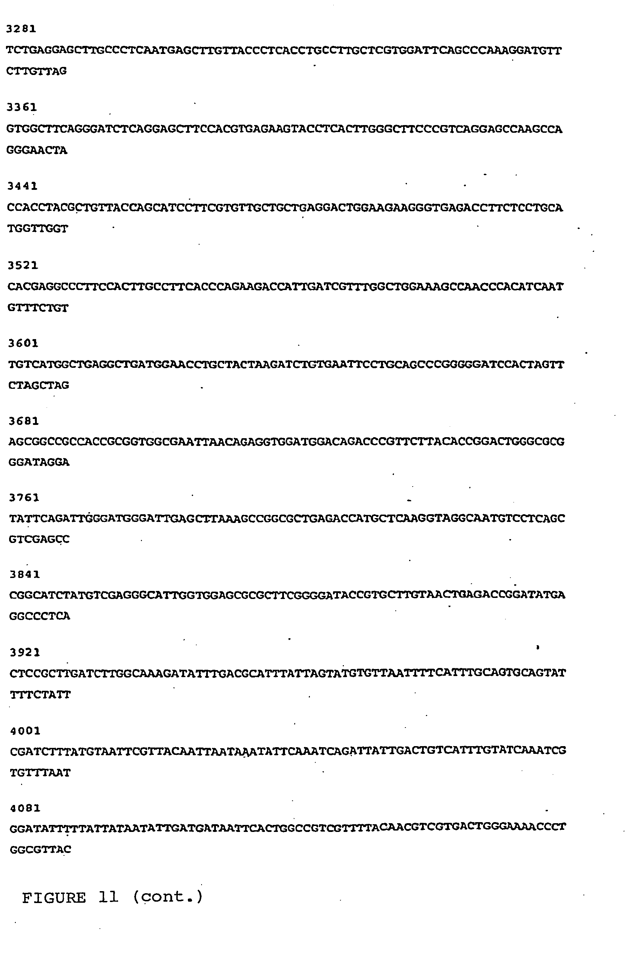

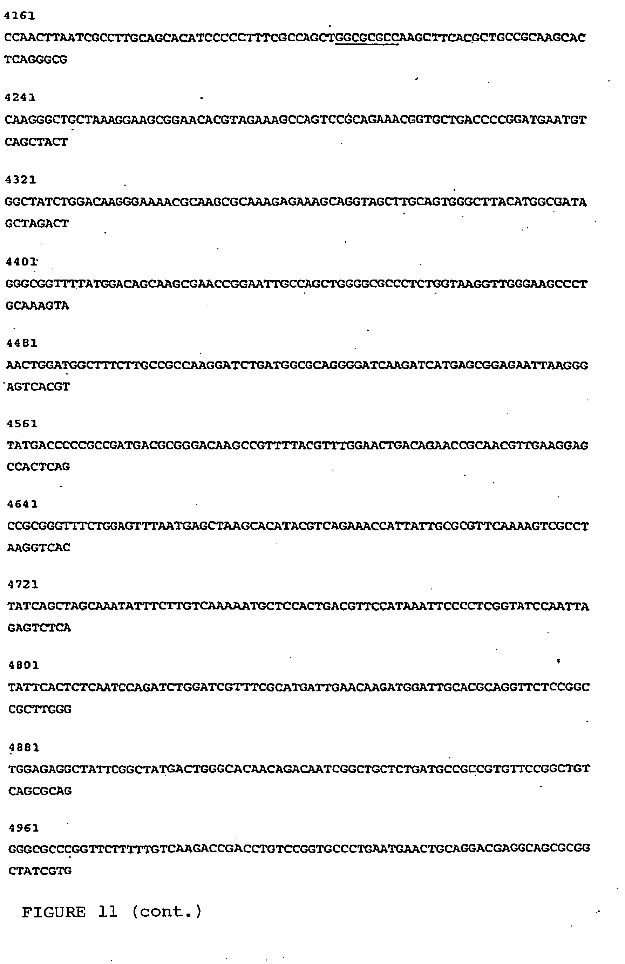

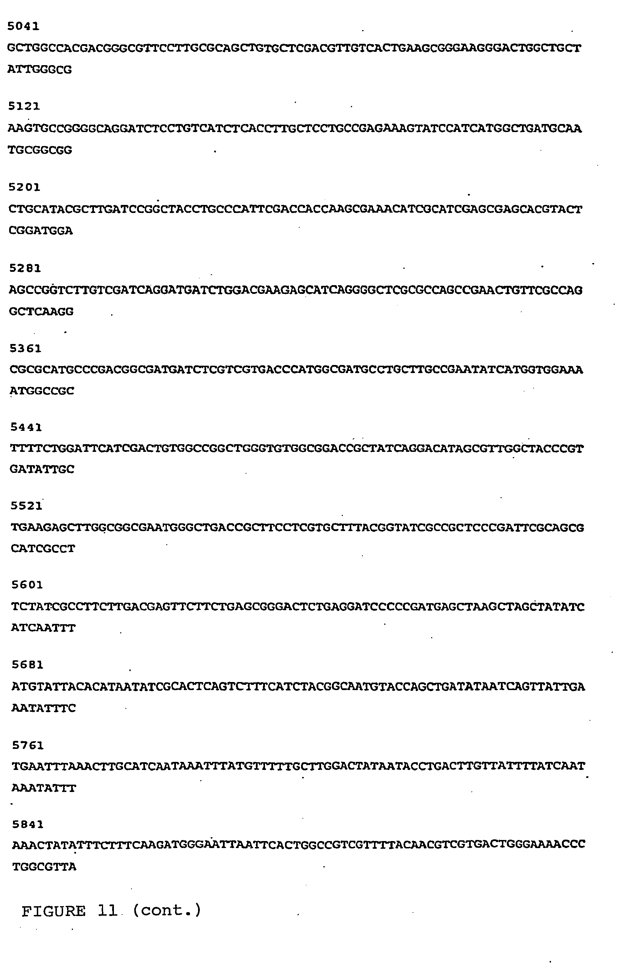

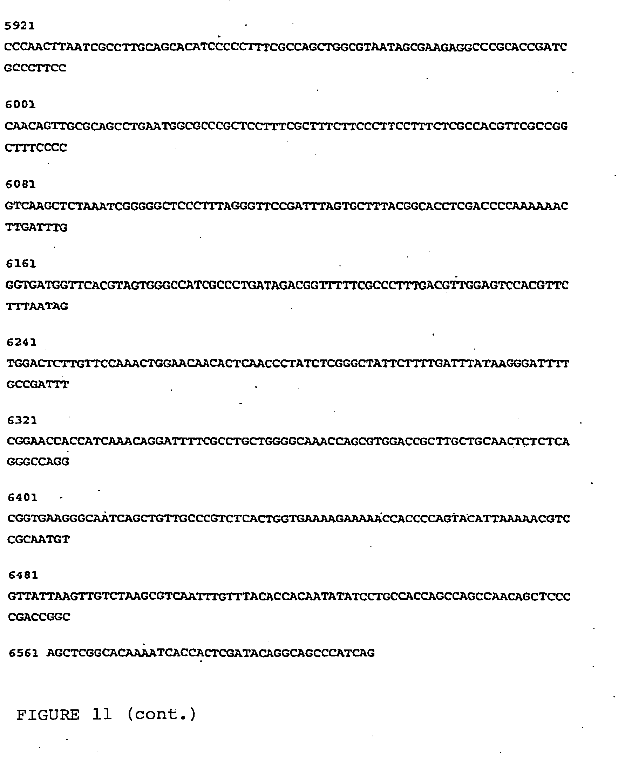

Sequence between the T-DNA borders of the plasmid pGPTV-kan-ocs-ATR-IgA2 is shown in **FIG.11****.** Sequence outside the left and right borders are as described (Becker et al., 1992). Nucleotides 18-187 represent the right T-DNA border. Nucleotides 311-630 represent the ocs 3' terminator region. Nucleotides 927-1976 represent the superMAS promoter. Nucleotides 1990-2017 represent a 5' untranslated region from the *Nicotiana sylvestris* psaDb gene (Yamamoto et al., 1995). The context around the initiation ATG (nucleotides 2012-2026) was designed to match that found in highly expressed plant genes (Sawant et al., 1999). Nucleotides 2018-2086 comprise a sequence encoding a modified version of the signal peptide of *Vicia faba* legumin (Baumlein et al., 1986). Nucleotides 2087-2605 comprise a sequence encoding the von Willebrand factor type A domain of ATR (Bradley et al., 2001). Nucleotides 2606-3631 comprise a sequence encoding the human IgA2m(2) constant region (Chintalacharuvu et al., 1994). Nucleotides 3794-4108 derive from the agropine synthase (ags) terminator. Nucleotides 4530-4800 represent the NOS promoter (Depicker et al., 1982). Nucleotides 4835-5626 encode the npt II gene (conferring resistance to kanamycin). Nucleotides 5648-5870 are the polyadenylation signal from *A. tumefaciens* gene 7 (Gielen et al., 1984). Nucleotides 6454-6602 represent the left T-DNA border.

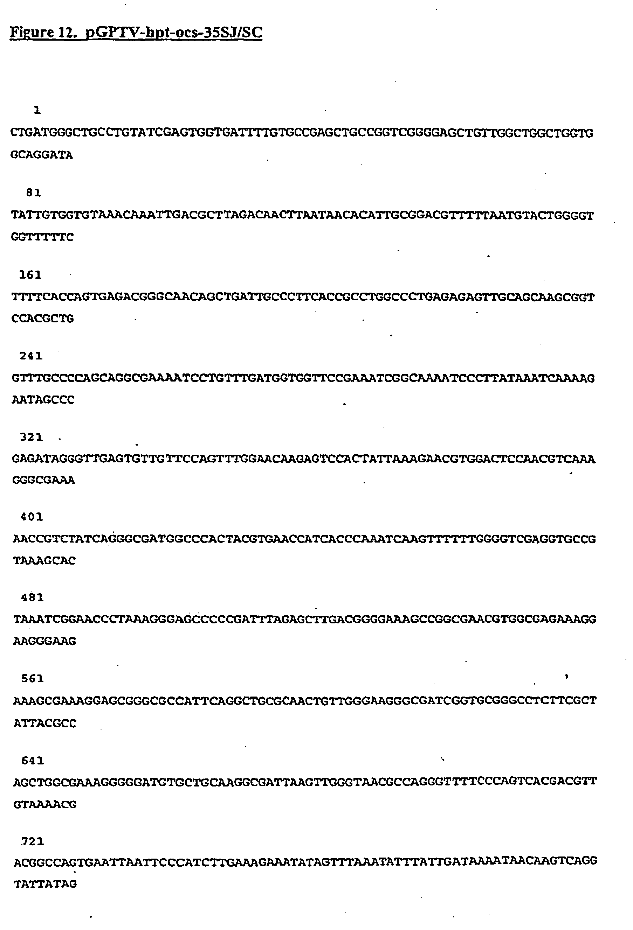

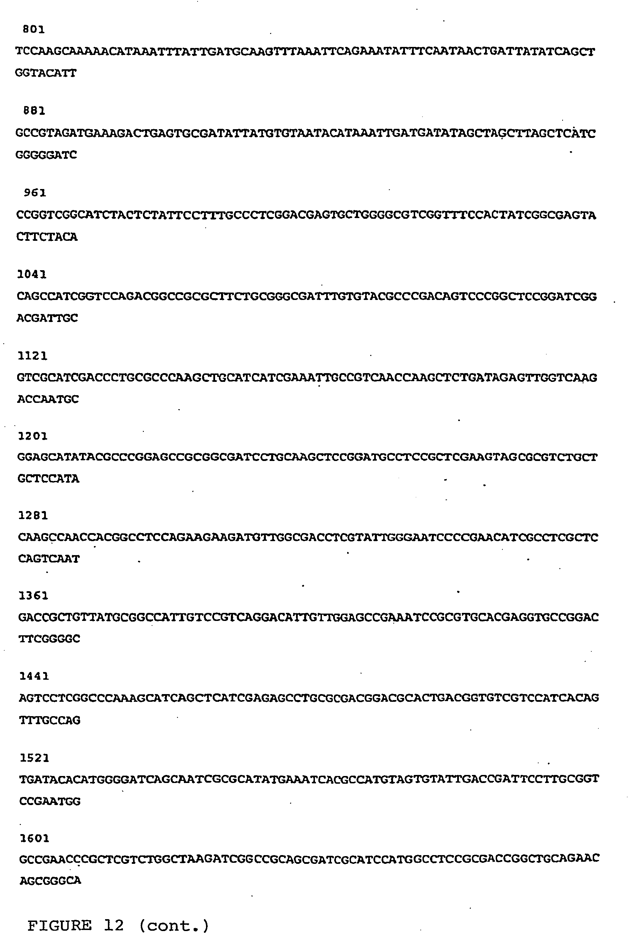

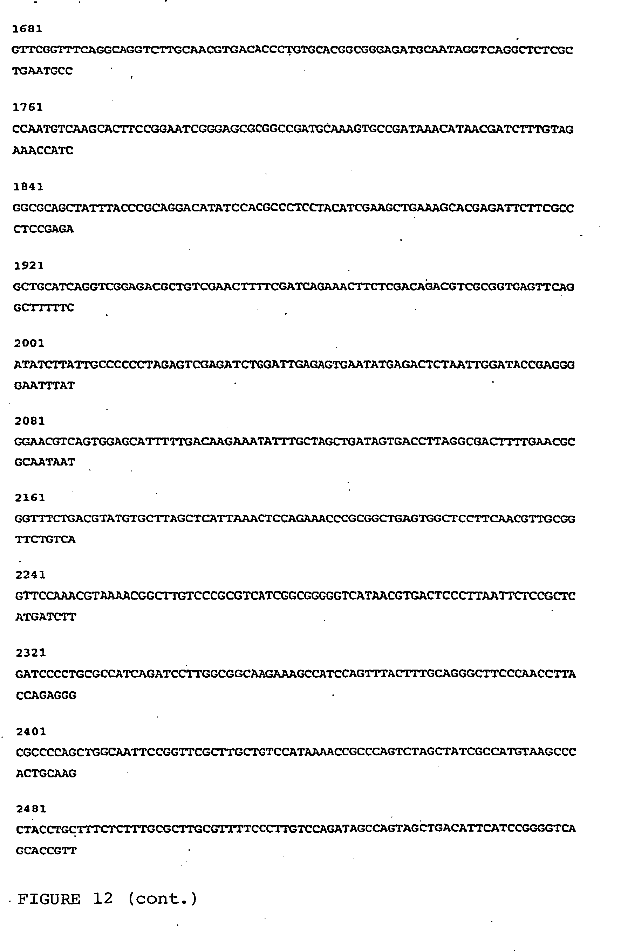

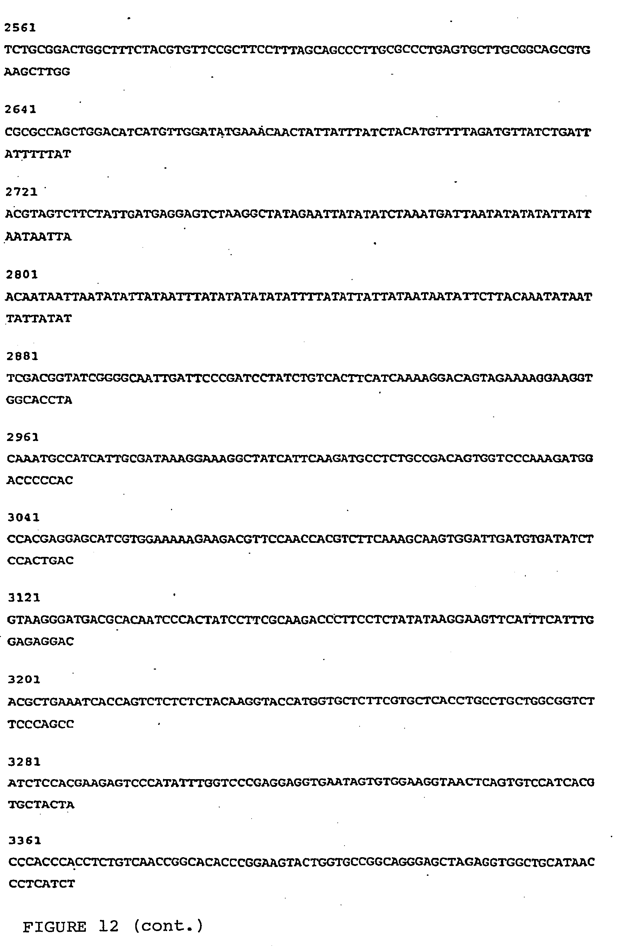

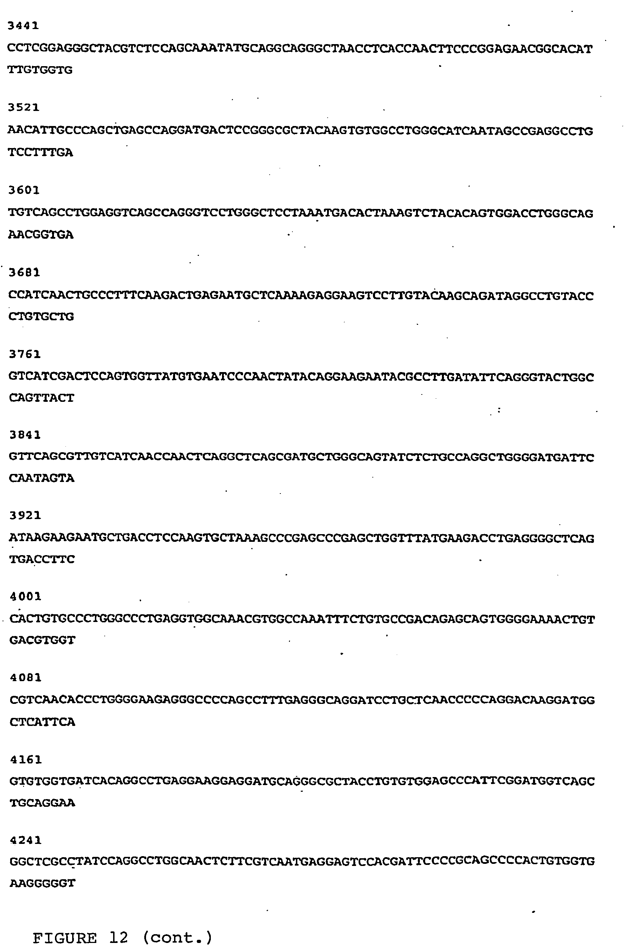

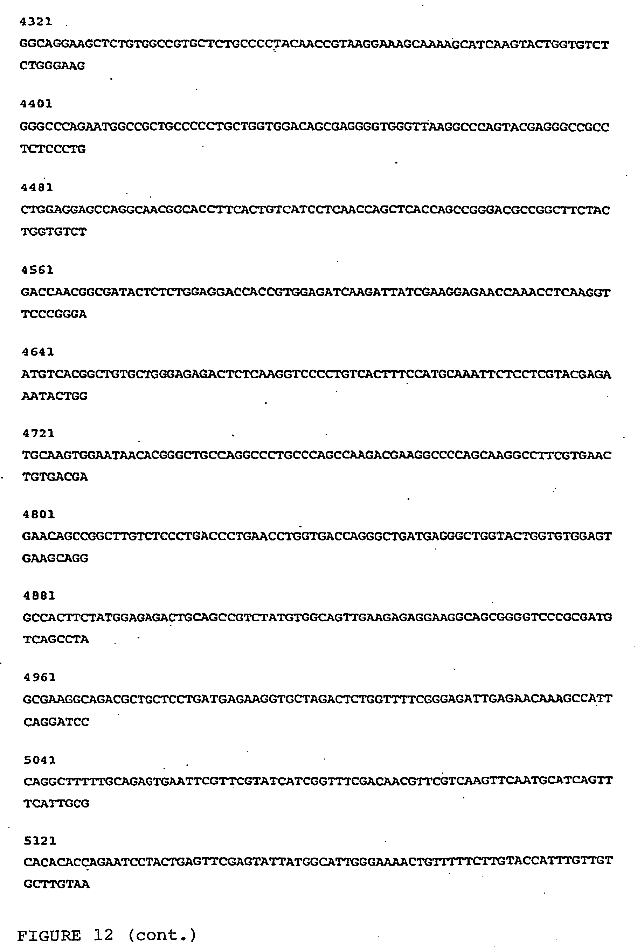

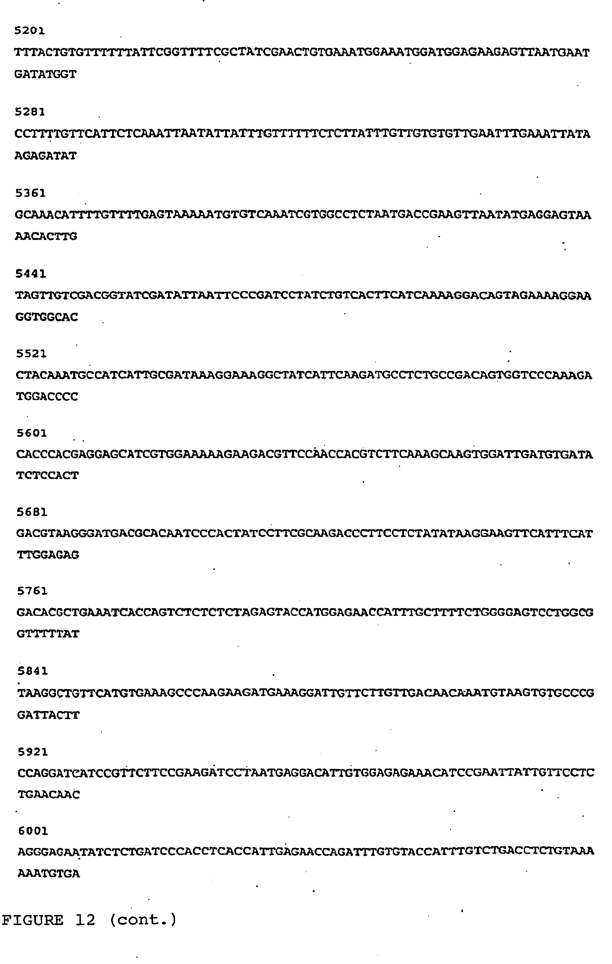

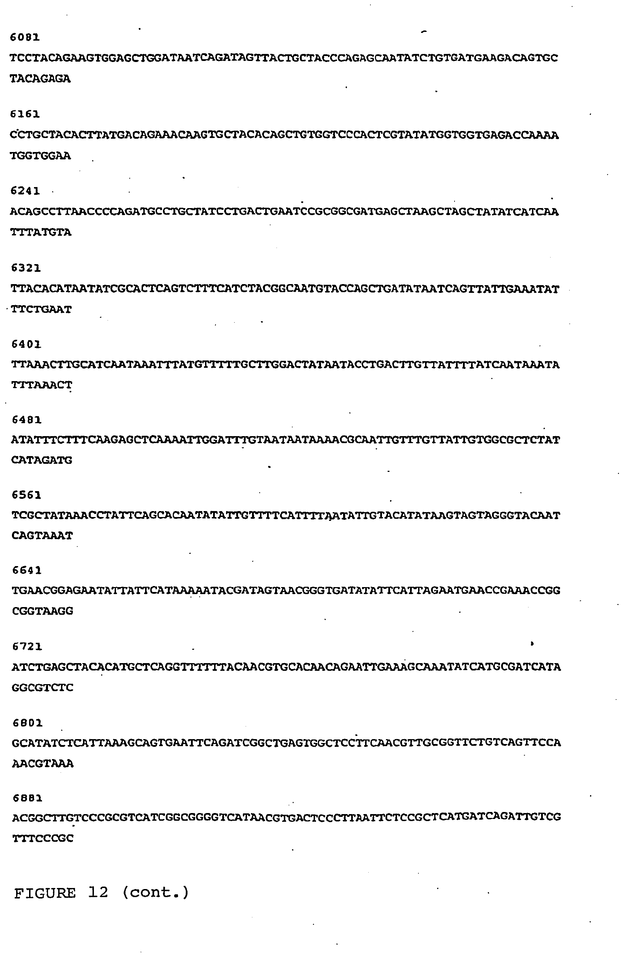

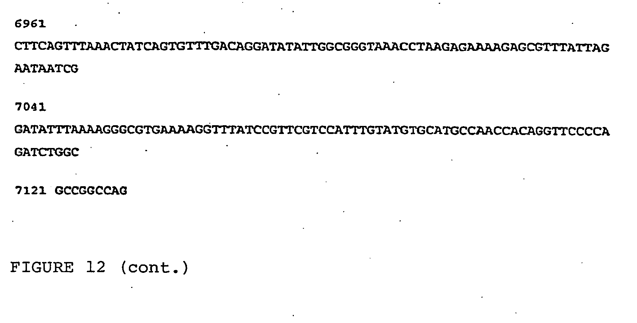

A construct for the expression in plants of human J chain and secretory component has also been developed. This construct, pGPTV-hpt-ocs-35SJ/SC, is based on the vector pGPTV-hpt-ocs, derived from pGPTV-hpt in the same manner as described for pGPTV-kan-ocs above. Sequence between the T-DNA borders of the plasmid pGPTV-hpt-ocs-35SJ/SC is shown in **FIG. 12****.** Sequence outside the left and right borders are as described (Becker et al., 1992). Nucleotides 1-149 represent the left T-DNA border. Nucleotides 733-955 (complement) represent the polyadenylation signal from *A. tumefaciens* gene 7 (Gielen et al., 1984). Nucleotides 980-2002 (complement) represent the hpt gene (conferring resistance to hygromycin). , Nucleotides 2049-2318 (complement) represent the NOS promoter (Depicker et al., 1982). Nucleotides 2898-3230 represent the cauliflower mosaic virus (CaMV) 35S promoter driving expression of the human secretory component gene including its native signal peptide (nucleotides 3236-5056), and nucleotides 5060-5445 represent the polyadenylation signal from the pea rbcS-E9 gene (Mogen et al., 1992). Nucleotides 5457-5788 represent a second copy of the CaMV 35S promoter driving expression of the human Joining (J) chain gene including its native signal peptide (nucleotides 5797-6273), and nucleotides 6281-6494 represent the gene 7 terminator. Nucleotides 6501-6819 (complement) represent the ocs 3' terminator region. Nucleotides 6944-7113 represent the right T-DNA border.

### Example 11: Plant Transformation and ATR Immunoadhesin Expression in Plants (not of the invention)

The expression cassettes described above are used to produce the assembled immunoadhesin in plants, via *Agrobacterium*-mediated transformation. Plasmids pGPTV-hpt-ocs-35SJ/SC and pGPTV-kan-ocs-ATR-IgA2 are introduced separately into A tumefaciens strain LBA4404. Overnight cultures of both strains are used for simultaneous "co-cultivation" with leaf pieces of tobacco, according to a standard protocol (Horsch et al., 1985). Transformed plant tissue is selected on regeneration medium containing both kanamycin (100 µg/mL) and hygromycin (25 µg/mL).

Plantlets that regenerate in the presence of antibiotic are screened for transgene expression. This is accomplished by preparing extracts of leaf tissue in phosphate buffered saline (PBS) and spotting clarified extracts on nitrocellulose paper. These "dot" blots are probed with alkaline-phosphatase-conjugated antisera specific for human IgA, J chain or secretory component. Plants that test positive on this first screen are subjected for further screens involving western blotting and PCR. The ATR-IgA2 immunoadhesin is expected to have a subunit MW of 59 kDa. Due to natural dimerization of the heavy chain constant region, dimers of ∼118 kDa are also expected to form. These dimers further dimerize within the plant cell in the presence of J chain, forming a molecule of ∼252 kDa. With the addition of secretory component, a molecular species of ∼320 kDa is observed.

The presence of a signal peptide in the chimeric heavy chain, J chain and secretory component constructs is important for assembly into a multimeric immunoadhesin. Upon translation of the mRNAs, signal peptide cleavage is predicted to deposit the each protein into the plant cell's endoplasmic reticulum (ER). Assembly into a multimeric immunoadhesin is expected to take place in the ER and golgi bodies, and the assembled molecule is then secreted from the cell.

### Example 12: Purification of Assembled ATR Immunoadhesin (not of the invention)

Purification of Assembled ATR Immunoadhesin can be accomplished essentially as described for the ICAM-1 immunoadhesin of Example 3, supra.

### Example 13: The ATR Immunoadhesin Inhibits Toxin Action on Mammalian Cells (not of the invention)

The expression cassettes described above are used to produce the assembled immunoadhesin, which is purified from plant extracts. The purified immunoadhesin is used to protect CHO-K1 cells from being killed in a simple bioassay. CHO-K1 cells have the receptor to which PA binds on their cell surfaces, but they are not sensitive to the toxin. They are killed when challenged with PA and LFN-DTA, a fusion protein composed of the N-terminal 255 amino acids of LF linked to the catalytic A chain of diptheria toxin. This recombinant toxin exploits the same LF-PA-receptor interactions that are required for the binding and entry of the native LF and OF proteins. To test the protective effect of the immunoadhesin, CHO-K1 cells are mixed with an increasing amount of ATR-IgA2 in the presence of a constant (toxic) amount of PA and LF_{N}-DTA, and the subsequent effect on protein synthesis is measured. ATR-IgA2 is an effective inhibitor of toxin action, inhibiting toxin action at a lower molar concentration than soluble ATR.

### Example 14: The ATR Immunoadhesin Inhibits Toxin Action in Human Subjects (not of the invention)

The purified immunoadhesin is prepared in a pharmaceutically acceptable buffer and is administered to human subjects infected with Anthrax. The route of administration may be either as an inhaled aerosol or as an injection. Subjects in late stages of infection who would normally die are protected from toxin action by the immunoadhesin.

### Example 15: Construction of an Alternative ATR Immunoadhesin Expression Cassette (not of the invention)

A cassette encoding the entire extracellular portion of human ATR (amino acids 24-320) is prepared by PCR cloning. Specifically, a fragment of 878 bp is amplified from plasmid ATR (Bradley et al., 2001), or from plasmid TEM8 (St Croix et al., 2000) using the following oligonucleotide primers:

5′-GGGGGACGCAGGGAGGATGGGGGTC (SEQ ID NO: 95)
CAG -3′

5′-GAGCTCCCGTCAGAACAGTGTGTGG (SEQ ID NO: 96)
TGGTG -3′

The second primer (SEQ ID NO: 96) is designed to introduce a Sac I site at the 3' end of the coding region of the ATR extracellular domain (solid underline). PCR is performed with Pfu polymerase (Stratagene) to reduce accumulation of errors. A second fragment of 121 bp, which includes a 5' untranslated region and a plant signal peptide, is amplified from plasmid .delta. ATG-TOPO#4, using the following oligonucleotide primers:
5'-GGTACCACTTCTCTCAATCCAACTTTC-3' (SEQ ID NO: 93)

5′-ATCCTCCCTGCGTCCCCCAGCCAAACTAGTA (SEQ ID NO: 97)
GAGGTGAACAAAAGC -3′

The first primer (SEQ ID NO: 93) is designed to introduce a Kpn I site at the 5' end of the PCR fragment (solid underline). The two PCR fragments have 20 nt of complementary sequence (dotted underlines). The two PCR fragments are mixed together, and a fragment of 981 bp is amplified using SEQ ID NO: 93 and SEQ ID NO: 96. The resulting PCR fragment is cloned into a plant expression cassette to form a genetic fusion with human IgA2 in the same manner as the partial ATR extracellular domain (Example 1).

An alternate construction using this same method would amplify amino acids 41-227.

### Example 16: Production of an Anthrax Chimeric Toxin Receptor Protein: CMG2-IgG and high affinity anti-PA antibody

### Construction of CMG2 fused to IgG (CMG2-IgG) and high affinity anti-PA antibody

Briefly, DNA fragments encoding codon-optimized anti-PA heavy and light chain Variable regions, and a fragment encoding a portion of the extracellular domain of a human anthrax toxin receptor (CMG2) were synthesized. The CMG2 and anti-PA heavy-chain Variable region sequences were separately ligated to DNA fragments encoding human IgG1 heavy constant region domains in an *Agrobacterium* binary vector under the control of a constitutive plant promoter (CaMV35S) and a plant 3' polyadenylation signal sequence. A similar kappa chain vector was prepared for the 1H light-chain V region. Construction of these vectors is described in detail below.

The program "Signal IP" was used to predict the signal peptide cleavage site of CMG2 as being between amino acids 33 and 34⁴⁵. Using a codon-usage table derived from highly-expressed plant genes, we designed and synthesized a DNA fragment of 697 bp that included a short 5' untranslated region, the signal peptide of the 2S2 albumin storage protein of Arabidopsis thaliana⁴⁶ and amino acids 34-232 of CMG2 **(****Figure 14****).** The encoded portion of CMG2 corresponded to that used by Scobie *et al.* as a soluble protein.³¹ After confirming the sequence, this fragment was cloned between Kpn I and Sac I sites in the vector pMSP-IgG, which already contains the constant region of human IgG1, resulting in plasmid pMSP-CMG2-IgG. This resulted in a genetic fusion of the extracellular domain of CMG2 and the constant region of human IgG1. This human IgG1 constant region had also been synthesized to use codons optimal for expression in tobacco. In the resulting construct, expression of the chimeric CMG2-IgG1 molecule was under the control of the highly expressed constitutive promoter CaMV35S47 and the *ags* 3' terminator region. The expected amino acid sequence of the mature protein is shown in Figure 14.

The entire expression cassette (promoter + CMG2-IgGl + terminator) was subcloned from pMSP-CMG2-IgG into the binary *Agrobacterium* Ti plasmid vector pGPTV-kan-ocs, resulting in plasmid pGPTV-kan-ocs-CMG2-IgG. The vector pGPTV-kan-ocs is derived from pGPTV-kan, which was itself derived from the more common binary vector pBIN19.⁴⁸ The expression cassette uses a 5' untranslated region from the *Nicotiana sylvestris psa*Db gene, which has characteristics of a translational enhancer.⁴⁹ The context around the initiation ATG was designed to match that found in highly expressed plant genes.⁵⁰

For expression of the anti-PA antibody, a DNA fragment encoding the heavy chain variable region of 1H, using plant-optimal codons, was synthesized with a *Sac* I site at the 3' end for ligation to our codon-optimized human IgG1 constant region sequence. Similarly, a sequence encoding the Vk region of antibody 1H, with a Hind III at the 3' end for ligation to our codon-optimized human kappa constant region, was also synthesized. These sequences incorporated the same 5' UTR and signal peptide as was used with the CMG2 gene. Both variable regions were cloned into separate expression vectors under the control of the CaMV35S promoter, in frame with the corresponding heavy and light chain constant regions.

### Protein production in tobacco using an Agrobacterium tumefaciens-mediated transient expression system

Two *Agrobacterium* strains carrying cassettes for expression of 1H heavy and light chains were co-infiltrated into leaves of *Nicotiana benthamiana,* which were harvested 7 days later and immediately processed for purification of antibody.⁵¹ Similarly, an *Agrobacterium* strain carrying the CMG2-IgG expression cassette was infiltrated into leaves. Leaf extracts from transiently transfected tobacco plants were prepared and fractionated by Protein A chromatography. Using this rapid, high capacity and reproducible approach we purified small samples of 1H and CMG2-IgG. **Table 6** shows the results of a typical experiment.

**Table 6. Isolation and purification of protein from transiently transfected N. benthamiana leaves**

| Protein | grams leaves | µg/gm (ELISA) | µg recovered | µg/gm yield (OD280) | nmoles |
|---|---|---|---|---|---|
| 1H IgG | 100 | 18 | 1740 | 17.4 | 11.6 |
| CMG2-IgG | 74 | No data | 1140 | 15.4 | 9.8 |

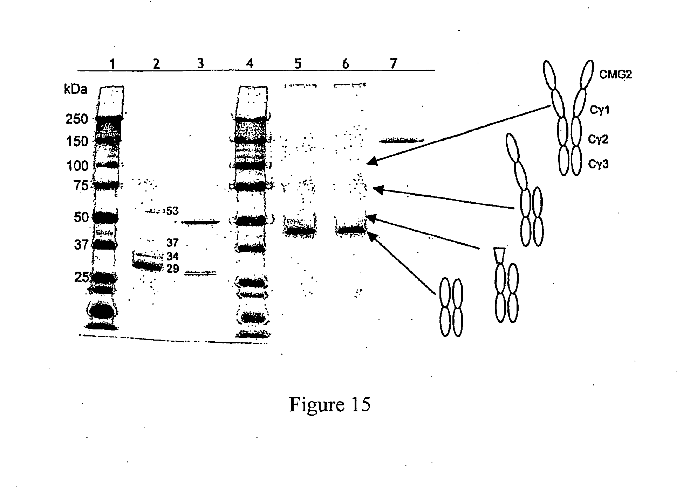

The purified proteins were separated by SDS-PAGE under reducing and non-reducing conditions and subjected to silver staining **(****Figure 15****).** The 1H IgG preparation contained a single band of 150 kDa, which reduced to two bands at 50 and 26 kDa. Western blotting confirmed these two bands as human gamma heavy and kappa light chain (not shown). The CMG2-IgG preparation that eluted from protein A was a heterogeneous mixture of proteins, with the major band at 45 kDa under non-reducing conditions and 29 kDa under reducing conditions. The expected size, based on amino acid sequence, of a CMG2-IgG monomer is 58 kDa. Under reducing conditions, there is a small amount of a 53 kDa band, which probably represents intact CMG2-IgG monomer. More rapidly migrating bands are proteolytic degradation products that are missing the VWA/I domain plus all or part of Cγ1. All bands reacted with anti-IgG1 antiserum (data not shown).

The CMG2-IgG preparation was again run under reducing conditions on SDS-PAGE, and the gel was blotted onto PVDF and stained with Coomassie. Three slices, containing the 53, 34 and 29 kDa fragments **(****Figure 15****)** were sent to the Molecular Structure Facility at UC Davis for Edman degradation (N-terminal amino acid sequencing). No sequence could be recovered from the 53 kDa band. This suggested the presence of a "blocked" residue, which is consistent with the Gln expected for the amino terminus of CMG2. The amino termini of the 34 and 29 kDa fragments were at amino acids 268 and 297 respectively (bold amino acids numbered **1** and **2** in **Figure 14****).** These results are consistent with the cartoon models in Figure 15.

### Example 17: Testing for in vitro anti-anthrax toxin activity of Anthrax Chimeric Toxin Receptor Protein

This Example demonstrates methods to test the anti-anthrax toxin activity of the chimeric toxin receptor proteins and immunoadhesins described herein.

The purified 1H IgG plantibody and the CMG2-IgG1 antitoxin were tested for the ability to neutralize PA in a mouse macrophage assay employing lethal toxin. Survival of RAW 264.7 mouse macrophage-like cells after administration of PA plus lethal factor (100 ng/ml PA, 50 ng/ml LF) together with different concentrations of plant-made 1H IgG or CMG2-IgG1 was determined.²⁵ Antitoxin proteins were pre-incubated with toxin for 30 minutes before adding to macrophages. After three hours of exposure to toxin, an indirect viability assay was performed. Titration of plant-made 1H IgG determined that the antibody had an IC₅₀ of ∼0.3 µg/ml, very similar to that of the same antibody made in animal cells (not shown). The CMG2-IgG preparation also protected macrophages in the concentration range 1 - 10 µg/ml. This suggested that CMG2-IgG was a potent anti-toxin.

### Example 18: Anthrax Toxin Receptor Protein Immunoadhesin CMG2-IgG Variants

This Example demonstrates representative modifications that were made to improve stability and yield of the chimeric toxin receptor proteins and immunoadhesins described herein.

### Production and Expression of Variants

Using PCR, modifications were introduced around the Sac I site at the junction between the CMG2 and IgG coding regions of the CMG2-IgG construct. Fourteen amino acids that were not part of the VWA/I domain as defined by crystal structure³⁴ were removed in variant 1 **(V1** in **Figure 14****).** The removed amino acid sequence and the corresponding nucleotide sequence is provided below:
TEILELQPSSVCVG (SEQ ID NO: 102)
act gaa atc cta gaa ttg cag ccc tca agt gtc tgt gtg ggg (SEQ ID NO: 103)

For variant 2a **(V2a in** **Figure 14****)** the Cγ1 domain was removed, since the analysis above suggested that there were protease-sensitive sites in this region. In variant 2b both the 14 amino acids and the Cγ1 domain were removed. In variant 3, the Cγ1 remains, but the 14 amino acids have been replaced by a flexible linker (Gly₃Ser)₃.

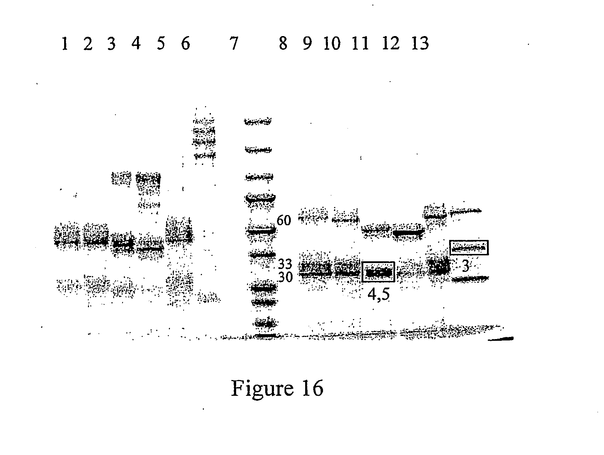

All four of these variants and the original CMG2-IgG were expressed transiently in N. benthamiana and purified from leaf extracts by Protein A chromatography. In addition, the original CMG-IgG was expressed along with a human kappa chain. Equal quantities of each protein were subjected to SDS-PAGE under non-reducing and reducing conditions, and the gel was stained with Coomassie blue **(****Figure 16****).** All of the variants contain significantly more of the highest molecular weight form than did the original CMG2-IgG. Variants V1 and V3 appear to have the same degradation fragments (30 and 33 kDa) as the original CMG2-IgG, while variant V2a has two different fragments of approximately 30 kDa. Amino terminal sequencing of these fragments (surrounded by a box and identified by the numbers **4** and **5** in **Figure 16****)** revealed that they begin just before the hinge region of V2a **(****Figure 15****).**

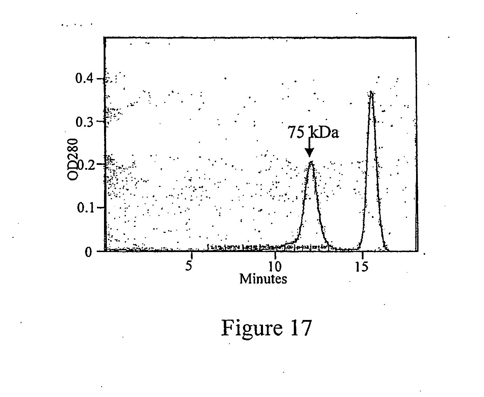

Variant V2b, comprising just the VWA/I domain of CMG2 plus Cγ2/Cγ3 of IgG, was more stable than the others. Under reducing conditions (lane 11, **Figure** 16) most of the protein appears in a 50 kDa band, with very little apparent degradation (expected size of this protein, based on aa sequence, is 46.5 kDa). Under non-reducing conditions **(****Figure 16****,** lane 5) there are major bands at ∼40 kDa and at -97 kDa. Both of these bands reduce to the 50 kDa monomer, indicating that they correspond to monomer and disulfide-bonded dimer forms (data not shown). Size exclusion chromatography (SEC) of the V2b protein shows a single peak at 75 kDa **(****Figure 17****).** The combination of SDS-PAGE and SEC suggests that the protein is a mixture of disulfide-linked dimers and non-covalently associated dimers, probably held together by interactions between Cγ3 domains.

### Expression of CMG2-IgG in presence of Kappa Light Chain

Also of interest is what happens to CMG2-IgG expressed in the presence of kappa chain. It forms a ladder of bands starting at approximately 137 kDa and going up in units of 35 kDa (lane 6, **Figure 16****).** When reduced, this ladder resolves into three bands of 60, 40 and 28 kDa (lane 13). The 28 kDa band is kappa chain, and the 60 kDa band is full-length CMG2-IgG. The 40 kDa band from this lane (number 3 in **Figure 16****)** was subjected to Edman degradation, which showed that its amino terminus was at V196. This demonstrated that kappa chain was able to prevent cleavage in Cγ1, but not in the extra 14 amino acid sequence from CMG2.

### Testing for Anti-toxin Activity

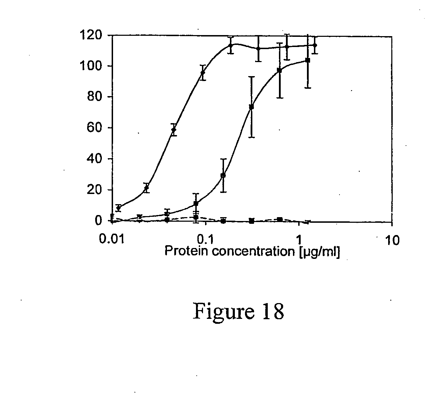

The original CMG2-IgG and CMG2-IgG V2b were tested for anti-toxin activity in the macrophage protection assay, along with a negative control antibody **(****Figure 18****).** CMG2-IgG V2b was superior to the original, with the other variants having intermediate activity (data not shown). The concentration of CMG2-IgG V2b required to neutralize anthrax toxin activity by 50% (the IC₅₀) was 35 ng/ml (mean of two assays, **Table 2).** This compares favorably with anti-PA antibodies being developed by Human Genome Sciences (IC₅₀ = 50 ng/ml),⁵² Elusys (IC₅₀ = 80 ng/ml),²⁷ and Avanir (IC₅₀ = 30 to 70 ng/ml).²³ The molar ratio of CMG2-IgG V2b to PA at the IC₅₀ is 0.38, meaning that each molecule of CMG2-IgG V2b can neutralize almost three molecules of anthrax Lethal Toxin.

### Structural Analysis

By overlaying three crystal structures - IgG Fc (1E4K in the Protein Data Base⁵³), the VWA/I domain of CMG2 (with internal disulfide bond, 1SHU³⁴), and CMG2 bound to PA (1T6B³⁶) a predicted model of what the CMG-IgG V2b structure might look like and how it might bind to PA was derived **(****Figure 19****).** Size exclusion chromatography suggests that almost all of the CMG2-IgG Var2b is in the dimeric form, but SDS-PAGE analysis suggests that at least 50% of the dimers are non-covalently bound. This may be due to the shape of the CMG2 VWA/I domain, which is more globular than the more elongated immunoglobulin Cγ1 domain **(****Figure 19**). Steric hindrance may prevent the cysteines in the hinge regions of the IgG Fc from approaching close enough to form disulfide bonds efficiently.

### Expression of CMG2-IgG in presence of CMG2-kappa

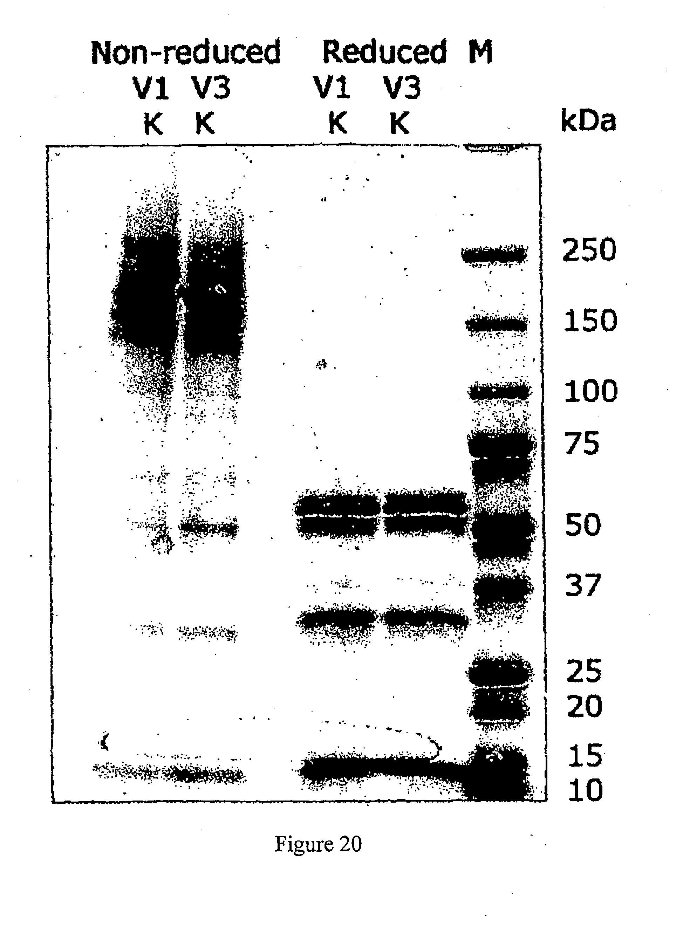

A construct comprised of the first 199 amino acids of CMG2 and a human kappa chain constant domain (CMG2-kappa) was assembled. CMG2-kappa was transiently expressed together with Variant 1 or Variant 3 (both of which contain intact Cγ1 domains), and the resulting proteins were purified using Protein A Sepharose. In both cases the dominant molecular species were a pair of bands at approximately 180 and 160 kDa **(****Figure 20****).** Under reducing conditions there were bands at 57 kDa, 34 kDa and 13 kDa. Immuno-blots (not shown) demonstrated that the 57 kDa band contained IgG heavy chain, while the smaller bands reacted with kappa-chain specific antiserum. The size of the smallest band would be consistent with cleavage in the 14 amino acids between the CMG2 VWA/I domain and the kappa constant region. The major non-reduced bands reacted with both anti-IgG and anti-kappa antisera, and probably represent assembled tetrameric molecules with two "heavy chains" and two "light chains". These molecules were tested in the RAW macrophage protection assay. The IC₅₀ was 44 ng/ml for V1/k and 32 ng/ml for V3/k **(Table 2).** Because of their larger size, the molar ratio of antitoxin:PA at the IC50 was much lower for both of the new proteins: 0.25 for V1/k and 0.18 for V3/k.

**Table 7. Calculated IC₅₀ (ng/mL) and molar ratio of antitoxin to PA at the IC₅₀. Preparations of Original CMG2-IgG and variant were mixed with 80 ng/ml of PA and 80 ng/ml of LF in the RAW macrophage protection assay (for protocol see D.1.5). Results are from assays on three different days.**

| | Assay 07/20/05 | | Assay 07/27/05 | | Assay 07/28/05 | |
|---|---|---|---|---|---|---|
| **Variant** | **IC50** | **Molar ratio** | **IC50** | **Molar ratio** | **IC50** | **Molar ratio** |
| **Original CMG2-IgG** | 242 | 2.16 | - | - | 224 | 2.00 |
| **V1** | - | - | - | - | 197 | 1.80 |
| **V2a** | - | - | - | - | 67 | 0.72 |
| **V2b** | 41 | 0.45 | - | - | 28 | 0.31 |
| **V3** | - | - | - | - | 100 | 0.91 |
| **V1/K** | - | - | 44 | 0.25 | - | - |
| **V3/K** | - | - | 32 | 0.18 | - | - |

### IgG Clearance via the FcγR and the effect of N-glycan

The mechanisms involved in the *in vivo* neutralization and clearance of anthrax toxin and/or *B. anthracis* infection by the various antitoxins currently being developed are poorly understood. In the case of antibodies and immunoadhesins, in vivo neutralization of toxin: antitoxin complexes may depend on interactions with the FcγR. Many types of leucocytes have cell surface receptors for Fc regions on IgG, known as FcγR Binding of antigen-antibody complexes to these cells through the Fc-FcγR interaction may result in a number of responses, including antibody-dependent cellular cytotoxicity (ADCC) and internalization of the receptor-ligand complexes.

Crystal structures show that FcγR binds to the lower hinge region of Cγ2 of the heavy chain Fc regions.⁵⁵ Genetic and biochemical approaches have identified amino acid residues that modulate this interaction, as well as the role of the *N*-linked glycan on IgG Fc. *N*-linked glycans are oligosaccharide structures that are covalently attached to certain asparagine residues of glycoproteins. Crystal structure analysis suggests that the N-glycan at Asn²⁹⁷ of human IgG stabilizes a specific conformation of the Fc region that promotes FcγR binding^{55, 56} **(Fig. 21).** Elimination of this N-glycan dramatically reduces the affinity of IgG for the three major FcγR isoforms *in vitro,* but does not affect *in vivo* half-life.^{57, 58, 59}

It is not yet known what effect glycosylation or lack thereof will have on the in vivo protective effect of CMG2-IgG against anthrax lethal toxin, or B. anthracis infection. If efficient clearance/destruction of antitoxin-PA complexes is affected by binding to FcγR, it may be affected by the glycosylation status of the antitoxin. Scientists at Avanir Pharmaceuticals compared the *in vivo* activity of glycosylated and aglycosyl forms of two of their anti-PA antibodies, and found very little difference in protective efficacy.²³ From this they concluded that lethal toxin neutralization is not Fc effector mediated. However, this was just one experiment, and there was a reduced efficacy with one of the aglycosyl antibodies. For this reason, aglycosyl (the form tested in these Examples) and glycosylated forms of the CMG2-IgG immunoadhesin will be tested.

There are subtle differences in the N-glycans of animal and plant glycoproteins, and some have raised concerns about the potential immunogenicity/allergenicity of plant glycoproteins.^{60, 61} The 1H IgG and the CMG2-IgG in these Examples was made using an aglycosyl heavy chain constant region. It is believed that immunogenicity will not be a major issue for an anthrax neutralization therapy based on a plant-made immunoadhesin, even if it is glycosylated. Should immunogenicity turn out to be a significant problem, it should be possible to genetically modify plants to "humanize" their N-glycans.⁶²⁻⁶⁷

Conclusion

A plant-made immunoadhesin, consisting of the extracellular domain of the human anthrax toxin receptor CMG2 fused to the Fc region of human IgG1 (CMG2-IgG), was able to protect mouse macrophages from the lethal effects of anthrax Lethal Toxin at nanomolar concentrations. The specific activity of the immunoadhesin appears to be comparable to the best available monoclonal antibodies.

### Example 19: Additional CMG2-IgG chimera Variants

This Example demonstrates representative modifications that can be made to improve stability and yield of the chimeric toxin receptor proteins and immunoadhesins described herein.

Variant 2b will be modified to further increase yield. The introduction of a flexible (Gly₃Ser)₃ linker between the VWA/I and Cγ2 domains (see structure in Figure 19), will result in a variant with contain a higher percentage of disulfide-bonded dimers, making it more stable and/or active in vivo. The 14 amino acids between the CMG2 VWA/I domain and Cκ in the CMG2-kappa construct will also be replaced with a (Gly₃Ser)₃ linker. These two new proteins will be produced in transiently transfected tobacco and purified them using Protein A-Sepharose chromatography. They will be tested for proper assembly and stability. Binding constants will be determined by surface plasmon resonance. Their activity will be compared to that of the existing immunoadhesins in the RAW macrophage protection assay. Glycosylation is not anticipated to affect activity in this cell-based assay. However, glycosylation may affect in vivo activity, so after selecting the most potent immunoadhesin antitoxin the glycosylation site in the Cγ2 domain will be reintroduced. Both glycosyl and aglycosyl versions will be tested in vivo.

### Molecular Constructs for Expression of CMG2-IgG in Tobacco

Variant 4 will be identical to variant 2b with the addition of a flexible linker, and will be constructed by ligating a DNA fragment encoding the VWA/I domain plus the (Gly₃Ser)₃ linker from variant 3 (amino acids 1-196, Figure 14) to a DNA fragment encoding the IgG hinge and constant domains Cγ2-Cγ3 from variant 2b (amino acids 298-531, Figure 14).

In addition, CMG2-kappa will be modified by replacing the extra 14 amino acids with a flexible (Gly₃Ser)₃ linker. Each variant will be placed in an expression cassette under the control of a constitutive plant promoter (CaMV35S, or another promoter of our choosing) and a plant 3' polyadenylation signal sequence. The expression cassette will be cloned into the binary vector pGPTV,⁴⁸ and the resulting plasmid transformed into Agrobacterium tumefaciens strain EHA105.

**Table 8**

| **Immunoadhesin** | **Domain Arrangement** | **Expected # of PA binding sites** |
|---|---|---|
| V2b | CMG2-Cγ2-Cγ3 | 2 |
| V4 | CMG2-(G₃S)₃-Cγ2-Cγ3 | 2 |
| | VWA/I-GGGSGGGSGGGS-EPKSCDKTHTCP- CH2-CH3 | |
| V3/CMG-kappa | CMG2-(G₃S)₃-Cγ1-Cγ2-Cγ3/CMG2-(G₃S)₃-Cκ | 4 |

After selecting the most potent antitoxin (using the macrophage protection assay) site-directed mutagenesis will be performed to convert a codon for Gln (at position 381 in Figure 14) to Asn, using a commercially available kit.

### Tobacco Transient Expression System

Strains of Agrobacterium tumefaciens carrying a binary vector for expression of Variant 2b, 4, 5 or 6, or two Agrobacterium strains carrying Variant 3 plus the new CMG2-kappa, will be infiltrated into leaves ofNicotiana benthamiana (approximately 100), along with an additional strain carrying a binary vector (P19) encoding a viral suppressor of silencing. The silencing suppressor is used to achieve high levels of expression. After infiltration, each Agrobacterium transfers its T-DNA (containing the expression cassettes) from the binary vector into the plant cells, where it is integrated and transcribed. The immunoadhesin then accumulates to high levels in the leaves, which are harvested 7 days after infiltration and processed for purification of immunoadhesin. Small amounts of each of these five chimeric proteins (<5 mg), sufficient for in vitro and cell culture analysis, as well as for rat in vivo toxin neutralization studies, can be produced in a short time using this tobacco transient expression system.⁵¹

### Small-Scale Immunoadhesin Purification and Biophysical Characterization

Tobacco leaves (N. tabacum or N. benthamiana) will be homogenized and extracted in an aqueous buffer, centrifuged and filtered to remove solids, and proteins with intact IgG-Fc regions will be purified by Protein A-Sepharose chromatography, dialyzed against PBS, and filter sterilized. The purified protein will be characterized by Coomassie staining and by immuno-blotting using antibodies against human IgG1 (or kappa chain). Four criteria will be used to evaluate success at this stage. First, a chimeric protein purified by Protein A will consist primarily of one size species. Second, it will have an apparent molecular weight consistent with the encoded protein sequence(s). Third, it will react with anti-IgG antisera. Fourth, it will have higher activity in the RAW macrophage protection assay than the current CMG-IgG V2b. We will measure the binding kinetics of the immunoadhesins to Protective Antigen (List Biological Labs) by surface plasmon resonance in a BIAcore (Pharmacia Biosensor), which will be used to calculate the K_{d}.

### Protection of mouse macrophages against toxin challenge.

Survival of RAW 264.7 mouse macrophage-like cells (ATCC #TIB-71) after administration of CMG2-IgG along with toxin will be determined essentially as described.⁶⁸ CMG2-IgG will be titrated from 10-1000 ng/ml and pre-incubated with toxin (80 ng/ml PA, 80 ng/ml LF) for 30 min before addition to cells. After three hours of exposure to toxin, an indirect viability assay will be performed using MTT (3-(4,5-dimethylthiazol-2-yl)-2,5-diphenyltetrazolium bromide). Cells will be incubated with medium containing 2 mg/ml MTT for 30 min, solubilized with acidic isopropanol (40 mM HCl, 0.5% SDS in 90% isopropyl alcohol) and the absorbance measured at A₅₉₅. The percentage of cells surviving toxin challenge at a specified reagent dose, as compared with sham-treated cells, will be reported as measured by MTT assay ((average test well - average of eight toxin-only wells) x 100%/average of eight no-toxin wells). From the dose response curve, IC₅₀ values (concentration of antitoxin at which 50% of activity is neutralized) will be estimated. The effective molar ratio, or the ratio of antitoxin to toxin at the IC₅₀, will also be determined for each chimeric protein.

Addition of the flexible (Gly₃Ser)₃ linker will lead to a measurable improvement in disulfide bonding of dimers, or in vitro neutralizing activity, or both.

### Example 20: Antitoxin activity of CMG2-IgG in animal models of anthrax infection

This Example demonstrates animal testing that can be performed to identify chimeric toxin receptor proteins and immunoadhesins of this invention.

The presence or absence of N-glycan on the Fc of the selected immunoadhesin may affect the in vivo efficacy against toxin, or against a *B. anthracis* spore challenge, because of differences in interactions with Fc receptors and/or serum half-life of the antitoxin. Both aglycosyl and glycosylated forms of CMG2-IgG will be tested in a Fischer rat toxin neutralization assay. This will be followed by tests of the in vivo half-life of both forms in rabbits, followed by efficacy in a rabbit spore challenge assay. The rat in vivo toxin neutralization assays will require approximately 3 mg of each antitoxin, which will be easily supplied from transient tobacco transfections. The rabbit pharmacokinetic and spore challenge studies will require about 200 mg of each antitoxin, which will be purified from stably transformed tobacco.

### Fischer rat in vivo anthrax toxin neutralization

The in vivo anthrax toxin neutralization experiments will be performed, using the glycosylated and aglycosyl forms of CMG2-IgG, essentially as described by Ivins.⁶⁹ Male Fischer 344 rats with jugular vein catheters, weighing between 200 and 250 g (Charles River Laboratories), will be anaesthetized in an Isofluorane EZ-anesthesia chamber (Euthanex Corp, PA) following manufacturer's guidelines. A dose of antitoxin (0.25 and 0.12 nmols/rat, corresponding to 1x and 0.5x molar equivalent to the lethal toxin) will be administered via the catheter in 0.2 ml PBS/0.1% BSA pH 7.4. Five minutes later lethal toxin (PA 20 µg / LF 4 µg in 0.2 ml/200 g rat) will be administered. The negative control will be an unrelated plant-made human IgG1 (our lab). Five animals will be used in each test group (5 animals/group x 2 doses x 2 antitoxins = 20 animals), and four animals in each control (4 animals/group x 2 doses control IgG = 8 animals). Animals will be monitored for discomfort and time of death versus survival, as assessed by cessation of breathing and heartbeat. Rats will be maintained under anesthesia for 5 hr after exposure to lethal toxin, or until death, to minimize discomfort. Rats that survive 5 hours will be monitored for a longer period of time (up to 72 hours). Serum samples will be collected from surviving rats at 5 hour intervals and assayed for the concentration of antitoxin by ELISA. In a similar study, one antibody produced by Avanir Pharmaceuticals protected all animals against this dose of lethal toxin at 0.5x molar equivalent to the toxin.²³ We would expect our plant-made immunoadhesin to perform as well. If necessary, experiments will be repeated with immunoadhesin at even lower molar ratio to the lethal toxin. The minimum effective dose will be determined for each of the two antitoxin forms.

### Pharmacokinetic parameters in Rabbits

To establish the pharmacokinetics (PK) of CMG2-IgG injected by the intravenous (i.v.) route, Dutch banded rabbits (three per group, 1.5 to 2.0 kg) will be injected with CMG2-IgG at 10 mg/rabbit i.v. via the medial ear artery. Blood samples will be collected from the ear artery of each animal at 1 h pre-injection and 1, 4, and 8 hours, and 1, 2, 3, 4, 5, 6, 8, 10, 12, 15, 17, 19 and 21 days post-injection. Sera will be obtained after clotting and centrifugation and stored frozen at -80°C until analysis.

The concentration of CMG2-IgG in rabbit serum samples will be measured by a functional ELISA. Purified CMG2-IgG will be used as a standard. Protective antigen (List Biological Labs) at a concentration of 1 µg/ml is coated onto wells (100 µl/well) of microtiter plates (Costar Corp.) and incubated overnight at 4°C. The unbound antigen is washed out, and the wells are blocked with PBS + 5% non-fat dry milk for 1h at room temperature. The blocking solution is then aspirated. The samples and standard are applied to the PA-coated plate for 1 h at 37°C followed by three washes. This process is followed by addition of goat anti-human IgG horseradish peroxidase (HRP) conjugate for 1 h at 37°C and washing again three times. Antibody complexes are detected by adding HRP substrate and incubating 30 minutes at room temperature. Color development (absorbance at 405 nm) is determined using a Benchmark Microplate Reader. The ELISA will be tested for rabbit serum interference and nonspecific binding.

Descriptive PK parameters will be determined by standard model-independent methods⁷⁰ based on individual serum concentration-time data for CMG2-IgG. The pre-dose time point for i.v. administration will be assigned the concentration value of the first time point for PK calculations. The maximal serum concentration (Cₘₐₓ), time taken to reach Cₘₐₓ, area under the curve, systemic clearance, volume of distribution at steady state, terminal half-life, and absolute bioavailability (F) will be analyzed for each rabbit.

### Aerosol challenge of rabbits with Bacillus anthracis spores

Animal models have been developed to study passive protection against anthrax infection in guinea pigs,^{29, 71, 72} mice^{9, 73-75} and rabbits.⁷⁶ One advantage of the mouse models is that they require much less antitoxin. However the best protection with anti-PA antibodies has been in rabbits,^{24, 27} so we plan to use this same model to test our CMG2-IgG.

Starting about month 16, both glycosyl and aglycosyl chimeric toxin receptor proteins will be tested in a pre-exposure prophylaxis study. If rabbits are successfully protected (100% at the highest dose of antitoxin) in this study, we will then perform a post-exposure treatment study, starting about month 21. Dutch banded rabbits (1.5 to 2 kg) will be randomized into groups containing four male and four female animals. For pre-exposure prophylaxis, rabbits on day 0 will receive a single dose of CMG2-IgG administered i.v. (1, 2, or 4 mg of CMG2-IgG/rabbit via the medial ear artery) or phosphate-buffered saline (PBS) administered i.v. as a control 30 to 45 min before anthrax spore challenge. To evaluate post-exposure efficacy, rabbits will be challenged with spores on day 0 and then given single i.v. injections of 4 mg of CMG2-IgG/rabbit at 24, 36, or 48 h post-challenge or PBS i.v. at 48 h post-challenge. Rabbits will be challenged via a muzzle-only exposure system according to standard protocols. The target aerosol exposure for the challenge is 200 times the 50% lethal dose (LD₅₀) of the Ames strain (Ames LD₅₀ = 1.05 x 10⁵ spores⁷⁶). Actual challenge doses will be determined from the starting concentration and a cumulative minute volume gathered throughout the exposure. All animals will be observed twice daily for 28 days post-challenge. Blood and serum will be collected pre-challenge and on days 1, 2, 7, 10, 14, 21, and 28 post-challenge. When possible, blood and serum samples will be collected from animals that are moribund or recently dead ("at death") and analyzed for the presence of B. anthracis. On day 28, all survivors will be euthanized, and the lungs, spleens, and intrathoracic lymph nodes will be harvested and cultured. Serum samples will be sterile filtered and deemed noninfectious by culturing prior to sending to Planet Biotechnology for ELISA analysis to determine serum CMG2-IgG levels (as in D.2.3) and rabbit anti-PA titers (described below).

### Measurement of rabbit anti-PA antibody response to immunoadhesins

Previous studies have found that rabbits protected from inhalational anthrax by anti-PA monoclonal antibodies developed protective titers of their own anti-PA antibodies. An ELISA, similar to that used to quantitate CMG2-IgG in serum, will be used to measure the immune response against PA in the rabbits challenged with anthrax spores. In this assay microtiter plates are coated with PA at a concentration of 1 µg/ml and incubated overnight at 4°C. The unbound antigen is washed out, and the wells are blocked. The blocking solution is then aspirated. Dilutions of serum samples are incubated on the plate for 1 hour at 37°C. A goat anti-rabbit IgG HRP conjugate (Santa Cruz Biotech) is added for 30 min at 37°C, and color is developed by adding HRP substrate and incubating 30 minutes at room temperature. Color development (absorbance at 405 nm) is determined using a Benchmark Microplate Reader. The serum dilution that results in an optical density signal of 1.0 will be used as a measure of the response (titer).

### Example 21 Botulinum Chimeric Toxin Receptor Proteins (not of the invention)

Chimeric botulinum toxin receptor proteins are constructed by fusing a botulinum toxin receptor to heavy chain constant regions. Synaptotagmin I (STI) botulinum chimeric toxin receptor proteins and SV2 botulinum chimeric toxin receptor proteins are constructed.

### Botulinum Toxin Receptor Fused to Heavy Chain

Portions of STI (fragSTI) and SV2 (fragSV2), those amino acid sequences sufficient for toxin binding, are fused to the heavy chain constant region with additional proteinaceous sequence(s) as is necessary for accumulation, solubility, purification, protease resistance, subsequent modification to alter immunogenicity or to increase the half-life in the patient and targeting, such as the ability to favorably interact with gangliosides such as Gt1b and Gd1a. Polypeptide linkers, such as ((Gly)xSer)n, are also included to insulate the folding requirements and functionality of these sequences. The nucleic acid sequences encoding these polypeptides are optimized to include the use of synonomous codons to avoid recombination during cloning and transformation and to avoid small RNA-mediated inhibition of expression.

The botulinum toxin receptor portions fused to the heavy chain constant region can also be homo- and hetero- multimers of fragSTI or fragSV2. For the homo-multimer, the botulinum toxin receptor portion fused to the heavy chain constant region is a single polypeptide chain where the sequence of either fragSTI or fragSV2 is present two or more times, i.e. the fragment's sequence is present in series in a single primary amino acid sequence. Multimerized binding fragments are expected to increase apparent binding affinities by an avidity effect and therefore increase potency. For the hetero-multimer, the botulinum toxin receptor portion fused to the heavy chain constant region is a single polypeptide chain where the sequences of both fragSTI and fragSV2 are present one or more times, i.e. the fragments' sequences are present in series in a single primary amino acid sequence. The position of repeated fragment sequences in the linear array is varied to increase binding avidity and thus increase potency.

### Chimeric Botulinum Toxin Receptor Protein

Each of the above described botulinum toxin receptor portions is fused to the Fc region of IgG or IgA, in particular to the amino or carboxy terminus of heavy chain constant regions 1, 2 and 3 (α1, a2, a3 and γ1, γ2 and γ3) or to heavy chain constant regions 2 and 3 (a2 and a3 or γ2 and y3). Chimeras of the heavy chain constant regions may also be constructed. For example, a chimera of the constant heavy regions γ1, γ2, a2 and a3 can be employed to facilitate purification by Protein G affinity chromatography.

### Improved Chimeric Botulinum Toxin Receptor Protein with Light Chain Constant Region

Each of the above described botulinum toxin receptor portions is fused to the Fc region of IgG or IgA as above and, in separate polypeptide chains, also fused to the constant region of the light chain (κ or λ), at either the amino or carboxy termini. The antibody constant regions are assembled during synthesis and cellular maturation into tetramers where the heavy and light chain constant regions are assembled in canonical fashion by virtue of covalent and non-covalent protein-protein interactions between the various constant regions.

### Improved Chimeric Botulinum Toxin Receptor Protein with Light Chain Constant Region and an Antibody Variable Domain

An antibody variable domain with a binding specificity that improves decoy performance is incorporated into a complex with the botulinum toxin receptor portion and a constant region of a light chain. The following types of variable domain specificities may be used to improve decoy performance: an anti-ganglioside binding specificity to direct the decoy to synaptic membranes, where botulinum toxin is known to concentrate; a binding specificity that directs the decoy away from the synaptic membranes to other serum or cellular components, such as those which participate in Fc-mediated clearance, to ensure rapid destruction of the bound toxin; an anti-toxin specificity to increase toxin binding or broaden toxin recognition by having variable regions recognize other toxin amino acid sequences which may be chosen to encourage toxin cross-linking as well as increased binding.

### Higher Order Multimers

Higher order multimer sIgA-like fusions are formed by fusing the polypeptides described above to the heavy chain constant regions a2 and α3, which directs the further assembly of Joining chain (J chain) and Secretory Component (SC) or Protection Protein (PP).

Higher order multimers are also formed by fusing the polypeptides described above to the heavy-chain constant regions of IgM, which allows the assembly of J chain and several (five) antibody-like fusions into an IgM-like decoy receptor.

### ADDITIONAL REFERENCES

1. Dixon, T. C., Fadl, A. A., Koehler, T. M., Swanson, J. A. & Hanna, P. C. Early Bacillus anthracis-macrophage interactions: intracellular survival and escape. Cell Microbiol 2, 453-63. (2000).
2. Leppla, S. H. Anthrax toxin edema factor: a bacterial adenylate cyclase that increases cyclic AMP concentrations of eukaryotic cells. Proc Natl Acad Sci U S A 79, 3162-6. (1982).
3. Vitale, G., Pellizzari, R., Recchi, C., Napolitani, G., Mock, M. & Montecucco, C. Anthrax lethal factor cleaves the N-terminus of MAPKKs and induces tyrosine/threonine phosphorylation of MAPKs in cultured macrophages. Biochem Biophys Res Commun 248, 706-11. (1998).
4. Duesbery, N. S. et al. Proteolytic inactivation of MAP-kinase-kinase by anthrax lethal factor. Science 280, 734-7. (1998).
5. Friedlander, A. M. Macrophages are sensitive to anthrax lethal toxin through an acid- dependent process. J Biol Chem 261, 7123-6. (1986).
6. Hanna, P. C., Acosta, D. & Collier, R. J. On the role of macrophages in anthrax. Proc Natl Acad Sci U S A 90, 10198-201. (1993).
7. Popov, S. G. et al. Systemic cytokine response in murine anthrax. Cell Microbiol 6, 225-33 (2004).
8. Alibek, K. & Handelman, S. Biohazard -- the chilling true story of the largest covert biological weapons program in the world -- told from inside by the man who ran it (Random House, 1999).
9. Karginov, V. A. et al. Treatment of anthrax infection with combination of ciprofloxacin and antibodies to protective antigen of Bacillus anthracis. FEMS Immunol Med Microbiol 40, 71-4 (2004).
10. Joellenbeck, L. M., Zwanziger, L. L., Durch, J. S. & Strom, B. L. (eds.) The Anthrax Vaccine: Is It Safe? Does It Work? (Natl. Acad. Press, Washington, DC, 2002).
11. Ivins, B. E., Welkos, S. L., Little, S. F., Crumrine, M. H. & Nelson, G. O. Immunization against anthrax with Bacillus anthracis protective antigen combined with adjuvants. Infect Immun 60, 662-8. (1992).
12. Hambleton, P. & Turnbull, P. C. Anthrax vaccine development: a continuing story. Adv Biotechnol Processes 13, 105-22 (1990).
13. Tan, Y., Hackett, N. R., Boyer, J. L. & Crystal, R. G. Protective immunity evoked against anthrax lethal toxin after a single intramuscular administration of an adenovirus-based vaccine encoding humanized protective antigen. Hum Gene Ther 14, 1673-82 (2003).
14. Galloway, D. R. & Baillie, L. DNA vaccines against anthrax. Expert Opin Biol Ther 4, 1661-7 (2004).
15. Rhie, G. E., Roehrl, M. H., Mourez, M., Collier, R. J., Mekalanos, J. J. & Wang, J. Y. A dually active anthr ax vaccine that confers protection against both bacilli and toxins. Proc Natl Acad Sci U S A 100, 10925-30 (2003).
16. Hermanson, G. et al. A cationic lipid-formulated plasmid DNA vaccine confers sustained antibody-mediated protection against aerosolized anthrax spores. Proc Natl Acad Sci U S A 101, 13601-6 (2004).
17. Watson, J., Koya, V., Leppla, S. H. & Daniell, H. Expression of Bacillus anthracis protective antigen in transgenic chloroplasts of tobacco, a non-food/feed crop. Vaccine 22, 4374-4384 (2004).
18. Rainey, G. J. & Young, J. A. Antitoxins: novel strategies to target agents of bioterrorism. Nat Rev Microbiol 2, 721-6 (2004).
19. Artenstein, A. W. et al. Chloroquine Enhances Survival in Bacillus anthracis Intoxication. The Journal of Infectious Diseases 190, 1655-1660 (2004).
20. Shoop, W. L. et al. Anthrax lethal factor inhibition. Proc Natl Acad Sci U S A 102, 7958-63 (2005).
21. Forino, M. et al. Efficient synthetic inhibitors of anthrax lethal factor. Proc Natl Acad Sci U S A 102, 9499-9504 (2005).
22. Wang, F. et al. Human monoclonal antibodies that neutralize anthrax toxin by inhibiting heptamer assembly. Hum Antibodies 13, 105-10 (2004).
23. Sawada-Hirai, R. et al. Human anti- anthrax protective antigen neutralizing monoclonal antibodies derived from donors vaccinated with anthrax vaccine adsorbed. J Immune Based Ther Vaccines 2, 5 (2004).
24. Peterson, J. W. et al. in American Society for Microbiology Human Monoclonal anti-PA antibody completely protects rabbits and is synergistic with ciprofloxacin in protecting mice and guinea pigs against inhalational anthrax. (2005).
25. Maynard, J. A. et al. Protection against anthrax toxin by recombinant antibody fragments correlates with antigen affinity. Nat Biotechnol 20, 597-601 (2002).
26. Harvey, B. R., Georgiou, G., Hayhurst, A., Jeong, K. J., Iverson, B. L. & Rogers, G. K. Anchored periplasmic expression, a versatile technology for the isolation of high-affinity antibodies from Escherichia coli-expressed libraries. Proc Natl Acad Sci U S A 101, 9193-8 (2004).
27. Mohamed, N. et al. A high-affinity monoclonal antibody to anthrax protective antigen passively protects rabbits before and after aerosolized Bacillus anthracis spore challenge. Infect Immun 73, 795-802 (2005).
28. Beebe, L. et al. in Program and abstracts of the 43rd Interscience Conference on Antimicrobial Agents and Chemotherapy 47 Protection against inhalation anthrax-induced lethality by a human monoclonal antibody to protective antigen in rabbits and cynomolgus monkeys [abstract 3836]. (American Society for Microbiology, (Chicago). Washington, DC, 2003).
29. Altboum, Z., Gozes, Y., Barnea, A., Pass, A., White, M. & Kobiler, D. Postexposure prophylaxis against anthrax: evaluation of various treatment regimens in intranasally infected guinea pigs. Infect Immun 70, 6231-41 (2002).
30. Bradley, K. A., Mogridge, J., Mourez, M., Collier, R. J. & Young, J. A. Identification of the cellular receptor for anthrax toxin. Nature 414, 225-9 (2001).
31. Scobie, H. M., Rainey, G. J., Bradley, K. A. & Young, J. A. Human capillary morphogenesis protein 2 functions as an anthrax toxin receptor. Proc Natl Acad Sci U S A 100, 5170-4 (2003).
32. Liu, S. & Leppla, S. H. Cell surface tumor endothelium marker 8 cytoplasmic tail-independent anthrax toxin binding, proteolytic processing, oligomer formation, and internalization. J Biol Chem 278, 5227-34 (2003).
33. Rosovitz, M. J. et al. Alanine-scanning mutations in domain 4 of anthrax toxin protective antigen reveal residues important for binding to the cellular receptor and to a neutralizing monoclonal antibody. J Biol Chem 278, 30936-44 (2003).
34. Lacy, D. B., Wigelsworth, D. J., Scobie, H. M., Young, J. A. & Collier, R. J. Crystal structure of the von Willebrand factor A domain of human capillary morphogenesis protein 2: an anthrax toxin receptor. Proc Natl Acad Sci U S A 101, 6367-72 (2004).
35. Lacy, D. B., Wigelsworth, D. J., Melnyk, R. A., Harrison, S. C. & Collier, R. J. Structure of heptameric protective antigen bound to an anthrax toxin receptor: A role for receptor in pH-dependent pore formation. Proc Natl Acad Sci U S A (2004).
36. Santelli, E., Bankston, L. A., Leppla, S. H. & Liddington, R. C. Crystal structure of a complex between anthrax toxin and its host cell receptor. Nature 430, 905-8 (2004).
37. Wigelsworth, D. J., Krantz, B. A., Christensen, K. A., Lacy, D. B., Juris, S. J. & Collier, R. J. Binding stoichiometry and kinetics of the interaction of a human anthrax toxin receptor, CMG2, with protective antigen. J Biol Chem 279,23349-56 (2004).
38. Scobie, H. M. & Young, J. A. Interactions between anthrax toxin receptors and protective antigen. Curr Opin Microbiol 8, 106-12 (2005).
39. Martin, S., Casasnovas, J. M., Staunton, D. E. & Springer, T. A. Efficient neutralization and disruption of rhinovirus by chimeric ICAM-1/immunoglobulin molecules. J Virol 67, 3561-8 (1993).
40. Ober, R. J., Martinez, C., Vaccaro, C., Zhou, J. & Ward, E. S. Visualizing the Site and Dynamics of IgG Salvage by the MHC Class I-Related Receptor, FcRn. J Immunol 172, 2021-2029 (2004).
41. Ma, J. et al. Characterization of a recombinant plant monoclonal secretory antibody and preventive immunotherapy in humans. Nature Medicine 4, 601-606 (1998).
42. Ma, J. K. et al. Generation and assembly of secretory antibodies in plants. Science 268, 716-719 (1995).
43. Larrick, J. W., Yu, L., Chen, J., Jaiswal, S. & Wycoff, K. Production of antibodies in transgenic plants. Res Immunol 149, 603-8. (1998).
44. Organisms, C. o. t. B. C. o. G. E. 6 Biological Confinement of Genetically Engineered Organisms. (National Research Council, Washington, D.C., 2004).
45. Nielsen, H., Engelbrecht, J., Brunak, S. & von Heijne, G. Identification of prokaryotic and eukaryotic signal peptides and prediction of their cleavage sites. Protein Engineering 10, 1-6 (1997).
46. Guerche, P., Tire, C., De Sa, F. G., De Clercq, A., Van Montagu, M. & Krebbers, E. Differential Expression of the Arabidopsis 2S Albumin Genes and the Effect of Increasing Gene Family Size. Plant Cell 2, 469-478 (1990).
47. Odell, J. T., Nagy, F. & Chua, N. H. Identification of DNA sequences required for activity of the cauliflower mosaic virus 35S promoter. Nature 313, 810-812 (1985).
48. Becker, D., Kemper, E., Schell, J. & Masterson, R. New plant binary vectors with selectable markers located proximal to the left T-DNA border. Plant Molecular Biology 20, 1195-1197 (1992).
49. Yamamoto, Y. Y., Tsuji, H. & Obokata, J. 5'-leader of a photosystem I gene in Nicotiana sylvestris, psaDb, contains a translational enhancer. J Biol Chem 270, 12466-70 (1995).
50. Sawant, S. V., Singh, P. K., Gupta, S. K., Madnala, R. & Tuli, R. Conserved nucleotide sequences in highly expressed genes in plants. Journal of Genetics 78, 123-131 (1999).
51. Voinnet, O., Rivas, S., Mestre, P. & Baulcombe, D. An enhanced transient expression system in plants based on suppression of gene silencing by the p19 protein of tomato bushy stunt virus. Plant J 33, 949-56 (2003).
52. Zhang, X. et al. in Program and abstracts of the 43rd Interscience Conference on Antimicrobial Agents and Chemotherapy 277 Selection of potent neutralizing human monoclonal antibodies to protective antigen of Bacillus anthracis [abstract 3976]. (American Society for Microbiology, (Chicago). Washington, DC, 2003).
53. Sondermann, P., Huber, R., Oosthuizen, V. & Jacob, U. The 3.2-A crystal structure of the human IgG1 Fc fragment-Fc gammaRIII complex. Nature 406, 267-73 (2000).
54. Wein, L. M. & Liu, Y. Analyzing a bioterror attack on the food supply: The case of botulinum toxin in milk. Proc Natl Acad Sci U S A 102, 9984-9 (2005).
55. Shields, R. L. et al. High resolution mapping of the binding site on human IgG1 for Fc gamma RI, Fc gamma RII, Fc gamma RIII, and FcRn and design of IgG1 variants with improved binding to the Fc gamma R. J Biol Chem 276, 6591-604 (2001).
56. Radaev, S. & Sun, P. D. Recognition of IgG by Fcgamma receptor. The role of Fc glycosylation and the binding of peptide inhibitors. J Biol Chem 276, 16478-83 (2001).
57. Jefferis, R. & Lund, J. Interaction sites on human IgG-Fc for FcgammaR: current models. Immunol Lett 82, 57-65 (2002).
58. Dorai, H., Mueller, B. M., Reisfeld, R. A. & Gillies, S. D. Aglycosylated chimeric mouse/human IgG1 antibody retains some effector function. Hybridoma 10, 211-7 (1991).
59. Hand, P. H. et al. Comparative biological properties of a recombinant chimeric anti- carcinoma mAb and a recombinant aglycosylated variant. Cancer Immunol Immunother 35, 165-74 (1992).
60. Cabanes-Macheteau, M. et al. N-Glycosylation of a mouse IgG expressed in transgenic tobacco plants. Glycobiology 9, 365-72 (1999).
61. Ko, K. et al. Function and glycosylation of plant-derived antiviral monoclonal antibody. Proc Natl Acad Sci U S A 100, 8013-8 (2003).
62. Lerouge, P., Bardor, M., Pagny, S., Gomord, V. & Faye, L. N-glycosylation of recombinant pharmaceutical glycoproteins produced in transgenic plants: towards an humanisation of plant N-glycans. Curr Pharm Biotechnol 1, 347-54 (2000).
63. Palacpac, N. Q. et al. Stable expression of human beta1,4-galactosyltransferase in plant cells modifies N-linked glycosylation patterns. Proc Natl Acad Sci U S A 96, 4692-7 (1999).
64. Bakker, H. et al. Galactose-extended glycans of antibodies produced by transgenic plants. Proc Natl Acad Sci U S A 98, 2899-904. (2001).
65. Misaki, R., Kimura, Y., Palacpac, N. Q., Yoshida, S., Fujiyama, K. & Seki, T. Plant cultured cells expressing human beta1,4-galactosyltransferase secrete glycoproteins with galactose-extended N-linked glycans. Glycobiology 13, 199-205 (2003).
66. Pagny, S. et al. Structural requirements for Arabidopsis beta1,2-xylosyltransferase activity and targeting to the Golgi. Plant J 33, 189-203 (2003).
67. Koprivova, A. et al. Targeted knockouts of Physcomitrella lacking plant-specific immunogenic N-glycans. Plant Biotechnology Journal 2, 517-523 (2004).
68. Varughese, M., Chi, A., Teixeira, A. V., Nicholls, P. J., Keith, J. M. & Leppla, S. H. Internalization of a Bacillus anthracis protective antigen-c-Myc fusion protein mediated by cell surface anti-c-Myc antibodies. Mol Med 4, 87-95 (1998).
69. Ivins, B. E., Ristroph, J. D. & Nelson, G. O. Influence of body weight on response of Fischer 344 rats to anthrax lethal toxin. Appl Environ Microbiol 55, 2098-100 (1989).
70. Perrier, D. & Gibaldi, M. General derivation of the equation for time to reach a certain fraction of steady state. J Pharm Sci 71, 474-5 (1982).
71. Little, S. F., Ivins, B. E., Fellows, P. F. & Friedlander, A. M. Passive protection by polyclonal antibodies against Bacillus anthracis infection in guinea pigs. Infect Immun 65, 5171-5 (1997).
72. Kobiler, D., Gozes, Y., Rosenberg, H., Marcus, D., Reuveny, S. & Altboum, Z. Efficiency of protection of guinea pigs against infection with Bacillus anthracis spores by passive immunization. Infect Immun 70, 544-60 (2002).
73. Lyons, C. R. et al. Murine model of pulmonary anthrax: kinetics of dissemination, histopathology, and mouse strain susceptibility. Infect Immun 72, 4801-9 (2004).
74. Steward, J., Lever, M. S., Simpson, A. J., Sefton, A. M. & Brooks, T. J. Post-exposure prophylaxis of systemic anthrax in mice and treatment with fluoroquinolones. J Antimicrob Chemother 54, 95-9 (2004).
75. Brossier, F., Levy, M., Landier, A., Lafaye, P. & Mock, M. Functional analysis of Bacillus anthracis protective antigen by using neutralizing monoclonal antibodies. Infect Immun 72, 6313-7 (2004).
76. Zaucha, G. M., Pitt, L. M., Estep, J., Ivins, B. E. & Friedlander, A. M. The pathology of experimental anthrax in rabbits exposed by inhalation and subcutaneous inoculation. Arch Pathol Lab Med 122, 982-92 (1998).
77. Horsch, R. B., Fry, J. E., Hoffmann, N. L., Eichholtz, D., Rogers, S. G. & Fraley, R. T. A simple and general method for transferring genes into plants. Science 227, 1229-1231 (1985).
78. Rogers, S. G., Horsch, R. B. & Fraley, R. T. Gene transfer in plants: Production of transformed plants using Ti plasmid vectors. Methods Enzymol. 118, 627-640 (1986).
79. Cundell, A. Microbial Testing in Support of Aseptic Processing. Pharmaceutical Technology 28, 56-66 (2004).
80. Hariharan, M., VanNoord, T. & Greden, J. F. A high-performance liquid-chromatographic method for routine simultaneous determination of nicotine and cotinine in plasma. Clin Chem 34, 724-9 (1988).
81. Little, S. F. et al. Characterization of lethal factor binding and cell receptor binding domains of protective antigen of Bacillus anthracis using monoclonal antibodies. Microbiology 142 (Pt 3), 707-15 (1996).
82. Bäumlein H, Wobus U, Pustell J, Kafatos FC (1986) The legumin gene family: structure of a B type gene of Vicia faba and a possible legumin gene specific regulatory element. Nucl. Acids Res. 14: 2707-2713
83. Becker D, Kemper E, Schell J, Masterson R (1992) New plant binary vectors with selectable markers located proximal to the left T-DNA border. Plant Mol. Biol. 20: 1195-1197
84. Bradley K A, Mogridge J, Mourez M, Collier R J, Young J A T (2001) Identification of the cellular receptor for anthrax toxin. Nature 414: pre-publication
85. Chintalacharuvu K R, Raines M, Morrison S L (1994) Divergence of human alpha-chain constant region gene sequences. A novel recombinant alpha 2 gene. Journal of Immunology 152: 5299-5304
86. Crump et al. (1994) Comparative Antirhinoviral Activities of Soluble Intercellular Adhesion Molecule-1 (sICAM-1) and Chimeric ICAM-1Immunoglobulin A Molecule. Antimicrobial Agents and Chemotherapy, 38:6, p. 1425-1427
87. Depicker A, Stachel S, Dhaese P, Zambryski P, Goodman H M (1982) Nopaline synthase: transcript mapping and DNA sequence. J. Mol. Appl. Genet. 1:561-573
88. Gielen J, De Beuckeleer M, Seurinck J, Deboeck F, De Greve H, Lemmers M, Van Montagu M, Schell J (1984) The complete nucleotide sequence of the TL-DNA of the Agrobacterium tumefaciens plasmid pTiAch5. Embo J 3: 83546
89. Greve et al.(1991) EP 0468257, Multimeric form of human rhinovirus receptor protein.
90. Horsch R B, Fry J E, Hoffmann N L, Eichholtz D, Rogers S G, Fraley R T (1985) A simple and general method for transferring genes into plants. Science 227: 1229-1231
91. Ingelbrecht I, Breyne P, Vancompernolle K, Jacobs A, Van Montagu M, Depicker A (1991) Transcriptional interference in transgenic plants. Gene 109: 239-242
92. MacDonald M H, Mogen B D, Hunt A G (1991) Characterization of the polyadenylation signal from the T-DNA-encoded octopine synthase gene. Nucleic Acids Res 19: 5575-81
93. Martin et al. (1993), Efficient Neutralization and Disruption of Rhinovirus by Chimeric ICAM-1/Immunoglobulin Molecules. J. of Virology, 67:6, p. 3561-3568.
94. Mogen B D, MacDonald M H, Leggewie G, Hunt A G (1992) Several distinct types of sequence elements are required for efficient mRNA 3' end formation in a pea rbcS gene. Mol Cell Biol 12: 5406-14
95. Ni M, Cui D, Einstein J, Narasimhulu S, Vergara C E, Gelvin S B (1995) Strength and tissue specificity of chimeric promoters derived from the octopine and mannopine synthase genes. Plant Journal 7: 661-676
96. Sawant S V, Singh P K, Gupta S K, Madnala R, Tuli R (1999) Conserved nucleotide sequences in highly expressed genes in plants. Journal of Genetics 78: 123-131
97. St Croix B, Rago C, Velculescu V, Traverso G, Romans K E, Montgomery E, Lai A, Riggins G J, Lengauer C, Vogelstein B, Kinzler K W (2000) Genes expressed in human tumor endothelium. Science 289: 1197-202.
98. Yamamoto Y Y, Tsuji H, Obokata J (1995) 5'-leader of a photosystem I gene in Nicotiana sylvestris, psaDb, contains a translational enhancer. J Biol Chem 270: 12466-70.

The foregoing examples are not limiting and merely representative of various aspects and embodiments of the present invention. All documents cited are indicative of the levels of skill in the art to which the invention pertains.

One skilled in the art will readily appreciate that the present invention is well adapted to carry out the objects and obtain the ends and advantages mentioned, as well as those inherent therein. The methods and compositions described illustrate preferred embodiments, are exemplary, and are not intended as limitations on the scope of the invention.

The invention illustratively described herein suitably may be practiced in the absence of any element or elements, limitation or limitations which is not specifically disclosed herein. The terms and expressions which have been employed are used as terms of description and not of limitation, and there is no intention in the use of such terms and expressions of excluding any equivalents of the features shown and described, or portions thereof. It is recognized that various modifications are possible within the scope of the invention claimed. Thus, it should be understood that although the present invention has been specifically disclosed by preferred embodiments, optional features, modifications and variations of the concepts herein disclosed may be resorted to by those skilled in the art.

In addition, where features or aspects of the invention are described in terms of Markush groups or other grouping of alternatives, those skilled in the art will recognize that the invention is also thereby described in terms of any individual member or subgroup of members of the Markush group or other group, and exclusions of individual members as appropriate.

## Claims

1. A chimeric toxin receptor protein comprising:
an immunoglobulin complex, wherein the immunoglobulin complex comprises at least a portion of an immunoglobulin G (IgG) heavy chain
and capillary morphogenesis protein 2 (CMG2) anthrax toxin receptor protein lacking amino acid residues of SEQ ID NO: 102 and covalently associated with the IgG heavy chain, or portion thereof.

2. A chimeric toxin receptor protein as claimed in claim 1, in the form of a dimer with a further chimeric toxin receptor of claim 1.

3. A chimeric toxin receptor protein as claimed in claim 1 or claim 2, wherein the immunoglobulin complex further comprises at least a portion of an immunoglobulin light chain; preferably a kappa or lambda chain.

4. A chimeric toxin receptor protein as claimed in any of claims 1 to 3, wherein the IgG heavy chain, or portion thereof, and CMG2 anthrax toxin receptor protein lacking amino acid residues of SEQ ID NO: 102 are covalently associated via a (Gly₃Ser)₃ linker.

5. A chimeric toxin receptor protein as claimed in any preceding claim, wherein the Cγ1 domain of the IgG heavy chain is omitted.

6. A chimeric toxin receptor protein as claimed in any of claims 1 to 3, wherein the covalent linkage between the CMG2 anthrax toxin receptor protein and the IgG heavy chain is an immunoglobulin hinge.

7. A chimeric toxin receptor protein as claimed in any preceding claim, wherein the portion of the IgG heavy chain only comprises heavy chain constant regions 2 and 3.

8. A chimeric toxin receptor protein as claimed in any preceding claim, wherein the IgG heavy chain and anthrax toxin receptor protein are human proteins.

9. A chimeric toxin receptor protein as claimed in any preceding claim, wherein the chimeric CMG2-IgG protein has an amino acid sequence of SEQ ID NO: 114.

10. A composition comprising a CMG2 chimeric toxin receptor protein of any of claims 1 to 9 and plant material.

11. A composition as claimed in claim 10, wherein the plant material is selected from the group consisting of: plant cell walls, plant organelles, plant cytoplasm, intact plant cells, plant seeds, and viable plants.

12. A method for reducing binding of an anthrax antigen to a host cell *in vitro,* the method comprising: contacting the anthrax antigen with the chimeric toxin receptor protein of any of claims 1 to 9, wherein the chimeric toxin receptor protein binds to the anthrax antigen and reduces the binding of the anthrax antigen to the host cell.

13. A chimeric toxin receptor protein of any of claims 1 to 9 for use as a medicament for a patient or animal.

14. A chimeric toxin receptor protein of any of claims 1 to 9 for use in preventing or for use in treating anthrax infection, or the effects of anthrax toxin.

15. An expression vector encoding a CMG2 chimeric toxin receptor protein as claimed in any of claims 1 to 9.

16. A method for producing a chimeric toxin receptor protein of any of claims 1 to 9, comprising introducing an expression vector of claim 15 into a cellular host; and expressing the immunoglobulin complex and the CMG2 anthrax toxin receptor protein to form the chimeric toxin receptor protein.

17. A method as claimed in claim 16, wherein the host is a plant.

18. A pharmaceutical composition comprising a chimeric toxin receptor protein as claimed in any of claims 1 to 9 and a pharmaceutically acceptable carrier.

## Patentansprüche

1. Chimäres Toxinrezeptorprotein, das folgendes umfasst:
einen Immunoglobulinkomplex, wobei der Immunoglobulinkomplex mindestens einen Teil einer schweren Kette von Immunoglobulin G (IgG) umfasst;
und einen kapillaren Morphogenese-Protein-2 (CMG2) Anthrax-Toxinrezeptor ohne Aminosäurerückstände der SEQ ID NO. 102 und kovalent der schweren IgG-Kette oder einem Teil dieser zugeordnet.

2. Chimäres Toxinrezeptorprotein nach Anspruch 1 in Form eines Dimers mit einem weiteren chimären Toxinrezeptor nach Anspruch 1.

3. Chimäres Toxinrezeptorprotein nach Anspruch 1 oder Anspruch 2, wobei der Immunoglobulinkomplex ferner mindestens einen Teil einer leichten Kette eines Immunoglobulins umfasst; vorzugsweise einer Kappa- oder einer Lambda-Kette.

4. Chimäres Toxinrezeptorprotein nach einem der Ansprüche 1 bis 3, wobei die schwere IgG-Kette oder ein Teil dieser und das CMG2-Anthrax-Toxinrezeptorprotein ohne Aminosäurerückstände der SEQ ID NO. 102 über einen (Gly₃Ser)₃-Linker kovalent zugeordnet sind.

5. Chimäres Toxinrezeptorprotein nach einem der vorstehenden Ansprüche, wobei die Cy1-Domäne der schweren IgG-Kette weggelassen wird.

6. Chimäres Toxinrezeptorprotein nach einem der Ansprüche 1 bis 3, wobei die kovalente Bindung zwischen dem CMG2-Anthrax-Toxinrezeptorprotein und der schweren IgG-Kette eine Immunoglobulinverbindung ist.

7. Chimäres Toxinrezeptorprotein nach einem der vorstehenden Ansprüche, wobei der Teil der schweren IgG-Kette nur die konstanten Regionen 2 und 3 der schweren Kette umfasst.

8. Chimäres Toxinrezeptorprotein nach einem der vorstehenden Ansprüche, wobei es sich bei der schweren IgG-Kette und dem Anthrax-Toxinrezeptorprotein um menschliche Proteine handelt.

9. Chimäres Toxinrezeptorprotein nach einem der vorstehenden Ansprüche, wobei das chimäre CMG2-IgG-Protein eine Aminosäuresequenz SEQ ID NO. 114 aufweist.

10. Zusammensetzung, welche ein chimäres CMG2-Toxinrezeptorprotein nach einem der Ansprüche 1 bis 9 und einen pflanzlichen Stoff umfasst.

11. Zusammensetzung nach Anspruch 10, wobei der pflanzliche Stoff aus der Gruppe ausgewählt wird, die folgendes umfasst: Pflanzenzellenwände, Pflanzenorganellen, Pflanzencytoplasma, intakte Pflanzenzellen, Pflanzensamen und lebensfähige Pflanzen.

12. Verfahren zum Reduzieren der Bindung eines Anthrax-Antigens an einer Wirtszelle in vitro, wobei das Verfahren folgendes umfasst: das Kontaktieren des Anthrax-Antigens mit dem chimären Toxinrezeptorprotein nach einem der Ansprüche 1 bis 9, wobei das chimäre Toxinrezeptorprotein an das Anthrax-Antigen bildet und die Bindung des Anthrax-Antigens an die Wirtszelle reduziert.

13. Chimäres Toxinrezeptorprotein nach einem der Ansprüche 1 bis 9 zur Verwendung als ein Arzneimittel für einen Patienten oder ein Tier.

14. Chimäres Toxinrezeptorprotein nach einem der Ansprüche 1 bis 9 zur Verwendung in der Prävention oder zur Verwendung in der Behandlung einer Anthraxinfektion oder den Auswirkungen von Anthrax-Toxin.

15. Expressionsvektor, der für ein chimäres CMG2-Toxinrezeptorprotein nach einem der Ansprüche 1 bis 9 codiert.

16. Verfahren zum Erzeugen eines chimären Toxinrezeptorproteins nach einem der Ansprüche 1 bis 9, wobei dieses das Einführen eines Expressionsvektors nach Anspruch 15 in einem zellularen Wirt umfasst sowie das Ausdrücken des Immunoglobulinkomplexes und des CMG2-Anthrax-Toxinrezeptorproteins, so dass das chimäre Toxinrezeptorprotein gebildet wird.

17. Verfahren nach Anspruch 16, wobei es sich bei dem Wirt um eine Pflanze handelt.

18. Pharmazeutische Zusammensetzung, die ein chimäres Toxinrezeptorprotein nach einem der Ansprüche 1 bis 9 und einen pharmazeutisch verträglichen Träger umfasst.

## Revendications

1. Protéine de récepteur de toxine chimérique comprenant :
un complexe immunoglobuline, dans lequel le complexe immunoglobuline comprend au moins une partie d'une chaîne lourde d'immunoglobuline G (IgG)
et une protéine de récepteur de toxine du charbon de protéine de morphogenèse capillaire de type 2 (CMG2) dépourvue de résidus d'acides aminés de SEQ ID N° : 102 et associée par covalence à la chaîne lourde IgG ou à une partie ce celle-ci.

2. Protéine de récepteur de toxine chimérique selon la revendication 1, sous la forme d'un dimère avec un autre récepteur de toxine chimérique selon la revendication 1.

3. Protéine de récepteur de toxine chimérique selon la revendication 1 ou 2, dans lequel le complexe immunoglobuline comprend en outre au moins une partie d'une chaîne légère d'immunoglobuline ; de préférence d'une chaîne kappa ou lambda.

4. Protéine de récepteur de toxine chimérique selon l'une quelconque des revendications 1 à 3, dans laquelle la chaîne lourde IgG, ou une partie de celle-ci, et la protéine de récepteur de toxine du charbon CMG2 dépourvue de résidus d'acides aminés de SEQ ID N° : 102 sont associées de manière covalente via un lieur (Gly₃Ser)₃.

5. Protéine de récepteur de toxine chimérique selon l'une quelconque des revendications précédentes, dans laquelle le domaine Cyl de la chaîne lourde IgG est omis.

6. Protéine de récepteur de toxine chimérique selon l'une quelconque des revendications 1 à 3, dans laquelle la liaison covalente entre la protéine de récepteur de toxine du charbon CMG2 et la chaîne lourde IgG est une charnière d'immunoglobuline.

7. Protéine de récepteur de toxine chimérique selon l'une quelconque des revendications précédentes, dans laquelle la partie de la chaîne lourde IgG comprend seulement des régions constantes de chaîne lourde 2 et 3.

8. Protéine de récepteur de toxine chimérique selon l'une quelconque des revendications précédentes, dans laquelle la chaîne lourde IgG et la protéine de récepteur de toxine du charbon sont des protéines humaines.

9. Protéine de récepteur de toxine chimérique selon l'une quelconque des revendications précédentes, dans laquelle la protéine CMG2-IgG chimérique a une séquence d'acides aminés de SEQ ID N° : 114.

10. Composition comprenant une protéine de récepteur de toxine chimérique CMG2 selon l'une quelconque des revendications 1 à 9 et une matière végétale.

11. Composition selon la revendication 10, dans lequel la matière végétale est choisie dans le groupe constitué de : parois cellulaires végétales, organites végétaux, cytoplasmes végétaux, cellules végétales intactes, graines végétales et plantes viables.

12. Procédé pour la réduction de la liaison d'un antigène du charbon à une cellule hôte *in vitro,* le procédé comprenant les étapes consistant à : mettre en contact l'antigène du charbon avec la protéine de récepteur de toxine chimérique selon l'une quelconque des revendications 1 à 9, dans lequel la protéine de récepteur de toxine chimérique se lie à l'antigène du charbon et réduit la liaison de l'antigène du charbon à la cellule hôte.

13. Protéine de récepteur de toxine chimérique selon l'une quelconque des revendications 1 à 9 pour une utilisation en tant que médicament pour un patient ou un animal.

14. Protéine de récepteur de toxine chimérique selon l'une quelconque des revendications 1 à 9 pour une utilisation dans la prévention ou pour une utilisation dans le traitement de l'infection charbonneuse, ou les effets de la toxine du charbon.

15. Vecteur d'expression codant une protéine de récepteur de toxine chimérique CMG2 selon l'une quelconque des revendications 1 à 9.

16. Procédé de production d'une protéine de récepteur de toxine chimérique selon l'une quelconque des revendications 1 à 9, comprenant les étapes consistant à introduire un vecteur d'expression selon la revendication 15 dans un hôte cellulaire ; et à exprimer le complexe immunoglobuline et la protéine de récepteur de toxine du charbon CMG2 pour former la protéine de récepteur de toxine chimérique.

17. Procédé selon la revendication 16, dans lequel l'hôte est un végétal.

18. Composition pharmaceutique comprenant une protéine de récepteur de toxine chimérique selon l'une quelconque des revendications 1 à 9 et un entraîneur pharmaceutiquement acceptable.
